# EUROPEAN PATENT APPLICATION

(11) **EP 4 276 104 A2**
(43) Date of publication of application: **15.11.2023**
(21) Application number: 23184769.0
(22) Date of filing: 18.03.2022
(51) Int. Cl.: C07H 21/00

(54) **IMPROVED OLIGONUCLEOTIDE SYNTHESIS SUPPRESSING DEPURINATION**

(30) Priority: 19.03.2021 EP 21163788
(62) Divisional of application: 22711569.8
(71) Applicant: Bachem Holding AG, 4416 Bubendorf (CH)
(72) Inventor: Eisenhuth, Ralf, 4053 Basel (CH); Haug, Karl Rüdiger, Danville, 94506 (US); Samson, Daniel, 4051 Basel (CH); Berthelmann, Arne, 79639 Grenzach-Wyhlen (DE); Creusen, Guido, 4127 Birsfelden (CH)
(74) Representative: Ruttekolk, Ivo Robert

(57) **Abstract**

An improved protocol for the synthesis of oligonucleotides is provided. A liquid composition for the cleavage of a di(p-methoxyphenyl)phenylmethyl protecting group from a hydroxyl group and the use thereof in chemical synthesis of oligonucleotides is disclosed.

## Description

The invention generally relates to the field of oligonucleotide synthesis at an industrial or laboratory scale. Improved methods for synthesis of oligonucleotides are disclosed. The invention is directed to methods of effectively removing a (temporary) protecting group comprising an optionally substituted triarylmethyl residue, preferably a di(p-methoxyphenyl)phenylmethyl protecting group, during iterative oligonucleotide synthesis. Further aspects of the invention are directed to a liquid composition and the use thereof for removing a protecting group comprising an optionally substituted triarylmethyl residue, preferably a di(p-methoxyphenyl)phenylmethyl protecting group, from a hydroxyl group during the chemical synthesis of an oligonucleotide.

Generally speaking, iterative methods of chemical oligonucleotide synthesis commonly rely on the use of a first type of protecting group for those functional groups located in the base/base analog and ribose/ribose analog moieties, which are not involved in chain elongation, and on the use of a second type of protecting group for controlling backbone extension. The latter type is a temporary protecting group, which is comprised in the building block to be added. Its function is to avoid double insertions of the building block and/or multimerization of building block moieties. The first and second type of protecting groups are typically orthogonal to each other, meaning that one type can be removed under conditions, which do not affect the other type of protecting group.

Commonly, the first type of protecting groups are cleavable under alkaline conditions and are "permanent" in that they will only be removed once chain assembly is complete; the second type of temporary protecting group is typically cleavable under acidic conditions and is removed once per synthetic cycle. Triarylmethyl protecting groups are commonly used for temporary protection. The coupling cycles therefore comprise a step of coupling a protected building block to an unprotected end of the oligonucleotide backbone, followed by deprotection of the thus-extended oligonucleotide in order to prepare for the subsequent coupling cycle. To facilitate separation from the reagents after each step, the growing oligonucleotide chain is commonly bound to a supporting moiety, which may be a solid support or a solubility modifying tag.

For phosphoramidite oligonucleotide synthesis, the synthesis cycle usually starts by selective deprotection of a hydroxyl group, e.g. the 5' hydroxyl group of the ribose moiety. Next, the growing oligonucleotide chain is incubated with an appropriately protected nucleoside phosphoramidite and activating reagents. The DMT (dimethoxytrityl aka. di(p-methoxyphenyl)phenylmethyl) group is often used as temporary protecting group. The tricoordinated phosphite triester resulting from the coupling reaction is then oxidized or sulfurized, which allows routine synthesis of a phosphate triester, a phosphorothioate, a phosphorodithioate, or mixed backbone structure. An optional capping step may be used to block any unreacted hydroxyl groups, thereby avoiding the occurrence of deletion sequences, which differ from the target oligonucleotide by the absence of only a single nucleoside subunit, in subsequent coupling rounds.

For phosphonate oligonucleotide synthesis, nucleoside H-phosphonate monoesters with an acid sensitive temporary protecting group such as DMT are used. The internucleosidic H-phosphonate diester linkages are typically oxidized or sulfurized at the end of the chain assembly. Depending on the reaction conditions used, phosphodiester linkages, phosphorthioate linkages, phosphorselenoates or phosphoramidate analogs and other derivatives may be generated in this step.

The step of removing the temporary triarylmethyl type protecting groups has proven surprisingly challenging for the synthesis of high quality oligonucleotides. Triarylmethyl type protecting groups, in particular the DMT group, are well known and heavily used in organic synthesis. Various cleavage protocols exist, one example for the cleavage of tritylamines being described in US 8299206.

In the context of oligonucleotide synthesis, however, the DMT cleavage step brings about the risk of acid induced base cleavage, because both reactions are acid-catalyzed. This side reaction is particularly pronounced with guanine and adenine type bases and is referred to as depurination. To avoid depurination, various strategies have been explored. Nucleobase protecting groups reducing the basicity, alternative temporary protecting groups, and coupling schemes using di- and trinucleotides have been tested, but none of these approaches has proven its value for routine oligonucleotide synthesis. In addition, the cleavage reaction itself and the reaction conditions have been studied. Russell et al. (Org. Biomol. Chem., 2009, 7, 52-57) demonstrated that in contrast to the deamination of 4,4'-dimethoxytritylamine, which is assumed to involve an initial protonation step, cleavage of a DMT group from nucleotides in non-aqueous solution likely occurs through a concerted general acid-catalyzed mechanism.

Habus and Agarwal (WO 96/03417) teach that a detritylation agent comprising 2% dicholoroacetic acid (DCA) and 0.1% of a lower alcohol such as ethanol or methanol, and/or 0.1 to 1% 1H-pyrrole suppresses depurination. Septak (Nucleic Acids Research, 1996, Vol. 24, No. 15, 3053-3058) and Cheruvallath (Organic Process Research & Development 2003, 7, 917-920) both teach to increase the concentration of dichloroacetic acid (DCA) in the detritylation reagent from 3% to 10% and to keep the contact time between detritylation reagent and oligonucleotide minimal in order to favor detritylation over depurination. This approach is still used for the industrial scale production of oligonucleotides such as those exemplarily usable as active pharmaceutical ingredients (APIs). However, having to limit the contact time between the detritylation reagent and the oligonucleotide severely limits the scalability of the process, because the maximal pumping capacity of the liquid handling systems is limited.

There is a long felt, still unsatisfied need for a method to deprotect the (backbone-related) hydroxyl group of a growing oligonucleotide chain, while suppressing depurination, i.e. unwanted base cleavage, wherein this method is scalable and amenable to large scale production of oligonucleotides.

The present invention provides a solution to this problem.

In one aspect, the present invention provides the use of a liquid composition C comprising an aprotic solvent, at least one alcohol selected from the group consisting of trifluoroethanol (i.e. 2,2,2-trifluoroethanol, TFE), hexafluoroisopropanol (i.e. 1,1,1,3,3,3-hexafluoro-2-propanol, HFIP), pentafluoropropanol, 1,1,1,3,3,3-hexafluoro-2-methyl-2-propanol, and nonafluoro tertiary butyl alcohol, and a salt of a base with a strong acid, wherein the salt's cation has a pka in the range of 1 to 4, for the cleavage of a protecting group comprising an optionally substituted triarylmethyl residue from a hydroxyl group during the chemical synthesis of an oligonucleotide. The liquid composition C may in particular be used to suppress acid-induced nucleobase cleavage, in particular depurination, while effecting cleavage of a protecting group comprising an optionally substituted triarylmethyl residue, preferably a DMT protecting group, from a hydroxyl group during the chemical synthesis of an oligonucleotide.

As used herein, the indefinite articles "a" and "an" may mean "one or more", unless indicated differently.

Accordingly, one aspect of the present invention also relates to the use of a liquid composition C comprising an aprotic solvent, at least one alcohol selected from the group consisting of trifluoroethanol, hexafluoroisopropanol, pentafluoropropanol, 1,1,1,3,3,3-hexafluoro-2-methyl-2-propanol, and nonafluoro tertiary butyl alcohol, and a salt of a base with a strong acid, wherein the salt's cation has a pKa in the range of 1 to 4, for suppressing nucleobase cleavage, in particular depurination, while effecting cleavage of a protecting group comprising an optionally substituted triarylmethyl residue, preferably a DMT protecting group, from a hydroxyl group during the chemical synthesis of an oligonucleotide.

As used herein, an "aprotic solvent" is a solvent that is not a hydrogen bond donor. Hence, an aprotic solvent may be a solvent without O-H or N-H bonds.

One aspect of the present invention relates to the use of a liquid composition C comprising an aprotic solvent, at least one alcohol selected from the group consisting of trifluoroethanol, hexafluoroisopropanol, pentafluoropropanol, 1,1,1,3,3,3-hexafluoro-2-methyl-2-propanol, and nonafluoro tertiary butyl alcohol, and a salt of a base with a strong acid, wherein the salt's cation has a pKa in the range of 1 to 4 and the strong acid has a pKa of less than 1, for suppressing nucleobase cleavage, in particular depurination, while effecting cleavage of a di(p-methoxyphenyl)phenylmethyl (DMT) protecting group from a hydroxyl group during the chemical synthesis of an oligonucleotide.

As known to those skilled in the art, "nucleobase cleavage" typically occurs with purine type nucleobases such as adenine and guanine, which may be referred to as "depurination". Adenine may be particularly prone to depurination.

It will be understood by those skilled in the art that the term "salt of a base with a strong acid" refers to a population of molecules of said base and said strong acid, wherein some of the base molecules and some of the acid molecules may be associated with each other, e.g. by means of electrostatic interactions. Typically, the strong acid may donate a proton to the base. This should however not be construed to mean that all molecules of said base and all molecules of said strong acid within the liquid composition C will be associated with each other at all times. In fact, as known to those skilled in the art, a solution of a salt may comprise the ions in solvated form. Additionally, protonation of the base by the strong acid may be followed by deprotonation of the protonated base (i.e. the salt's cation), e.g. by transferring the proton to the oxygen atom from which the protecting group comprising an optionally substituted triarylmethyl residue (e.g. the DMT group) is to be removed. The strong acid may also protonate species different from the base in the first place or engage in hydrogen bonding with such species, e.g. with nucleobases of the oligonucleotide. Accordingly, as used throughout this text, the expression "liquid composition comprising an aprotic solvent and a salt of a base with a strong acid, wherein the salt's cation has a pka in the range of 1 to 4" is synonymous with the expression "a liquid composition comprising an aprotic solvent, a base, whose protonated form has a pKa in the range of 1 to 4, and a strong acid". Both expressions are used interchangeably throughout this text.

Thus, one aspect of the present invention relates to the use of a liquid composition C comprising an aprotic solvent, at least one alcohol selected from the group consisting of trifluoroethanol, hexafluoroisopropanol, pentafluoropropanol, 1,1,1,3,3,3-hexafluoro-2-methyl-2-propanol, and nonafluoro tertiary butyl alcohol, a base, whose protonated form has a pKa in the range of 1 to 4, and a strong acid having a pKa of less than 1, for suppressing nucleobase cleavage, in particular depurination, while effecting cleavage of a di(p-methoxyphenyl)phenylmethyl (DMT) protecting group from a hydroxyl group during the chemical synthesis of an oligonucleotide.

Further, the present invention also relates to the use of a liquid composition C comprising a non-halogenated and/or (hetero)aromatic aprotic solvent, at least one alcohol selected from the group consisting of trifluoroethanol, hexafluoroisopropanol(aka. 1,1,1,3,3,3-hexafluor-2-propanol), pentafluoropropanol, 1,1,1,3,3,3-hexafluoro-2-methyl-2-propanol, and nonafluoro tertiary butyl alcohol, and a salt of a base with a strong acid, wherein the salt's cation has a pKa in the range of 1 to 4 and the strong acid has a pKa of less than 1, for suppressing nucleobase cleavage, in particular depurination, while effecting cleavage of a protecting group comprising an optionally substituted triarylmethyl residue, preferably a DMT protecting group, from a hydroxyl group during the chemical synthesis of an oligonucleotide.

Further, the present invention also relates to the use of a liquid composition C comprising a non-halogenated and/or (hetero)aromatic aprotic solvent, trifluoroethanol, and a salt of a base with a strong acid, wherein the salt's cation has a pKa in the range of 1 to 4 and the strong acid has a pKa of less than 1, for suppressing nucleobase cleavage, in particular depurination, while effecting cleavage of a protecting group comprising an optionally substituted triarylmethyl residue, preferably a DMT protecting group, from a hydroxyl group during the chemical synthesis of an oligonucleotide.

Further, the present invention also relates to the use of a liquid composition C comprising a non-halogenated and/or (hetero)aromatic aprotic solvent, hexafluoroisopropanol, and a salt of a base with a strong acid, wherein the salt's cation has a pKa in the range of 1 to 4, for suppressing nucleobase cleavage while effecting cleavage of a protecting group comprising an optionally substituted triarylmethyl residue from a hydroxyl group during the chemical synthesis of an oligonucleotide.

DMT (dimethoxytrityl) is a preferred triarylmethyl protecting group (or triarylmethyl residue), and preferred salts or combinations of strong acids and bases for DMT cleavage are detailed herein below. Unless indicated differently, the terms dimethoxytrityl group dimethoxytrityl protecting group, dimethoxytrityl residue, DMT group, and DMT residue are used interchangeably and refer to the di(p-methoxyphenyl)phenylmethyl protecting group.

As used in this context, the term "suppressing" may be understood in the broadest sense as reducing the rate of (undesired) nucleobase cleavage (e.g., depurination). Nucleobase cleavage may be prevented completely or partly upon cleavage of the triarylmethyl type protecting group, e.g. the DMT protecting group.

The degree of nucleobase cleavage/depurination may be used to compare different detritylation protocols with regard to undesired nucleobase cleavage. Briefly, in a chromatogram obtained from HPLC-MS analysis of a synthesized oligonucleotide (cleaved from the support, if support-assisted synthesis was used), the peak areas of the nucleobase cleavage-derived / depurination-derived side products may be summed up to obtain the summed up peak area of all identified nucleobase cleavage-derived / depurination-derived side products. The degree of nucleobase cleavage / depurination in percent (%) may then be determined by dividing the summed up peak areas of nucleobase cleavage-derived/ depurination-derived side products by the area of the product (i.e. the target oligonucleotide) of the respective oligonucleotide synthesis, followed by multiplication with 100% to arrive at a value in percent (%).

In some embodiments, suppressing nucleobase cleavage is reducing the degree of nucleobase cleavage by at least 5%, or at least 10%, or at least 25%, or at least 50%, or at least 75%, or at least 90% in comparison to a comparable detritylation step differing only in that 10% DCA (v/v) in toluene is used instead of the liquid composition C of the invention. Along the same lines, in some embodiments, suppressing depurination is reducing the degree of depurination by at least 5%, or at least 10%, or at least 25%, or at least 50%, or at least 75%, or at least 90% in comparison to a comparable detritylation step differing only in that 10% DCA (v/v) in toluene is used instead of the liquid composition C of the invention.

In this context, the terms "comparable cleaving step" and "comparable detritylation step" may refer to a step of removing a protecting group comprising an optionally substituted triarylmethyl residue, preferably a DMT protecting group, under similar conditions, e.g. same substrate, scale, temperature, time, and volume of the detritylation cocktail, differing only with respect to the composition of the detritylation cocktail, e.g. 10% DCA (v/v) in toluene vs a liquid composition C of the present invention.

The meaning of the term "reducing" in the context of the degree of nucleobase cleavage or the degree of depurination will be understood by a person skilled in the art, and may be exemplified as follows: If dividing the degree of depurination of a first synthesis A using the liquid composition C of the invention for detritylation by the degree of depurination of a second synthesis B using 10% DCA (v/v) in toluene for detritylation affords a quotient of 0.95, the degree of depurination is said to be reduced by 5%.

One aspect of the present invention relates to the use of a liquid composition C comprising an aprotic solvent, at least one alcohol selected from the group consisting of trifluoroethanol, hexafluoroisopropanol, pentafluoropropanol, 1,1,1,3,3,3-hexafluoro-2-methyl-2-propanol, and nonafluoro tertiary butyl alcohol, and a salt of a base with a strong acid, wherein the salt's cation has a pKa in the range of 1 to 4 and the strong acid has a pKa of less than 1, for suppressing cleavage of adenine or guanine, in particular adenine, while effecting cleavage of a di(p-methoxyphenyl)phenylmethyl (DMT) protecting group from a hydroxyl group during the chemical synthesis of an oligonucleotide.

In another aspect, the present invention provides a liquid composition C for use in the cleavage of a protecting group comprising an optionally substituted triarylmethyl residue, preferably a DMT protecting group, from a hydroxyl group, the composition comprising a non-halogenated and/or (hetero)aromatic, aprotic solvent, at least one alcohol selected from the group consisting of trifluoroethanol, hexafluoroisopropanol, pentafluoropropanol, 1,1,1,3,3,3-hexafluoro-2-methyl-2-propanol, and nonafluoro tertiary butyl alcohol, and a salt of a base with a strong acid, wherein the salt's cation has a pKa in the range of 1 to 4.

One aspect of the present invention relates to a liquid composition C for use in the cleavage of a di(p-methoxyphenyl)phenylmethyl (DMT) protecting group from a hydroxyl group, the composition comprising an aprotic solvent, at least one alcohol selected from the group consisting of trifluoroethanol, hexafluoroisopropanol, pentafluoro-propanol, 1,1,1,3,3,3-hexafluoro-2-methyl-2-propanol, and nonafluoro tertiary butyl alcohol, and a salt of a base with a strong acid, wherein the salt's cation has a pKa in the range of 1 to 4 and the strong acid has a pKa of less than 1, and the aprotic solvent is non-halogenated and selected from the group consisting of a (hetero)aromatic solvent, an alkyl (hetero)aromatic solvent, a (hetero)aromatic ether, and an alkyl (hetero)aryl ether.

Thus, one aspect of the present invention relates to a liquid composition C for use in the cleavage of a di(p-methoxyphenyl)phenylmethyl (DMT) protecting group from a hydroxyl group, the composition comprising an aprotic solvent, at least one alcohol selected from the group consisting of trifluoroethanol, hexafluoroisopropanol, pentafluoro-propanol, 1,1,1,3,3,3-hexafluoro-2-methyl-2-propanol, and nonafluoro tertiary butyl alcohol, and a base, whose protonated form has a pKa in the range of 1 to 4, and the strong acid having a pKa of less than 1, wherein the aprotic solvent is non-halogenated and selected from the group consisting of a (hetero)aromatic solvent, an alkyl (hetero)aromatic solvent, a (hetero)aromatic ether, and an alkyl (hetero)aryl ether.

In some embodiments of the liquid composition C of the invention, said liquid composition is for use in the cleavage of a di(p-methoxyphenyl)phenylmethyl (DMT) protecting group from a hydroxyl group, wherein the nucleoside moiety carrying the DMT group comprises a purine type nucleobase, preferably a nucleobase selected from the group consisting of adenine and guanine, in particular adenine.

In another aspect, the present invention provides a method for the synthesis of oligonucleotides, comprising steps a) through f):
a) providing a nucleoside or oligonucleotide bound to a supporting moiety, wherein the nucleoside or oligonucleotide comprises a backbone hydroxyl moiety, which is protected by a protecting group comprising an optionally substituted triarylmethyl residue, preferably a DMT protecting group;
b) cleaving the protecting group comprising an optionally substituted triarylmethyl residue, preferably the DMT protecting group, from the nucleoside or oligonucleotide by incubating said compound with a liquid composition C, thereby generating a free backbone hydroxyl moiety;
c) reacting the free backbone hydroxyl group resulting from step b) with a phosphorus moiety of a nucleoside or oligonucleotide building block, which building block further comprises a hydroxyl group protected by a protecting group comprising an optionally substituted triarylmethyl residue, preferably a DMT protecting group, thereby producing a covalent linkage between the oxygen atom of said free hydroxyl group and the phosphorus atom of said building block;
d) optionally modifying the phosphorus moiety;
e) optionally reiterating the sequence of steps b) and c); and
f) cleaving the oligonucleotide from the supporting moiety;
wherein the liquid composition C comprises an aprotic solvent, at least one alcohol selected from the group consisting of trifluoroethanol, hexafluoroisopropanol, pentafluoropropanol, 1,1,1,3,3,3-hexafluoro-2-methyl-2-propanol, and nonafluoro tertiary butyl alcohol, and a salt of a base with a strong acid, wherein the salt's cation has a pKa in the range of 1 to 4.

One aspect of the present invention relates to a method for the synthesis of oligonucleotides, comprising the following steps a) through f):
a) providing a nucleoside or oligonucleotide bound to a supporting moiety, wherein the nucleoside or oligonucleotide comprises a backbone hydroxyl moiety, which is protected by a di(p-methoxyphenyl)phenylmethyl (DMT) protecting group;
b) cleaving the di(p-methoxyphenyl)phenylmethyl (DMT) protecting group from the nucleoside or oligonucleotide by incubating said compound with a liquid composition C, thereby generating a free backbone hydroxyl moiety;
c) reacting the free backbone hydroxyl group resulting from step b) with a phosphorus moiety of a nucleoside or oligonucleotide building block, which building block further comprises a hydroxyl group protected by a di(p-methoxyphenyl)phenylmethyl (DMT) protecting group, thereby producing a covalent linkage between the oxygen atom of said free hydroxyl group and the phosphorus atom of said building block;
d) optionally modifying the phosphorus moiety;
e) optionally reiterating the sequence of steps b) to c) or b) to d); and
f) cleaving the oligonucleotide from the supporting moiety;
wherein the liquid composition C comprises an aprotic solvent, at least one alcohol selected from the group consisting of trifluoroethanol, hexafluoroisopropanol, pentafluoropropanol, 1,1,1,3,3,3-hexafluoro-2-methyl-2-propanol, and nonafluoro tertiary butyl alcohol, and a salt of a base with a strong acid, wherein the salt's cation has a pKa in the range of 1 to 4 and the strong acid has a pKa of less than 1.

Thus, one aspect of the present invention relates to a method for the synthesis of oligonucleotides, comprising the following steps a) through f):
a) providing a nucleoside or oligonucleotide bound to a supporting moiety, wherein the nucleoside or oligonucleotide comprises a backbone hydroxyl moiety, which is protected by a di(p-methoxyphenyl)phenylmethyl (DMT) protecting group;
b) cleaving the di(p-methoxyphenyl)phenylmethyl (DMT) protecting group from the nucleoside or oligonucleotide by incubating said compound with a liquid composition C, thereby generating a free backbone hydroxyl moiety;
c) reacting the free backbone hydroxyl group resulting from step b) with a phosphorus moiety of a nucleoside or oligonucleotide building block, which building block further comprises a hydroxyl group protected by a di(p-methoxyphenyl)phenylmethyl (DMT) protecting group, thereby producing a covalent linkage between the oxygen atom of said free hydroxyl group and the phosphorus atom of said building block;
d) optionally modifying the phosphorus moiety;
e) optionally reiterating the sequence of steps b) to c) or b) to d); and
f) cleaving the oligonucleotide from the supporting moiety;
wherein the liquid composition C comprises an aprotic solvent, at least one alcohol selected from the group consisting of trifluoroethanol, hexafluoroisopropanol, pentafluoropropanol, 1,1,1,3,3,3-hexafluoro-2-methyl-2-propanol, and nonafluoro tertiary butyl alcohol, a base whose protonated form has a pKa in the range of 1 to 4, and the strong acid having a pKa of less than 1.

In some embodiments, the present invention provides a method for the synthesis of oligonucleotides, comprising steps a) through f):
a) providing a nucleoside or oligonucleotide bound to a supporting moiety, wherein the nucleoside or oligonucleotide comprises a backbone hydroxyl moiety, which is protected by a protecting group comprising an optionally substituted triarylmethyl residue, preferably a DMT protecting group;
b) cleaving the protecting group comprising an optionally substituted triarylmethyl residue, preferably the DMT protecting group, from the nucleoside or oligonucleotide by incubating said compound with a liquid composition C, thereby generating a free backbone hydroxyl moiety;
c) reacting the free backbone hydroxyl group resulting from step b) with a phosphorus (III) moiety of a nucleoside or oligonucleotide building block, which building block further comprises a hydroxyl group protected by a protecting group comprising an optionally substituted triarylmethyl residue, preferably a DMT protecting group, thereby producing a covalent linkage between the oxygen atom of said free hydroxyl group and the phorsphorus (III) atom of said building block;
d) oxidizing or sulfurizing the phosphorus (III) atom to a phosphorus (V) atom, thereby creating the desired type of internucleoside linkage;
e) optionally either reiterating the sequence of steps b) through d) or reiterating the sequence of steps b) and c) before executing step d); and
f) cleaving the oligonucleotide from the supporting moiety;
wherein the liquid composition C comprises an aprotic solvent, at least one alcohol selected from the group consisting of trifluoroethanol, hexafluoroisopropanol, pentafluoropropanol, 1,1,1,3,3,3-hexafluoro-2-methyl-2-propanol, and nonafluoro tertiary butyl alcohol, and a salt of a base with a strong acid, wherein the salt's cation has a pKa in the range of 1 to 4.

A protecting group comprising an optionally substituted triarylmethyl residue may also be designated as a triarylmethyl-based residue, triarylmethyl type group, or trityl-type residue triarylmethyl group, or trityl group, or the like.

Some embodiments of the present invention relate to a method for the synthesis of oligonucleotides, comprising the following steps a) through f):
a) providing a nucleoside or oligonucleotide bound to a supporting moiety, wherein the nucleoside or oligonucleotide comprises a backbone hydroxyl moiety, which is protected by a di(p-methoxyphenyl)phenylmethyl (DMT) protecting group;
b) cleaving the di(p-methoxyphenyl)phenylmethyl (DMT) protecting group from the nucleoside or oligonucleotide by incubating said compound with a liquid composition C, thereby generating a free backbone hydroxyl moiety;
c) reacting the free backbone hydroxyl group resulting from step b) with a phosphorus (III) moiety of a nucleoside or oligonucleotide building block, which building block further comprises a hydroxyl group protected by a di(p-methoxyphenyl)phenylmethyl (DMT) protecting group, thereby producing a covalent linkage between the oxygen atom of said free hydroxyl group and the phorsphorus (III) atom of said building block;
d) oxidizing or sulfurizing the phosphorus (III) atom to a phosphorus (V) atom, thereby creating the desired type of internucleoside linkage;
e) optionally either reiterating the sequence of steps b) through d) or reiterating the sequence of steps b) and c) before executing step d); and
f) cleaving the oligonucleotide from the supporting moiety;
wherein the liquid composition C comprises an aprotic solvent, at least one alcohol selected from the group consisting of trifluoroethanol, hexafluoroisopropanol, pentafluoropropanol, 1,1,1,3,3,3-hexafluoro-2-methyl-2-propanol, and nonafluoro tertiary butyl alcohol, and a salt of a base with a strong acid, wherein the salt's cation has a pKa in the range of 1 to 4 and the strong acid has a pKa of less than 1.

In some embodiments of the method of the invention comprising the aforementioned steps a) through f), in at least one iteration of step a), the backbone hydroxyl group, which is protected by a DMT protecting group, is part of a nucleoside moiety comprising a purine type nucleobase, preferably a nucleobase selected from the group consisting of adenine and guanine, in particular adenine.

In some embodiments of the method of the invention comprising the aforementioned steps a) through f), step e) is performed and comprises reiterating the sequence of steps b) through d). In some embodiments of the method of the invention comprising the aforementioned steps a) through f), step e) is performed and comprises reiterating the sequence of steps b) through d) 4-99 times, 9-99 times, 14-99 times, 19-99 times, 19-49 times, 19-29 times, 4-22 times, or 4-19 times.

In some embodiments of the method of the invention comprising the aforementioned steps a) through f), step e) is performed and comprises reiterating the sequence of steps b) through d) 4-99 times, with the proviso that in at least one, at least 2, at least 3, at least 4, at least 5, at least 6, at least 7, at least 8, at least 9, at least 10, or at least 11 iterations of step b), the backbone hydroxyl group, from which the DMT protecting group is cleaved, is part of a nucleoside moiety comprising a purine type nucleobase, preferably a nucleobase selected from the group consisting of adenine and guanine, in particular adenine. In some embodiments of the method of the invention comprising the aforementioned steps a) through f), step e) is performed and comprises reiterating the sequence of steps b) through d) 9-99 times, with the proviso that in at least one, at least 2, at least 3, at least 4, at least 5, at least 6, at least 7, at least 8, at least 9, at least 10, or at least 11 iterations of step b), the backbone hydroxyl group, from which the DMT protecting group is cleaved, is part of a nucleoside moiety comprising a purine type nucleobase, preferably a nucleobase selected from the group consisting of adenine and guanine, in particular adenine. In some embodiments of the method of the invention comprising the aforementioned steps a) through f), step e) is performed and comprises reiterating the sequence of steps b) through d) 19-99 times, with the proviso that in at least one, at least 2, at least 3, at least 4, at least 5, at least 6, at least 7, at least 8, at least 9, at least 10, or at least 11 iterations of step b), the backbone hydroxyl group, from which the DMT protecting group is cleaved, is part of a nucleoside moiety comprising a purine type nucleobase, preferably a nucleobase selected from the group consisting of adenine and guanine, in particular adenine.

In some embodiments of the method comprising the aforementioned steps a) through f):
- step a) comprises providing a compound according to formula I 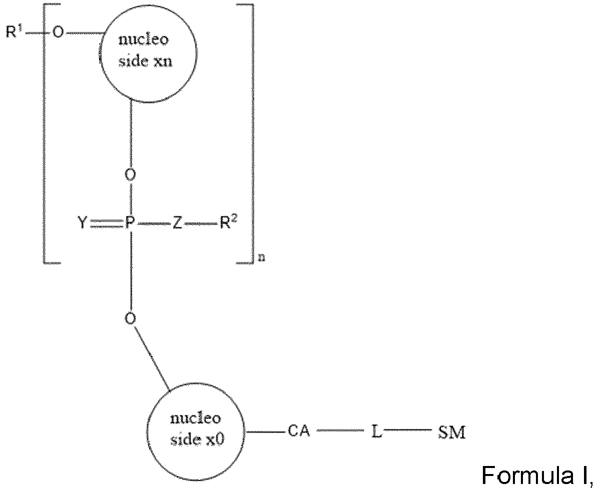 wherein:
   R¹ is a di(p-methoxyphenyl)phenylmethyl protecting group;
   n is an integer equal to or larger than 0;
   Y is selected independently for each repetitive unit n from the group consisting of S and O;
   Z is selected independently for each repetitive unit n from the group consisting of O and S;
   R² is a protecting group, which may be the same or different for each repetitive unit n;
   each of the nucleosides x0 to xn may be the same or different;
   CA is a capping moiety or single bond;
   L is a linker moiety or single bond; and
   SM is a supporting moiety;
- step c) comprises reacting the free hydroxyl group resulting from step b) with a nucleoside or oligonucleotide phosphoramidite building block, which building block comprises a backbone hydroxyl moiety protected by a di(p-methoxyphenyl)phenylmethyl protecting group, thereby producing a covalent linkage between the oxygen atom of said free hydroxyl group and the phosphorus (III) atom of said building block; and
- step e) comprises optionally reiterating the series of steps b) through d).

In some embodiments, the compound according to formula I is a compound according to the following formula I-a: 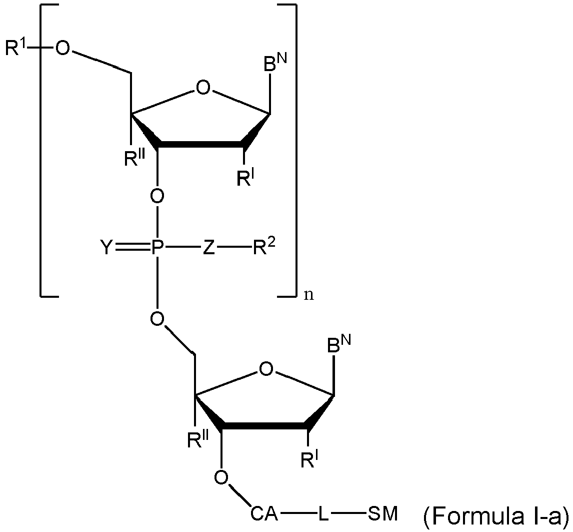 wherein
R¹ is a di(p-methoxyphenyl)phenylmethyl protecting group;
n is an integer equal to or larger than 0;
Y is selected independently for each repetitive unit n from the group consisting of S and O;
Z is selected independently for each repetitive unit n from the group consisting of O and S;
R² is a protecting group, which may be the same or different for each repetitive unit n;
CA is a capping moiety or single bond;
L is a linker moiety or single bond;
SM is a supporting moiety;
B^{N} is a nucleobase, which may be the same or different at each occurrence;
R^{I} is independently at each occurrence selected from the group consisting of H, F, -O-(C₁-C₅ alkyl), -O-(C₁-C₅ alkyl)-O-(C₁-C₅ alkyl), -O-Si(C₁-C₅ alkyl)₃, and -O-CH₂-O-Si(C₁-C₅ alkyl)₃; and
R^{II} is independently at each occurrence H or R^{II} and R^{I} together form a methylene or ethylene bridge of the chemical structure +-CH₂-O-++, +-CH(CH₃)-O-++, or +-CH₂-CH₂-O-++, where + is the point of attachment to the 4'-carbon atom (i.e. the carbon atom to which R^{II} is bonded) and ++ is the point of attachment to the 2'-carbon (i.e. the carbon atom to which R^{I} is bonded).

In some embodiments, Z in Formula I or I-a is for each repetitive unit n O.

In some embodiments, CA in Formula I and/or I-a is a single bond. In some embodiments, L in Formula I and/or I-a is a single bond. In some embodiments, CA and L in Formula I and/or I-a are a single bond. It will be understood that, if both CA and L in Formula I and/or I-a are a single bond, they together represent the same single bond linking the supporting moiety to the nucleoside x0.

In some embodiments, the integer n in Formula I and/or I-a is in the range of 0-150, 0-100, 0-75, 0-50, 0-35, 0-30, 0-20, 0-10, 4-30, 9-30, 9-25, or 9-20. It will be understood that, if the integer n in Formula I and/or I-a is 0, the protecting group R¹ is still comprised in the compound according to Formula I and/or I-a and is bonded to the oxygen atom of the nucleoside x0 which would engage in a covalent bond to the phosphorus atom of the first repetitive unit n, if n was not 0.

In some embodiments, wherein step a) comprises providing a compound according to Formula I and/or I-a, the terminal nucleoside xn which carries the -OR¹ group is a nucleoside comprising a purine type nucleobase, preferably nucleobase selected from the group consisting of adenine and guanine, in particular adenine.

For example, in one embodiment, the present invention relates to a method for synthesizing an oligonucleotide comprising the following steps a*) through f*):
a*) providing a compound according to formula I, 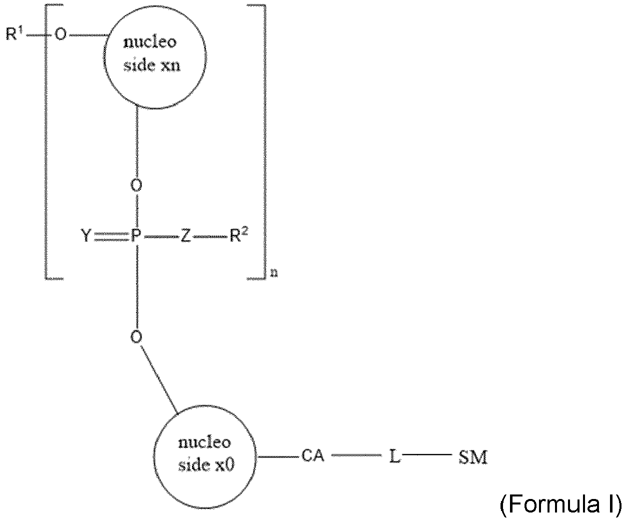 wherein:
   R¹ is a protecting group comprising an optionally substituted triarylmethyl residue;
   n is an integer equal to or larger than 0;
   Y is selected independently for each repetitive unit n from the group consisting of S and O;
   Z is selected independently for each repetitive unit n from the group consisting of O and S;
   R² is a protecting group, which may be the same or different for each repetitive unit n;
   each of the nucleosides x0 to xn may be the same or different;
   CA is a capping moiety or a single bond (i.e., CA may be missing);
   L is a linker moiety or a single bond (i.e., L may be missing); and
   SM is a supporting moiety;
b*) cleaving the protecting group comprising an optionally substituted triarylmethyl residue R1 from the compound according to formula I by incubating said compound with a liquid composition C;
c*) reacting the free hydroxyl group resulting from step b) with a nucleoside or oligonucleotide phosphoramidite building block, which building block comprises a backbone hydroxyl moiety protected by a protecting group comprising an optionally substituted triarylmethyl residue, thereby producing a covalent linkage between the oxygen atom of said free hydroxyl group and the phorsphorus (III) atom of said building block;
d*) oxidizing or sulfurizing said phosphorus (III) moiety to a phosphorus (V) moiety;
e*) optionally reiterating either the sequence of steps b) through d); and
f*) cleaving the oligonucleotide from the linker moiety;
wherein the liquid composition C comprises an aprotic solvent, at least one alcohol selected from the group consisting of trifluoroethanol, hexafluoroisopropanol, pentafluoropropanol, 1,1,1,3,3,3-hexafluoro-2-methyl-2-propanol, and nonafluoro tertiary butyl alcohol, and a salt of a base with a strong acid, wherein the salt's cation has a pKa in the range of 1 to 4.

It should be noted that the order of steps indicated herein may be altered and that steps indicated as optional may be omitted.

In some embodiments of the method comprising the aforementioned steps a) through f):
- step a) comprises providing a compound according to formula III 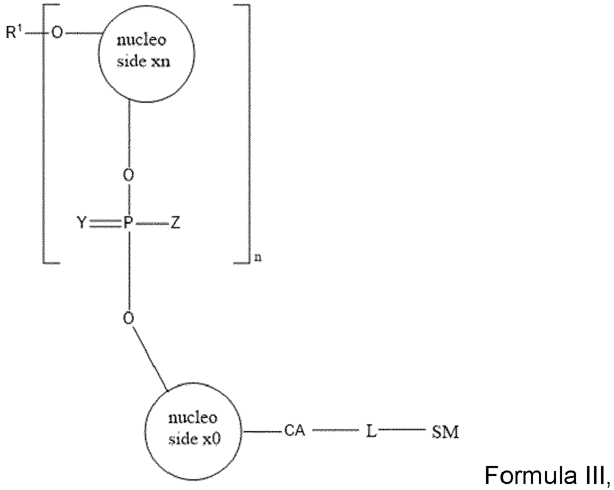 wherein:
   R¹ is a di(p-methoxyphenyl)phenylmethyl protecting group;
   Y is O; and
   Z is H;
   each of the nucleosides x0 to xn may be the same or different;
   CA is a capping moiety or a single bond;
   L is a linker moiety or a single bond; and
   SM is a supporting moiety;
- step c) comprises reacting the free hydroxyl group resulting from step b) with a H-phosphonate building block, which building block comprises a backbone hydroxyl moiety protected by a di(p-methoxyphenyl)phenylmethyl protecting group, thereby producing a covalent linkage between the oxygen atom of said free hydroxyl group and the phorsphorus (III) atom of said building block; and
- step e) comprises optionally assembling a desired oligonucleotide sequence by reiterating the sequence of steps b) and c) before executing step d).

In some embodiments, the compound according to formula III is a compound according to the following formula III-a: 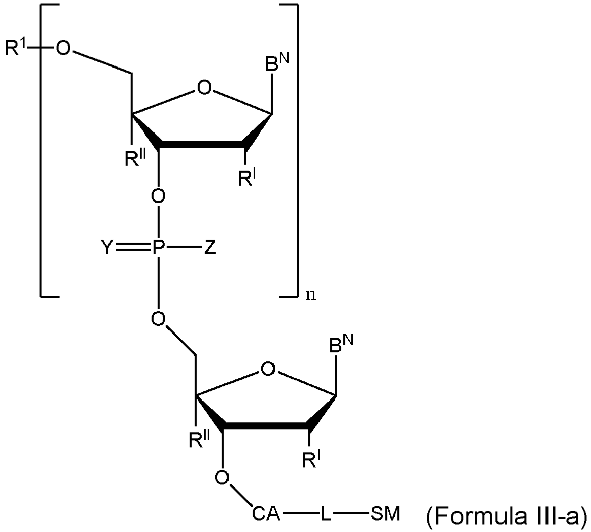 wherein
R¹ is a di(p-methoxyphenyl)phenylmethyl protecting group;
n is an integer equal to or larger than 0;
Y is O; and
Z is H;
CA is a capping moiety or single bond;
L is a linker moiety or single bond;
SM is a supporting moiety;
B^{N} is a nucleobase, which may be the same or different at each occurrence;
R^{I} is independently at each occurrence selected from the group consisting of H, F, -O-(C₁-C₅ alkyl), -O-(C₁-C₅ alkyl)-O-(C₁-C₅ alkyl), -O-Si(C₁-C₅ alkyl)₃, and -O-CH₂-O-Si(C₁-C₅alkyl)₃; and
R^{II} is independently at each occurrence H or R^{II} and R^{I} together form a methylene or ethylene bridge of the chemical structure +-CH₂-O-++, +-CH(CH₃)-O-++, or +-CH₂-CH₂-O-++, where + is the point of attachment to the 4'-carbon atom (i.e. the carbon atom to which R^{II} is bonded) and ++ is the point of attachment to the 2'-carbon (i.e. the carbon atom to which R^{I} is bonded).

In some embodiments, CA in Formula III and/or III-a is a single bond. In some embodiments, L in Formula III and/or III-a is a single bond. In some embodiments, CA and L in Formula III and/or III-a are a single bond. It will be understood that, if both CA and L in Formula III and/or III-a are a single bond, they together represent the same single bond linking the supporting moiety to the nucleoside x0.

In some embodiments, the integer n in Formula III and/or IIIa is in the range of 0-150, 0-100, 0-75, 0-50, 0-35, 0-30, 0-20, 0-10, 4-30, 9-30, 9-25, or 9-20. It will be understood that, if the integer n in Formula III and/or IIIa is 0, the protecting group R¹ is still comprised in the compound according to Formula III and/or IIIa and is bonded to the oxygen atom of the nucleoside x0 which would engage in a covalent bond to the phosphorus atom of the first repetitive unit n, if n was not 0.

In some embodiments, wherein step a) comprises providing a compound according to Formula III and/or Illa, the terminal nucleoside xn which carries the -OR¹ group is a nucleoside comprising a purine type nucleobase, preferably nucleobase selected from the group consisting of adenine and guanine, in particular adenine.

It will be understood that each nucleobase B^{N} in any of formulas I-a, and III-a may be the same or different at each occurrence and may optionally be protected, i.e. carry one or more protecting groups. A person skilled in the art is familiar with nucleobase protecting groups and knows how to select them for a specific synthesis. In particular, any exocyclic amino groups such as e.g. present in adenine, guanine, and cytosine may be protected. Table T-1 gives a non-limiting overview of commonly used protecting groups.

In some embodiments, in formula I-a and/or formula III-a:
B^{N} is selected independently at each occurrence from the group consisting of adenine, guanine, cytosine, thymine, and uracil;
R^{I} is selected independently at each occurrence from the group consisting of H, F, -O-CH₃ (i.e. methoxy), -O-CH₂-CH₂-O-CH₃ (i.e. 2-methoxyethyl-1-oxy), -O-Si(CH₃)₃ (i.e. trimethylsilyloxy), -O-Si(CH₃)₂(C(CH₃)₃) (i.e. *tert-*butyl(dimethyl)silyloxy), and -O-CH₂-O-Si(C(CH₃)₃)₃ (i.e. ((triisopropylsilyl)oxy)-methyloxy); and
R^{II} is H at each occurrence.

In some embodiments, in formula I-a and/or formula III-a:
B^{N} is selected independently at each occurrence from the group consisting of adenine, guanine, cytosine, thymine, and uracil;
R^{I} is selected independently at each occurrence from the group consisting of H, F, -O-CH₃ (i.e. methoxy); and
R^{II} is H at each occurrence.

In some embodiments, in formula I-a and/or formula III-a:
B^{N} is selected independently at each occurrence from the group consisting of adenine, in which the exocyclic amino group is benzoyl-protected, guanine, in which the exocyclic amino group is isobutyryl-protected, cytosine, in which the exocyclic amino group is benzoyl- or acetyl-protected, thymine, and uracil;
R^{I} is selected independently at each occurrence from the group consisting of H, F, -O-CH₃ (i.e. methoxy); and
R^{II} is H at each occurrence.

As another example, the present invention provides a method for synthesizing an oligonucleotide comprising the following steps a') through f'):
a') providing a compound according to formula III, 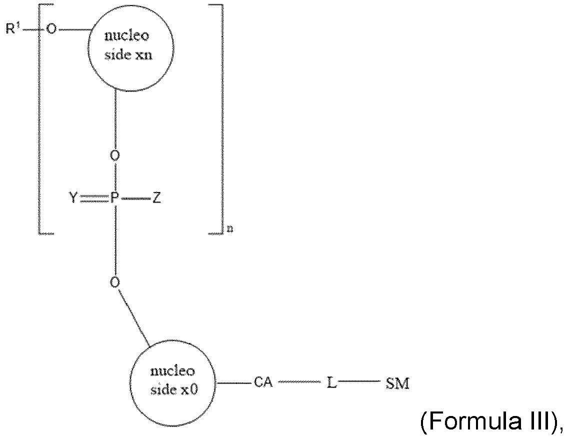 wherein:
   R¹ is a protecting group comprising an optionally substituted triarylmethyl residue;
   n is an integer equal to or larger than 0;
   Y is O and Z is H;
   each of the nucleosides x0 to xn may be the same or different;
   CA is a capping moiety or a single bond (i.e., CA may be missing);
   L is a linker moiety or a single bond (i.e., L may be missing); and
   SM is a supporting moiety;
b') cleaving the protecting group comprising an optionally substituted triarylmethyl residue from the compound according to formula III by incubating said compound with a liquid composition C;
c') reacting the free hydroxyl group resulting from step b') with a H-phosphonate building block, which building block comprises a backbone hydroxyl moiety protected by a protecting group comprising an optionally substituted triarylmethyl residue, thereby producing a covalent linkage between the oxygen atom of said free hydroxyl group and the phorsphorus (III) atom of said building block;
d') oxidizing or sulfurizing said phosphorus (III) moiety to a phosphorus (V) moiety;
e') optionally repeating steps b) and c) for subsequent couplings of additional building blocks prior to step d'); and
f') cleaving the oligonucleotide from the linker moiety;
wherein the liquid composition C comprises an aprotic solvent, at least one alcohol selected from the group consisting of trifluoroethanol, hexafluoroisopropanol, pentafluoropropanol, 1,1,1,3,3,3-hexafluoro-2-methyl-2-propanol, and nonafluoro tertiary butyl alcohol, and a salt of a base with a strong acid, wherein the salt's cation has a pKa in the range of 1 to 4.

As used herein, the expression oligonucleotide/s is used in a most general way to relate to any oligomers comprising at least two nucleosides linked by a phosphodiester bond or by an analogous structure as shown in formula A below and any isomeric forms and stereoisomers thereof. In a preferred embodiment, an oligonucleotide in the sense of the present invention comprises two or more nucleoside moieties conjugated with each other via a linker comprising or consisting of a structure according to Formula A or a salt thereof: 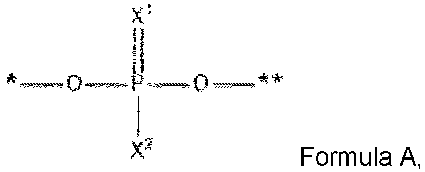 wherein, in Formula A:
* and ** each independently from another denotes a point of attachment to a nucleoside moiety each (i.e., the antecedent and following nucleoside moiety, respectively) or H (i.e., in case of a terminal end of the oligonucleotide);
X₁ is selected from S and O; and
X₂ can be any conceivable stable atom or functional group, preferably is selected from the group consisting of -OH, -OR, -NHR, a C₃-C₂₀-(hetero)aromatic moiety, -CH₃, -F, -Cl, -SH, -SeH, and -SR,
where R is any residue, preferably a residue not having more than 30 carbon atoms, preferably a residue selected from the group consisting of a branched, unbranched or cyclic C₁-C₂₀ (hetero)alkyl residue, a branched, unbranched or cyclic C₂-C₂₀ (hetero)alkenyl residue, a branched, unbranched or cyclic C₂-C₂₀ (hetero)alkinyl residue, a C₆-C₂₀ aryl residue, a C₃-C₁₉ heteroaryl residue, wherein the aforementioned residues are optionally substituted with deuterium, halogen, a branched, unbranched or cyclic C₁-C₂₀ (hetero)alkyl residue, a branched, unbranched or cyclic C₂-C₂₀ (hetero)alkenyl residue, a branched, unbranched or cyclic C₂-C₂₀ (hetero)alkynyl residue, a C₁-C₂₀ (hetero)cycloalkyl residue, a C₆-C₂₀ aryl residue, and/or a C₃-C₁₉ heteroaryl residue.

As used throughout the present invention, the terms "alkyl", "heteroalkyl" may be understood in the broadest sense. It may for instance be methyl or ethyl or isopropyl. A heteroalkyl may comprise at least one heteroatom such as, e.g. N, O, S or P. This may also embrace alkoxy (e.g., methoxy (-O-CH₃, -OMe)) and similar residues such as -NHCH₃ or -N(CH₃)₂ or salts thereof. Preferably, an alkyl is a branched, unbranched or cyclic C₁-C₂₀ (hetero)alkyl, optionally substituted with deuterium, halogen, a branched, unbranched or cyclic C₁-C₂₀ (hetero)alkyl residue, a branched, unbranched or cyclic C₂-C₂₀ (hetero)alkenyl residue, a branched, unbranched or cyclic C₂-C₂₀ (hetero)alkynyl residue, a C₁-C₂₀ (hetero)cycloalkyl residue, a C₆-C₂₀ aryl residue, and/or a C₃-C₁₉ heteroaryl residue.

As used throughout the present invention, the terms "alkenyl" and "heteroalkenyl" may be understood in the broadest sense any may have one, two or more double bonds. A heteroalkenyl may comprise at least one heteroatom such as, e.g. N, O, S or P. Preferably, an alkenyl is a branched, unbranched or cyclic C₁-C₂₀ (hetero)alkenyl, optionally substituted with deuterium, halogen, a branched, unbranched or cyclic C₁-C₂₀ (hetero)alkyl residue, a branched, unbranched or cyclic C₂-C₂₀ (hetero)alkenyl residue, a branched, unbranched or cyclic C₂-C₂₀ (hetero)alkynyl residue, a C₁-C₂₀ (hetero)cycloalkyl residue, a C₆-C₂₀ aryl residue, and/or a C₃-C₁₉ heteroaryl residue.

As used throughout the present invention, the terms "alkynyl" and "heteroalkynyl" may be understood in the broadest sense any may have one, two or more triple bonds. A heteroalkynyl may comprise at least one heteroatom such as, e.g. N, O, S or P. Preferably, an alkynyl is a branched, unbranched or cyclic C₁-C₂₀ (hetero)alkynyl, optionally substituted with deuterium, halogen, a branched, unbranched or cyclic C₁-C₂₀ (hetero)alkyl residue, a branched, unbranched or cyclic C₂-C₂₀ (hetero)alkenyl residue, a branched, unbranched or cyclic C₂-C₂₀ (hetero)alkynyl residue, a C₁-C₂₀ (hetero)cycloalkyl residue, a C₆-C₂₀ aryl residue, and/or a C₃-C₁₉ heteroaryl residue.

As used throughout the present invention, the terms "aryl" and "heteroaryl" may be understood in the broadest sense. A heteroaryl may comprise at least one heteroatom such as, e.g. N, O, S and/or P, preferably in the cyclic structure.

The type of internucleosidic linkage is not necessarily the same in all positions of an oligonucleotide. For example, the middle of the oligonucleotide strand may comprise phosphodiester bonds, and phosphorothioate bonds may be present at the extremities of the strand.

It is clear from formula A that each nucleoside comprises two hydroxyl moieties (i.e., hydroxyl groups or moieties derived from hydroxyl groups, typically involved in ester bonds linking the residue of formula A with the nucleotide moieties), which are involved in internucleosidic linkages, namely in phosphoester type linkages with a phosphor (V) moiety located between adjacent nucleosides. One of the hydroxyl moieties is involved in the internucleosidic linkage to the antecedent nucleoside, the other hydroxyl moiety is involved in the internucleosidic linkage to the following nucleoside. Therefore, these hydroxyl moieties are referred to as "backbone hydroxyl moiety/moieties" or "backbone hydroxyl group/groups" throughout this text.

Obviously, the very first and the very last nucleoside unit are involved in only one internucleosidic linkage.

They may therefore comprise a free backbone hydroxyl group (e.g. the so-called 5' OH group and the 3' OH group in case of a phosphoribose backbone as is found in DNA and RNA), or the hydroxyl moiety may be linked to another moiety, e.g. to a capping structure.

Herein, the terms "internucleosidic linkage" and "internucleoside linkage" are used interchangeably.

Oligonucleotides may be conjugated to additional moieties for various purposes. Conjugation may be through the terminal backbone hydroxyl groups or via other functional groups of nucleoside moieties, which may be located at the ends or within the oligonucleotide strand. For example, the 5' OH group of the antisense strand of siRNA may be modified by vinyl phosphonate to inhibit cellular degradation and improve potency. A further example is conjugation to N-acetylgalactosamine (GalNAc), which is of interest for targeted oligonucleotide delivery to hepatocytes. GalNac structures, often branched to accommodate 3 or 4 GalNac moieties, may be conjugated, e.g., to the 5' OH group of an oligonucleotide (cf., e.g., WO2016055601), to the 3' OH group (cf., e.g., WO2009073809), or monovalent GalNac moieties may be conjugated via a linker to the 2' position of subsequent ribose moieties within the oligonucleotide strand (cf., e.g., WO2019075419).

Nucleoside units may be glycosylamines comprising a sugar moiety, commonly a ribose moiety, and a nucleobase. As used herein, the expression nucleoside or nucleoside unit encompasses naturally occurring nucleosides as well as non-natural compounds, where the ribose moiety and/or the nucleobase has been modified or replaced by a functional equivalent or an abasic site. Examples of natural nucleosides comprise, but are not limited to, adenosine, guanosine, cytidine, ribothymidine, uridine, desoxyuridine, desoxythymidine, inosine, desoxyadenosine, desoxyguanosine, desoxycytidine, and methylated derivatives thereof. Further examples are queusine acheaeosine, wybutosine, lysidine, and N⁶-threonylcarbamoyladenosine. Non-natural nucleosides may comprise altered ribose moieties, which may, e.g., be "locked" (i.e. comprise a methylene bridge connecting the 2' oxygen and 4' carbon), exhibit ethylene bridges, or carry -F, -OMe (-O-CH₃), and/or 2-methoxyethyl-1-oxy (-O-CH₂-CH₂-O-CH₃, aka. MOE, -O-methoxyethyl) substituents. Further, the ribose moiety may be replaced by a functional equivalent, e.g. by another pentose such as arabinose, a hexose such as mannose, or a glycerol moiety. As used herein, the expression nucleobase encompasses both non-natural nucleobases and naturally occurring nucleobases such as, e.g., adenine, guanine, uracil, cytosine, and thymine.

Non-natural nucleobases are functional equivalents of natural nucleobases in that they are capable of a specific interaction with a complementary nucleobase. The interaction between complementary nucleobases may be mediated by hydrogen bonds, which is known as Watson-Crick base pairing. Such non-natural nucleobases may be derivatives of purine or pyrimidine, which are capable of a specific interaction with another nucleobase. Herein nucleobases derived from purine are also referred to as purine type nucleobases and nucleobases derived from pyridimide are also referred to as pyrimidine nucleobases. Adenine and guanine are naturally occurring purine type nucleobases. Cytosine, thymine, and uracil are naturally occurring pyrimidine type nucleobases.

As used herein, the term "oligonucleotide" and "polynucleotide" may be understood interchangeably. It follows from the above explanations that non-limiting examples of oligonucleotides are deoxynucleic acids (DNA), ribonucleic acids (RNA), locked nucleic acids (LNA), constrained ethyl nucleic acid analogs (cEt), bridged nucleic acids (BNA), tricycloDNA, unlocked nucleic acids (UNA), small interfering RNA (siRNA), microRNA, antisense oligonucleotides (ASO), gapmers, glycerol nucleic acids, oligonucleotide phosphorothioates, phosphorodithioates, diastereomerically pure phosphorothioates, as well as derivatives and analogs thereof. The expression "oligonucleotide" further comprises conjugates of an oligonucleotide moiety with other moieties such as peptides, carbohydrates, and the like. Particular interesting examples of oligonucleotides are phosphorthioates built from nucleosides comprising a modified ribose moiety, e.g. with 2' substituents selected from - F, -OMe (-O-CH₃), or 2-methoxyethyl-1-oxy (-O-CH₂-CH₂-O-CH₃, aka. MOE, -O-methoxyethyl substituents), and a nucleobase capable of Watson-Crick base pairing with a natural target sequence. The skilled artisan is well aware of the fact that the above moieties may have numerous stereoisomers, all of which are encompassed in the above definitions. An "oligonucleotide" as used herein, may be a population of oligonucleotide molecules having essentially the same sequence, which is a mixture of numerous discrete stereoisomers, or which is enriched in one specific stereoisomeric form, or which essentially consists of one specific stereoisomeric form.

It will be understood by a person skilled in the art that an oligonucleotide as used herein may optionally bear any counter ions known in the art, such as anions or cations, such as e.g., chloride ions, acetate ions, carbonate ions, hydrocarbonate ions, sodium ions, potassium ions, magnesium ions, trifluoroacetic acid (i.e. trifluoroacetate TFA) ions, bromide ions, perchlorate ions, ammonium ions and/or cations or anions of residuals of protecting groups. Further, an oligonucleotide may optionally be covalently or non-covalently associated to traces of one or more scavengers, such as, e.g., triisopropylsilane (TIS), dithiothreitol (DTT), anisole, thioanisole or 1,2-ethanedithiol.

As used herein, the expression "nucleoside building block" refers to mono- or oligomeric building blocks allowing to assemble an oligonucleotide strand. The skilled person will readily understand that basically any coupling chemistry compatible with the use of a temporary protecting group comprising an optionally substituted triarylmethyl residue, preferably a DMT protecting group, may be used in the methods of the present invention and that the chemical nature of the phosphorous moiety may consequently vary. In some embodiments, the building block may comprise a H-phosphonate moiety. In other embodiments, the building block may be a phosphorous V reagent as taught in, e.g., WO 2019200273. In yet other embodiments, the building block may be a phosphoramidite building block of the general structure given in formula II: 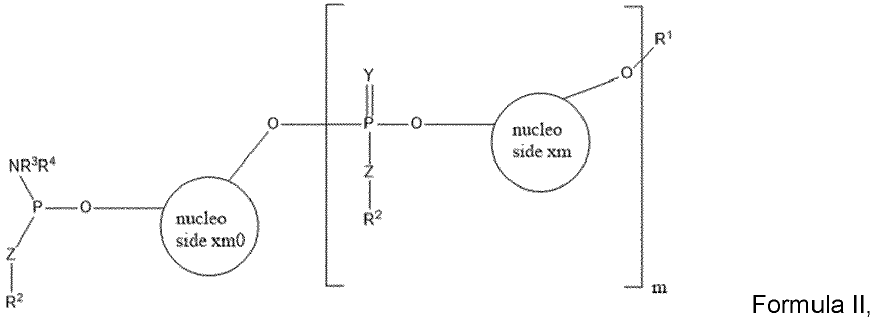 wherein:
R¹ is a protecting group comprising an optionally substituted triarylmethyl residue, preferably a DMT protecting group;
m is an integer equal to or larger than 0;
Y is selected independently for each repetitive unit m from the group consisting of S and O;
Z is selected independently for each position (i.e. for the terminal position and for each repetitive unit m) from the group consisting of O and S;
R² is a protecting group, which may be the same or different for each position (i.e. for the terminal position and each repetitive unit m);
R³ and R⁴ are protecting groups, which may be the same or different; and
each of the nucleosides xm0 to xm may be the same or different.

In some embodiments of the method of the invention, wherein said method comprises the aforementioned steps a) through f), the nucleoside or oligonucleotide building block (of step c)) is a compound according to formula II 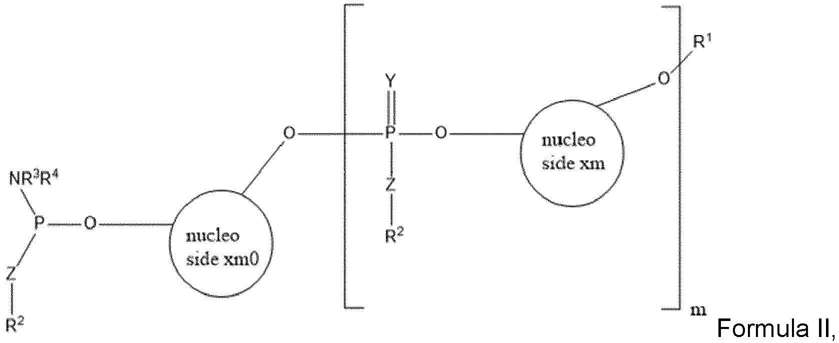 wherein:
R¹ is a di(p-methoxyphenyl)phenylmethyl protecting group ;
m is an integer equal to or larger than 0;
Y is selected independently for each repetitive unit m from the group consisting of S and O;
Z is selected independently for each position (i.e. for the terminal position and for each repetitive unit m) from the group consisting of O and S;
R² is a protecting group, which may be the same or different for each position;
R³ and R⁴ are protecting groups, which may be the same or different; and
each of the nucleosides xm0 to xm may be the same or different.

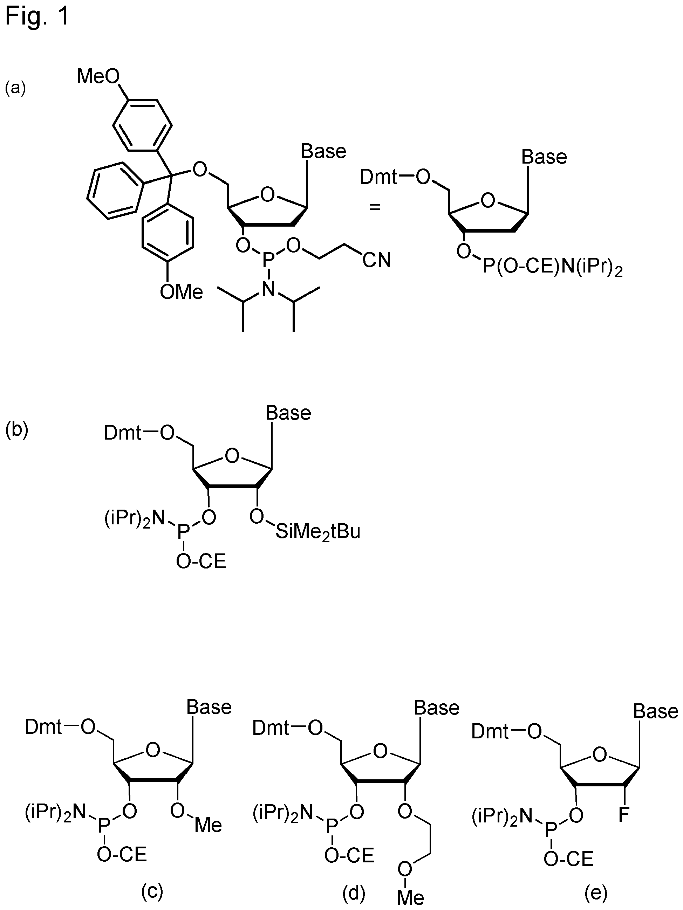

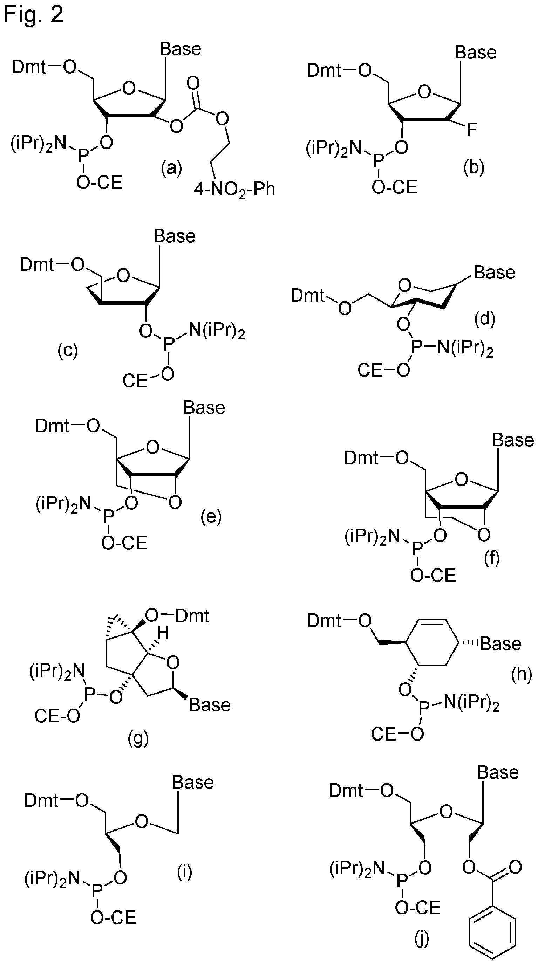

Specific, non-limiting examples of mononucleoside phosphoamidite building blocks are given in Figures 1 and 2. It should be noted that the nucleosides xm0 to xm and x0 to xn in formulae I, II, and III may differ from each other with respect to the identity and substitution pattern of the ribose or ribose analog moiety (aka. ribose surrogate), with respect to the nucleobase, and with respect to the presence and identity of any protecting groups.

In some embodiments, the compound according to formula II is a compound according to the following formula II-a: 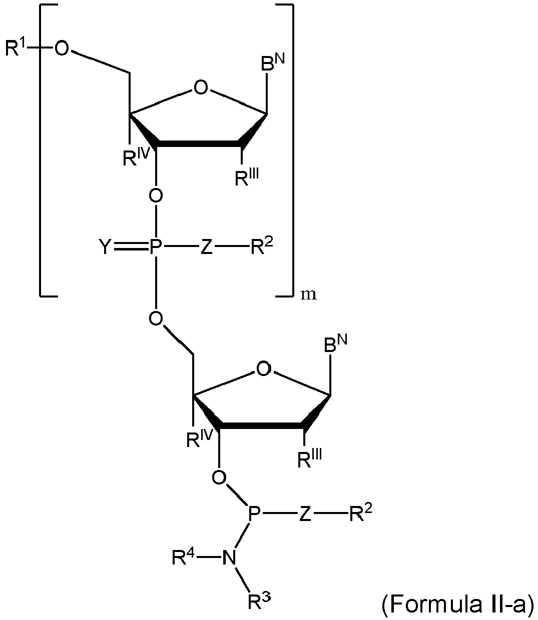 wherein
R¹ is a di(p-methoxyphenyl)phenylmethyl protecting group;
m is an integer equal to or larger than 0;
Y is selected independently for each repetitive unit m from the group consisting of S and O;
Z is selected independently for each position from the group consisting of O and S;
R² is a protecting group, which may be the same or different for each position;
R³ and R⁴ are protecting groups, which may be the same or different;
B^{N} is a nucleobase, which may be the same or different at each occurrence;
R^{III} is selected independently at each occurrence from the group consisting of H, F, -O-(C₁-C₅ alkyl), -O-(C₁-C₅ alkyl)-O-(C₁-C₅ alkyl), -O-Si(C₁-C₅ alkyl)₃, and -O-CH₂-O-Si(C₁-C₅ alkyl)₃; and
R^{IV} is independently at each occurrence H or R^{III} and R^{IV} together form a methylene or ethylene bridge of the chemical structure +-CH₂-O-++, +-CH(CH₃)-O-++, or +-CH₂-CH₂-O-++, where + is the point of attachment to the 4'-carbon atom (i.e. the carbon atom to which R^{IV} is bonded) and ++ is the point of attachment to the 2'-carbon (i.e. the carbon atom to which R^{III} is bonded).

In some embodiments, Z in Formula II and/or II-a is O for each position (i.e. for the terminal position and for each repetitive unit m).

In some embodiments, different types of building blocks with different coupling chemistry may be used in different coupling cycles. For example, phosphorothioate bonded nucleosides may be introduced using P(V) chemistry, and phosphodiester bonded nucleosides may be introduced using P(III) chemistry.

In some embodiments, in Formula II and/or II-a, m is an integer between 0 and 2, i.e. the building blocks are mononucleoside, dinucleotide, or trinucleotide phosphoramidite building blocks. It will be understood that, if the integer m in Formula II and/or II-a is 0, the protecting group R¹ is still comprised in the compound according to Formula II and/or II-a and is bonded to the oxygen atom of the nucleoside xm0 which would engage in a covalent bond to the phosphorus atom of the first repetitive unit m, if m was not 0.

It will be understood that each nucleobase B^{BN} in formula III-a may be the same or different at each occurrence and may optionally be protected, i.e. carry one or more protecting groups. A person skilled in the art is familiar with nucleobase protecting groups and knows how to select them for a specific synthesis. In particular, any exocyclic amino groups such as e.g. present in adenine, guanine, and cytosine may be protected. Table T-1 gives a non-limiting overview of commonly used protecting groups.

In some embodiments, in formula II-a:
B^{N} is selected independently at each occurrence from the group consisting of adenine, guanine, cytosine, thymine, and uracil;
R^{III} is selected independently at each occurrence from the group consisting of H, F, -O-CH₃ (i.e. methoxy), -O-CH₂-CH₂-O-CH₃ (i.e. 2-methoxyethyl-1-oxy), and -O-Si(CH₃)₃ (i.e. trimethylsilyloxy), -O-Si(CH₃)₂(C(CH₃)₃) (i.e. *tert-*butyl(dimethyl)silyloxy), and -O-CH₂-O-Si(C(CH₃)₃)₃ (i.e. ((triisopropylsilyl)oxy)-methyloxy); and
R^{IV} is H at each occurrence.

In some embodiments, in formula II-a:
B^{N} is selected independently at each occurrence from the group consisting of adenine, guanine, cytosine, thymine, and uracil;
R^{III} is selected independently at each occurrence from the group consisting of H, F, -O-CH₃ (i.e. methoxy); and
R^{IV} is H at each occurrence.

In some embodiments, in formula II-a:
B^{N} is selected independently at each occurrence from the group consisting of adenine, in which the exocyclic amino group is benzoyl-protected, guanine, in which the exocyclic amino group is isobutyryl-protected, cytosine, in which the exocyclic amino group is benzoyl- or acetyl-protected, thymine, and uracil;
R^{III} is selected independently at each occurrence from the group consisting of H, F, -O-CH₃ (i.e. methoxy); and
R^{IV} is H at each occurrence.

The term "protecting group" as used herein may be understood in the broadest sense as a group which is introduced into a molecule by chemical modification of a functional group to block said group from reaction in subsequent process steps, e.g. to prevent side reactions at the backbone or the nucleobases of the oligonucleotide. A typical example for a suitable protecting group R² of formulae I, I-a, II, and II-a is the 2-cyanoethyl group; and a typical example for both R³ and R⁴ of formula II is the isopropyl group. Thus, in some preferred embodiments, R² of Formula I and/or I-a is at each position (i.e. for each repetitive unit n) a 2-cyanoethyl group. In some preferred embodiments, R² of Formula II and/or II-a is at each position (i.e. at each occurrence) a 2-cyanoethyl group. In some preferred embodiments, R³ and R⁴ of Formula II and/or II-a are independently of each other a C₁-C₆ alkyl group, more preferably R³ and R⁴ are both isopropyl groups. Alternatively, in a compound according to Formula II and/or II-a R² and R³ may form an ethylene bridge and the phosphoramidite may take the form of a chiral group of formula B, where ** denotes the point of attachment to the backbone hydroxyl group.

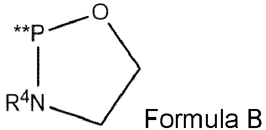

The building blocks used in the methods of the present invention may exhibit further protecting groups, e.g. to block the extracyclic amino groups of the nucleobases from side reactions. Suitable nucleoside derivatives are commercially available. Table T-1 gives a non-limiting overview of commonly used protecting groups for nucleoside building blocks.

**Table T-1: non-limiting overview of commonly used protecting groups for nucleoside building blocks.**

| Protecting group | Often used for |
|---|---|
| Bz = benzoyl; iBu = isobutyryl; | exocyclic amino group of adenine |
| PAC = phenoxyacetyl | |
| Ac = acetyl; Bz = benzoyl | exocyclic amino group of cytosine |
| iBu = isobutyryl; | exocyclic amino group of guanine |
| i-Pr-PAC = 4-isopropylphenoxyacetyl); | |
| dimethylformamidino | |
| TBDMS = t-butyldimethylsilyl; | protection of 2' OH-group |
| TOM = tri-isopropylsilyloxymethyl | |

Of particular interest for the present invention are protecting groups each comprising an optionally substituted triarylmethyl residue, which are being used as temporary hydroxyl protecting groups to avoid multiple insertions of the same nucleoside or oligonucleotide building block. The skilled person is well aware of the fact that the properties of such protecting groups each comprising an optionally substituted triarylmethyl residue may differ depending on their substitution pattern. Substituents, which stabilize the carbocation leaving group, may facilitate cleavage, such that the corresponding protecting group may be cleaved under less acidic conditions. The present inventions may preferably be used with protecting groups each comprising an optionally substituted triarylmethyl residue comprising at least one substituent, which exerts a carbocation stabilizing effect, for instance at least one substituent, which exerts a carbocation stabilizing inductive effect and/or a carbocation stabilizing mesomeric effect. For example, the triaryl methyl protecting group may comprise at least one substituent selected from the group consisting of methoxy groups and halogen atoms.

Preferred protecting groups each comprising an optionally substituted triarylmethyl residue comprise the p-methoxyphenyldiphenyl-methyl (MMT) group, the di(p-methoxyphenyl)phenylmethyl (DMT) group, the tri(p-methoxyphenyl)methyl ether (TMT) group, the 4,4'-dimethoxy-3"-[N-(imidazolylmethyl)]trityl (IDT) group, the 4,4'-dimethoxy-3"-[N-(imidazolylethyl)carbamoyl]trityl ether (IET) group, the bis(4-methoxyphenyl)-1'-pyrenylmethyl (Bmpm) group, and the 4-(17-tetrabenzo[a,c,g,i]fluorenylmethyl)-4',4"-dimethoxytrityl (Tbf-DMT) group. The DMT group is particularly preferred. The protecting group comprising an optionally substituted triarylmethyl residue may be the same or different for different building blocks used in a method according to the present inventions.

Herein, the process of cleaving off a protecting group comprising an optionally substituted triarylmethyl residue may be referred to as "detritylation".

Step a) of the methods described herein comprises providing a nucleoside or oligonucleotide bound to a supporting moiety. This may be understood in the broadest possible sense. For example, nucleoside loaded solid supports for many standard nucleosides may simply be acquired from commercial suppliers such as Merck and Cytiva, among many others. Alternatively, appropriately protected nucleosides may be loaded to a given supporting moiety. The skilled person is aware of a plethora of possible immobilization strategies for this purpose and will routinely select one. Generally speaking, the first nucleoside (referred to as nucleoside x0 in Formulae I and III) may be immobilized to the supporting moiety in any conceivable way, unless indicated differently in the context of specific embodiments. For example, the first nucleoside may be covalently linked via one of the backbone hydroxyl groups, or via another functional group located in the ribose/ribose analogue moiety or in the nucleobase. It should be noted that if step a) comprises providing an oligonucleotide bound to the supporting moiety, this is not limited to embodiments where the oligonucleotide is bound via one of its terminal nucleosides to the supporting moiety, unless indicated differently in the context of specific embodiments. However, as many methods of oligonucleotide synthesis involve addition of the following building block to the 5' end of the phosphoribose backbone, immobilization via the 3' end is particularly common.

Linker moieties may be used to establish the linkage between the supporting moiety and the nucleoside. As used herein, the expression "linker moiety" refers to a moiety, which exhibits at least two functional groups, wherein one of said groups is capable of binding to a functional group on the supporting moiety and the other is capable of binding to a functional group on the nucleoside. As one example, a succinic acid linker or a hydroquinone diacetate linker may mediate the linkage of a nucleoside's hydroxyl group (e.g. the 3' hydroxyl group of the ribose moiety) to an amino group present on a supporting moiety (e.g. an aminomethyl group of a polymer resin obtained from co-polymerization of poly(styrene) with divinylbenzene). Other commonly used linkers are of the so-called universal linkers, e.g. the "Unylinker" type described in US7202264. If a supporting moiety modified with a linker such as said universal linker is to be used, step a) of the present methods may comprise loading the first nucleoside or an oligonucleotide to the supporting moiety via the universal linker.

At the end of strand assembly, the newly synthesized oligonucleotide is cleaved from the supporting moiety, i.e. the bond between the oligonucleotide and the supporting moiety or -where applicable- between the oligonucleotide and the linker is broken to set the oligonucleotide free (step f)). However, in some cases, it may be desired to attach a capping moiety to the first nucleoside. This may be achieved by including the capping moiety between the supporting moiety or -where applicable- between the linker and the first nucleoside. In other words: the capping moiety differs from the linker moiety in that it is not removed from the oligonucleotide during final cleavage of the oligonucleotide from the supporting moiety. Therefore, as used herein, the expression "capping moiety" refers to any moiety, which is conjugated to a ultimate nucleoside of the oligonucleotide strand. The capping moiety is comprised in the final oligonucleotide product. In case the capping moiety is present between the first nucleoside and the linker moiety or between the first nucleoside and the supporting moiety, it may be attached to the 3' end of the oligonucleotide. An example for such a strategy is the insertion of a 3' GalNac conjugate as described in, e.g., WO2009073809.

Any supporting moiety known in the art may be used with the present invention. As used herein, the term "supporting moiety" refers to any macroscopic or molecular structure, to which the oligonucleotide to be synthesized can be tethered and which enables separation of the oligonucleotide to be synthesized from a liquid phase. Hence, the term encompasses the terms "resin" and "[resin]", which may be understood in the broadest sense as a particulate structure usable for solid phase oligonucleotide synthesis. The terms "resin", "solid support" and "solid phase" may be used interchangeably herein. For solid phase oligonucleotide synthesis, the supporting moiety may be an insoluble, solid or gel-like substrate. Widely used examples of suitable solid supports are controlled pore glass (CPG), e.g. long-chain alkylamine-CPG bead supports, crosslinked polystyrene beads, and crosslinked polystyrene-PEG composites, e.g. Tentagel^{™} resins. Step a) of the present methods may involve conditioning the solid support by washing steps with various solvents. In some embodiments of the method of the invention comprising the aforementioned steps a) through f), the supporting moiety is a solid support (and thus, the method is a method for the solid phase synthesis of oligonucleotides).

A person skilled in the art knows how to cleave an oligonucleotide (i.e. the fully assembled oligonucleotide chains) from the support (i.e. the supporting moiety) after support assisted synthesis. The terms support and supporting moiety are herein used interchangeably. Typically, such cleavage (as in step f) of the method of the invention) may be achieved by treating the support-bound oligonucleotide with a base such as an organic amine or alkali hydroxides, wherein concentrated aqueous ammonia (i.e. aqueous ammonium hydroxide solution) is most common and herein most preferred. This base (e.g. ammonia) treatment may be performed at room temperature or under heating, for example to 40-60 °C, e.g. in an autoclave or sealed vessel. Under such alkaline conditions, the typical nucleobase protecting groups will be cleaved as well. As non-limiting, but common examples, any isobutyryl groups from the exocyclic amino group of guanine, any benzoyl groups from the exocyclic amino group of adenine, and any benzoyl or acetyl groups from the exocyclic amino group of cytosine will typically be cleaved (i.e. removed) under such alkaline conditions. The protecting groups R² of Formula I, I-a, II, and II-a will typically also be selected so that they may be cleaved (i.e. removed) under alkaline conditions, and thus during the cleavage step f). R² being the 2-cyanoethyl protecting group is a prime example for this strategy. Any 2-cyanoethyl protecting groups R² may be removed in the course of the base treatment to effect cleavage from the support. However, as known to those skilled in the art, the cleavage step f) may comprise subsequent treatments with different types of bases. First, a solution of an organic amine such as diethylamine or trimethylamine, e.g. in a suitable solvent such as acetonitrile, may be used to remove the 2-cyanoethyl protecting groups, preferably at room temperature, followed by treatment with concentrated aqueous ammonia to effect cleavage from the support and removal of base labile permanent protecting groups such as the nucleobase protecting groups.

Further, the term "supporting moiety" encompasses any molecular tags allowing for separation of the tag with oligonucleotide to be synthesized tethered to it from a liquid phase. For liquid phase synthesis, the supporting moiety may be a solubility modifying tag, as is disclosed in, e.g., WO2012157723. Such supporting moieties may also be referred to as pseudo solid phase protecting groups. They essentially function to modify the solubility of the growing oligonucleotide strand by attaching a hydrophobic moiety. This enables separation of the growing oligonucleotide strand from other reagents by simple precipitation or extraction steps. In some embodiments of the method of the invention comprising the aforementioned steps a) through f), the supporting moiety is a pseudo solid phase protecting group (and thus, the method is a method for the liquid phase synthesis of oligonucleotides).

Based on the foregoing, it will be clear to the skilled artisan that the term "supporting moiety" as used herein refers to a population of entities, e.g. of bead-like particles or of molecular tags, and is used in the singular form only for the sake of readability.

The method steps c), i.e. the coupling of the building block to the growing oligonucleotide strand, and d), i.e. the oxidation or sulfurization of the phosphorous (III) moiety, depend on coupling chemistry chosen and will be routinely carried out by the skilled artisan. In order to avoid the formation of difficult-to-remove deletion sequences, the methods of the invention may comprise a further step of blocking (also referred to as capping) unreacted free hydroxyl groups after step c) or step d). In some embodiments, the method of the invention comprising the aforementioned steps a) through f) further comprises a step g) of blocking unreacted free hydroxyl groups after step c) or after step d). In these embodiments, step g) will be performed after at least one iteration of a step c) or a step d), but typically not after both step c) and step d) of the same elongation cycle (i.e. coupling cycle). Thus, in some embodiments, the further step g) is performed after at least one iteration of step c) or step d). In some embodiments, the further step g) is performed at least once, preferably exactly once, in each repetition of steps b) to c) or b) to d). In some embodiments, the further step g) is performed after each iteration of step c). In some embodiments, the further step g) is performed after each iteration of step d). It should be noted that the order of steps indicated in the claims and the description of the present methods is not necessarily binding, unless indicated differently. Moreover, the present methods may comprise further steps. For example, the assembled oligonucleotide moiety may be conjugated to another moiety before executing cleavage step f) of the present methods. Further, the skilled artisan may use intervening washing steps, e.g. when the composition of solvent changes between process steps, or in order to remove traces of reagents from a previous method step. As another example, step f) of the method of the invention may be preceded by a final (and isolated, i.e. not part of an elongation cycle) step b) to remove the 5'-terminal hydroxyl protecting group R¹ derived from the nucleoside or oligonucleotide building block of the final iteration of step c). This final R¹ removal may also be performed after step f).

In step b) of the methods disclosed herein, cleavage of the temporary protecting group from the backbone hydroxyl group is carried out by incubating the nucleoside or oligonucleotide compound with a liquid composition C. As used herein, the expression "composition C" relates to the cleavage composition comprising the specified ingredients. The reference sign "C" is added to improve readability and is not to be construed as implying any limitation.

Cleavage of the temporary protecting group comprising an optionally substituted triarylmethyl residue will normally be carried out at ambient temperature, e.g. at a temperature of between 15 and 35°C. However, it is to be understood that the reaction may be carried out at any suitable temperature, e.g. at a temperature of between 0°C and 90°C. Incubation with the liquid composition C is not particularly time sensitive and may be carried out for, e.g, at least 5 min, 10 min, 20 min, or 30 min. The incubation may involve mixing the liquid composition C with the compound to be deprotected in a stirred bed reactor, a sparged bed reactor (where agitation is achieved by passing nitrogen gas through the reaction solution), in a batch reactor, or circulating the liquid composition C through a column reactor for the time needed. The cleavage step may be reiterated with fresh composition C several times.

It is a particular advantage of the present invention that the methods of oligonucleotide synthesis can be scaled up without a need to increase the maximal flow rate of the liquid handling system. Furthermore, compared to the standard deprotection protocol using 10% dichloroacetic acid (DCA), the methods of the present invention allow to increase batch size by a factor of at least 5 fold, preferably as least 10, 20, 30, or 40 fold, while using the same liquid handling system for supplying liquid to and draining liquid from the reaction vessel. For the sake of clarity, it is noted that the reaction vessel may be changed to accommodate the larger amount of reagents.

The methods of the present application may in particular be used at a scale of above 0.1, 0.2, 0.3, 0.4, 0.5, 0.6, 0.7, 0.8, 0.9, 1.0, 2, 3, 4, 5, 6, 7, 8, 9, 10, 11, 12, 13, 14, 15, 20, 25, 30, or 35 mol. This means that the maximum theoretical amount of final product expected is at least 0.1, 0.2, 0.3, 0.4, 0.5, 0.6, 0.7, 0.8, 0.9, 1.0, 2, 3, 4, 5, 6, 7, 8, 9, 10, 11, 12, 13, 14, 15, 20, 25, 30, or 35 mol for one single synthesis process. The theoretical amount of final product expected may be calculated based on the molar amount of starting nucleoside or oligonucleotide bound to the supporting moiety, which is provided in step a), under the assumption that all subsequent process steps are 100% efficient (i.e. proceed with quantitative yield of the desired product). In some embodiments of the method of the invention comprising the aforementioned steps a) through f), the synthesis is carried out at a scale of 100 mmol oligonucleotide product or above.

Solid phase oligonucleotide synthesis is commonly carried out using column reactors packed with a solid support, to which the growing oligonucleotide chain is tethered. The column reactors may typically comprise a bottom frit and a top frit, with the solid support packed between. The size and dimensions of the reaction vessel can be chosen according to the scale of synthesis intended. For example, column reactors with a minimal inner volume of 5, 10, 30, 40, 50, 60, 70, 75, 80, 90, 100, 110, 120, 140, 150, 160, 170, 180, 200, 220, 240, 260, 280, or 300 liters may be used. In certain embodiments, column reactors with a maximal inner volume of 1 to 500 liters, of 5 to 450 liters, of 10 to 400 liters, of 50 to 300 liters, or 100 to 300 liters may be used. The present inventors surprisingly found that the flow rate of the liquid composition C through the column reactor may optionally be rather low, e.g. below 300 cm/h, below 290 cm/h, below 280 cm/h, below 270 cm/h, 260 cm/h, below 250 cm/h, below 240 cm/h, below 230 cm/h, 220 cm/h, below 210 cm/h, below 200 cm/h, below 190 cm/h, below 180 cm/h, or below 160 cm/h. This increases the scalability of the process drastically. Thus, in some embodiments of the method of the invention comprising the aforementioned steps a) through f), said method is carried out in a column reactor and the flow rate of the liquid composition C through the column reactor is below 300 cm/h in at least one iteration of step b). In some embodiments of the method of the invention comprising the aforementioned steps a) through f), said method is carried out in a column reactor and the flow rate of the liquid composition C through the column reactor is below 300 cm/h in each iteration of step b).

As an alternative, the inventors found that a batch reactor may be used with the methods of the present invention. In one embodiment, the batch reactor may be a stirred bed reactor. Such a reactor may comprise a mixing device and a bottom frit or filter cloth to retain the support inside the reactor when e.g. draining solvents and dissolved components. As further examples, batch reactors with a maximal inner volume of 1 to 1000 liters, of 5 to 650 liters, of 5 to 300 liters, of 10 to 600 liters, of 50 to 500 liters, or more than 1000 liters may be used. As further examples, batch reactors with a minimal inner volume of 5, 10, 30, 40, 50, 60, 70, 75, 80, 90, 100, 150, 200, 250, 300, 450, 600, 650, 700, 750, or 800 liters may be used. In some embodiments of the method of the invention comprising the aforementioned steps a) through f), at least one iteration of step b) is carried out in a batch reactor. In some embodiments of the method of the invention comprising the aforementioned steps a) through f), at least step b) is carried out in a batch reactor. In some embodiments of the method of the invention comprising the aforementioned steps a) through f), each iteration of step b) is carried out in a batch reactor.

In a preferred embodiment, the contact time between the composition C (usable as composition for cleaving the protecting group comprising an optionally substituted triarylmethyl residue, preferably the DMT protecting group) and the oligonucleotide bound to a supporting moiety (e.g. a solid phase in a reactor) is at least 5 min, at least 6 min, at least 7 min, at least 8 min, at least 9 min, at least 10 min, at least 15 min, at least 20 min, at least 30 min, or even more than 1 hour.

Such comparably long time intervals allow the use of standard pumps having moderate pumping rates for comparably large-size reactors (e.g., column or batch reactors). Such long contacting times would result in undesired cleavage of nucleobases, in particular depurination, when used for cleavage solutions commonly used in the art such as, e.g., containing larger contents of acids such as dichloroacetic acid (DCA) without a base and an alcohol as defined in the present invention.

As known to those skilled in the art, oligonucleotide synthesis (including cleavage from the support, if a support has been used), is typically followed by a step of isolating the oligonucleotide.

Thus, in some embodiments, the method of the invention comprising the aforementioned steps a) through f) further comprises the following step h): h) isolating the support-cleaved oligonucleotide.

The means of isolating oligonucleotides upon chemical oligonucleotide synthesis form part of the common knowledge of those skilled in the art. Typically, such a step of isolating an oligonucleotide comprises one or more purification steps and one or more steps aiming at obtaining the oligonucleotide in solid form. For oligonucleotide purification, chromatographic methods may typically be used, in particular ion exchange (especially anion exchange) chromatography and reversed phase (RP) HPLC, e.g. in form of hydrophobic interaction HPLC. These techniques are known to those skilled in the art. Additionally, a step of isolating an oligonucleotide may comprise ultrafiltration and/or desalting steps. For example, a solution obtained after cleaving oligonucleotides from the support in support-assisted oligonucleotide synthesis may be submitted to ultrafiltration and/or desalting, ion exchange chromatography, and another round of ultrafiltration and/or desalting. Alternatively, the support-cleaved oligonucleotides may be submitted to reversed phase (RP) HPLC, e.g. in form of hydrophobic interaction HPLC. The latter method may preferably be performed, if the oligonucleotides still carry the 5'-terminal hydroxyl protecting group, e.g. the DMT group. Said 5'-protecting group may even be removed on the RP-HPLC column by passing an acidic solution through the column. If the 5'-terminal protecting group has been removed prior to purification, e.g. prior to cleaving the oligonucleotide from the support, ion exchange chromatography may be preferred. Purification is typically followed by one or more steps aiming at obtaining the oligonucleotide in solid form. Lyophilization and spray drying may for example be used. In some cases, it may be desirable to obtain the oligonucleotide in form of a salt with certain counter ions. In such cases salt exchange may be performed, typically prior to lyophilization or spray drying.

Preferably, the salt comprised in the liquid composition C is not very hygroscopic and is soluble in the mixture of organic solvents present in the cleavage composition C (i.e. the liquid composition C). Such salts may be obtained from the reaction of a weak base with a strong acid. The skilled person is aware of how acids and bases may form salts and that salt formation may typically occur in a certain stoichiometry depending on the charge of the comprised ions. An acid having a single acidic functional group and thus donating a single proton may be referred to as a monobasic acid, whose conjugate base (i.e. deprotonated form) has a single negative charge. If a molecule of such a monobasic acid, e.g. trifluoroacetic acid (TFA), hydrochloric acid (HCl) or methanesulfonic acid (MSA), donates a proton to a base having a single basic functional group (resulting in a single positive charge upon protonation) such as e.g. a pyridine derivative, salt formation may typically occur in a 1:1 stoichiometry of said acid and said base. It is understood that said salt may be solvated in the liquid composition C of the invention as laid out above.

The base comprised in the liquid composition C may serve to deprotonate the strong acid, resulting in a protonated form of the base (i.e. the salt's cation) being less acidic than the strong acid (vide infra). It is preferred that the total molar amount of the base comprised in the liquid composition C is equal to or larger than the total molar amount of the strong acid.

In some preferred embodiments, in the liquid composition C, the total molar amount of said base is equal to or larger than the total molar amount of said strong acid. In some embodiments, in the liquid composition C, the total molar amount of said base is in the range of 1.0-3.0, 1.0-2.0, 1.00-1.50, 1.00-1.40, 1.00-1.30, 1.00-1.20, 1.00-1.10, or even 1.000-1.050 equivalents relative to the total molar amount of said strong acid. It will be understood that the total molar amount of the base, refers to the total molar amount of the base added into said liquid composition C and does not differentiate between protonated and non-protonated molecules of the base. Likewise, it will be understood that the molar amount of the strong acid, refers to the total molar amount of the strong acid added into said liquid composition C and does not differentiate between protonated and non-protonated molecules of the strong acid. The term "total molar amount" of a certain species such as the base, the strong acid or the at least one alcohol of the composition C may refer to the sum of molar amounts of more than one base or acid or alcohol. For example, if the base contained in the composition C is a mixture of 4-cyanopyridine and 3-cyanopyridine, the total molar amount of said base is the sum of the molar amounts of 4-cyanopyridine and 3-cyanopyridine. The same rational applies e.g. to said strong acid and to said alcohol.

In the context of the present application, a weak base is a base, whose conjugated acid has a pKa of between 1 and 4. Likewise, in the context of the present application, a strong acid is an acid with a pKa of less than 1. As used herein, the pKa is such determinable in water (i.e. aqueous solution) at 25 °C, unless indicated differently. The means of determining the pKa value of an acid or a base are known to those skilled in the art, and for most common acids and bases, pKa values are readily available from the literature. For example, the pKa values of the base, in particular aromatic cyclic amine bases (e.g. pyridine derivatives), of the liquid composition C referred to herein may be taken from or determined according to the procedure disclosed in: A. Fischer, W.J. Galloway, J. Vaughan, Journal of the Chemical Society 1964, 3591-3596.

Preferred acids comprise trifluoroacetic acid, hydrochloric acid, and sulfonic acids. In some embodiments of the liquid composition C, the use thereof, and the method of the invention, the strong acid contained in the composition C is selected from the group consisting of a carboxylic acid, a mineral acid, sulfonic acids, and a mixture thereof. In some embodiments of the liquid composition C, the use thereof, and the method of the invention, the strong acid contained in the composition C is selected from the group consisting of trifluoroacetic acid, hydrochloric acid, sulfonic acids, and a mixture thereof, preferably, wherein the strong acid is selected from the group consisting of trifluoroacetic acid, hydrochloric acid, methanesulfonic acid, and a mixture thereof. In some embodiments of the liquid composition C, the use thereof, and the method of the invention, the strong acid contained in the composition C is selected from the group consisting of trifluoroacetic acid, hydrochloric acid, and a mixture thereof. In some embodiments of the liquid composition C, the use thereof, and the method of the invention, the strong acid contained in the composition C is trifluoroacetic acid.

Preferred bases comprise cyclic amines and heterocyclic amines. The base may be selected from the group consisting of: a pyrimidine, a pyridine, a pyrazine, a thiazole, a pyridazine, a pyrazole or a triazole, which may optionally be substituted with electron donating or electron withdrawing groups. For example, the base may be a pyrimidine, a pyridine, a thiazole, a pyridazine, a pyrazole, and a 1,2,4-triazole, substituted with one or more electron withdrawing substituent(s). In some embodiments of the liquid composition C, the use thereof, and the method of the invention, the base contained in the composition C is a cyclic amine, preferably wherein the cyclic amine is selected from the group consisting of a pyridine, a pyrimidine, a pyrazine, a thiazole, a pyridazine, a pyrazole, and a triazole. In some embodiments of the liquid composition C, the use thereof, and the method of the invention, the base contained in the composition C is a cyclic amine, preferably wherein the cyclic amine is selected from the group consisting of pyridine, pyrimidine, pyrazine, thiazole, pyridazine, pyrazole, and triazole, which may optionally be substituted with electron donating or electron withdrawing substituents. Herein, the terms "group(s)", "residue(s)", "moiety(or -ies)", and "substituent(s)" may be used interchangeably, so that e.g. the terms "electron withdrawing group(s)" and "electron withdrawing substituent(s)" may be used interchangeably. The electron withdrawing substituent(s) may be selected from the group consisting of a halogen atom such as a chlorine, fluorine, or bromine atom, a cyano group, an aldehyde group, a keto group, a carboxyester group, or a carboxamide group. As another example, the base may be a pyrimidine or a pyrazine substitued with one or more electron donating substituent(s). The electon donating substituent(s) may be a methoxy group. Herein, residues (i.e. substituents or groups) may be denoted interchangeably by their full name, e.g. chlorine atome or cyano group or in the form of e.g. Cl (instead of chlorine) or -CN (instead of cyano group). In such cases, especially if more than one atom is comprised in the residue, a hyphen may be used to indicate the atom with which said residue binds to the core structure, e.g. the carbon atom of the cyano group.

In some embodiments of the liquid composition C, the use thereof, and the method of the invention, the base contained in the composition C is a pyridine, which is substituted with one or more electron withdrawing substituents selected from the group consisting of an halogen atom, a cyano group, an aldehyde group, a keto group, a carboxyester group, and a carboxamide group.

In some embodiments of the liquid composition C, the use thereof, and the method of the invention, the base contained in the composition C is a pyridine, in which exactly one hydrogen residue is substituted by an electron-withdrawing substituent selected from the group consisting of a cyano group and a halogen atom.

In some embodiments of the liquid composition C, the use thereof, and the method of the invention, the base contained in the composition C is selected from the group consisting of 4-cyanopyridine, 3-cyanopyridine, 4-chloropyridine, 3-chloropyridine, and a mixture thereof. In some embodiments of the liquid composition C, the use thereof, and the method of the invention, the base contained in the composition C is selected from the group consisting of 4-cyanopyridine, 3-cyanopyridine, 4-chloropyridine, and 3-chloropyridine.

In some embodiments of the liquid composition C, the use thereof, and the method of the invention, the base contained in the composition C is a pyridine, in which exactly one hydrogen residue is substituted by an electron-withdrawing substituent selected from the group consisting of a -CN, Cl, Br, F, and I. In some embodiments of the liquid composition C, the use thereof, and the method of the invention, the base contained in the composition C is a pyridine, in which exactly one hydrogen residue is substituted by an electron-withdrawing substituent selected from the group consisting of a -CN, Cl, Br, and F. In some embodiments of the liquid composition C, the use thereof, and the method of the invention, the base contained in the composition C is a pyridine, in which exactly one hydrogen residue is substituted by an electron-withdrawing substituent selected from the group consisting of a -CN, Cl, and Br. In some embodiments of the liquid composition C, the use thereof, and the method of the invention, the base contained in the composition C is a pyridine, in which exactly one hydrogen residue is substituted by an electron-withdrawing substituent selected from the group consisting of a -CN and Cl. In some embodiments of the liquid composition C, the use thereof, and the method of the invention, the base contained in the composition C is a pyridine, in which exactly one hydrogen residue is substituted by -CN.

In some embodiments of the liquid composition C, the use thereof, and the method of the invention, the base contained in the composition C is 4-cyanopyridine. In some embodiments of the liquid composition C, the use thereof, and the method of the invention, the base contained in the composition C is 3-cyanopyridine. In some embodiments of the liquid composition C, the use thereof, and the method of the invention, the base contained in the composition C is 4-chloropyridine. In some embodiments of the liquid composition C, the use thereof, and the method of the invention, the base contained in the composition C is 3-chloropyridine. In some embodiments of the liquid composition C, the use thereof, and the method of the invention, the base contained in the composition C is 4-bromopyridine. In some embodiments of the liquid composition C, the use thereof, and the method of the invention, the base contained in the composition C is 3-bromopyridine.

In some embodiments of the liquid composition C, the use thereof, and the method of the invention, the base contained in the composition C is a cyclic amine of formula S-1: 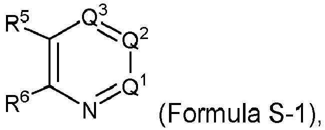 wherein in formula S-1:
Q¹ is N or C-R⁷;
Q² is N or C-R⁸;
Q³ is N or C-R⁹;
R⁵, R⁶, R⁷, R⁸, and R⁹ are independently of each other selected from the group consisting of H, a C₁-C₅-alkyl group, and an electron withdrawing substituent;
with the proviso that if none of Q¹, Q², and Q³ is N, at least one substituent selected from the group consisting of R⁵, R⁶, R⁷, R⁸, and R⁹ is an electron withdrawing substituent; and with the further proviso that not more than one of Q¹, Q², and Q³ is N.

In some embodiments of the liquid composition C, the use thereof, and the method of the invention, the base contained in the composition C is a cyclic amine of formula S-1, wherein:
Q¹ is N or C-R⁷;
Q² is N or C-R⁸;
Q³ is N or C-R⁹;
R⁵, R⁶, R⁷, R⁸, and R⁹ are independently of each other selected from the group consisting of H, a C₁-C₅-alkyl group, and an electron withdrawing substituent selected from the group consisting of a -CN, Cl, Br, and F;
with the proviso that if none of Q¹, Q², and Q³ is N, at least one substituent selected from the group consisting of R⁵, R⁶, R⁷, R⁸, and R⁹ is an electron withdrawing substituent; and with the further proviso that not more than one of Q¹, Q², and Q³ is N.

In some embodiments of the liquid composition C, the use thereof, and the method of the invention, the base contained in the composition C is a cyclic amine of formula S-1, wherein:
Q¹ is C-R⁷;
Q² is C-R⁸;
Q³ is C-R⁹;
R⁵, R⁶, R⁷, R⁸, and R⁹ are independently of each other selected from the group consisting of H and an electron withdrawing group selected from the group consisting of -CN, Cl, Br, and F;
with the proviso that at least one, preferably exactly one, substituent selected from the group consisting of R⁵, R⁶, R⁷, R⁸, and R⁹ is an electron withdrawing group.

In some embodiments of the liquid composition C, the use thereof, and the method of the invention, the base contained in the composition C is a cyclic amine of formula S-1, wherein:
Q¹ is C-R⁷;
Q² is C-R⁸;
Q³ is C-R⁹;
R⁵, R⁶, R⁷, R⁸, and R⁹ are independently of each other selected from the group consisting of H and an electron withdrawing group selected from the group consisting of -CN, Cl, and Br;
with the proviso that at least one, preferably exactly one, substituent selected from the group consisting of R⁵, R⁶, R⁷, R⁸, and R⁹ is an electron withdrawing group.

In some embodiments of the liquid composition C, the use thereof, and the method of the invention, the base contained in the composition C is a cyclic amine of formula S-1, wherein:
Q¹ is C-R⁷;
Q² is C-R⁸;
Q³ is C-R⁹;
R⁵, R⁶, R⁷, R⁸, and R⁹ are independently of each other selected from the group consisting of H and an electron withdrawing group selected from the group consisting of -CN, and Cl;
with the proviso that at least one, preferably exactly one, substituent selected from the group consisting of R⁵, R⁶, R⁷, R⁸, and R⁹ is an electron withdrawing group.

One aspect of the invention relates to the use of a liquid composition C comprising an aprotic solvent, at least one alcohol selected from the group consisting of trifluoroethanol, hexafluoroisopropanol, pentafluoropropanol, 1,1,1,3,3,3-hexafluoro-2-methyl-2-propanol, and nonafluoro tertiary butyl alcohol, and a salt of a base with a strong acid for suppressing nucleobase cleavage while effecting cleavage of a di(p-methoxyphenyl)phenylmethyl protecting group from a hydroxyl group during the chemical synthesis of an oligonucleotide, wherein:
- the base is a pyridine, which is substituted with one or more electron withdrawing substituents selected from the group consisting of a halogen atom, a cyano group, an aldehyde group, a keto group, a carboxyester group, and a carboxamide group; and
- the strong acid contained in the composition C is selected from the group consisting of trifluoroacetic acid, hydrochloric acid, sulfonic acids, and a mixture thereof.

One aspect of the invention relates to the use of a liquid composition C comprising an aprotic solvent, at least one alcohol selected from the group consisting of trifluoroethanol, hexafluoroisopropanol, pentafluoropropanol, 1,1,1,3,3,3-hexafluoro-2-methyl-2-propanol, and nonafluoro tertiary butyl alcohol, and a salt of a base with a strong acid for suppressing nucleobase cleavage while effecting cleavage of a di(p-methoxyphenyl)phenylmethyl protecting group from a hydroxyl group during the chemical synthesis of an oligonucleotide, wherein:
- the base is a pyridine, in which exactly one hydrogen residue is substituted by an electron-withdrawing substituent selected from the group consisting of a cyano group and a halogen atom, more preferably, wherein the base is selected from the group consisting of 4-cyanopyridine, 3-cyanopyridine, 4-chloropyridine, 3-chloropyridine, and a mixture thereof; and
- the strong acid contained in the composition C is selected from the group consisting of trifluoroacetic acid, hydrochloric acid, methanesulfonic acid, and a mixture thereof.

A further aspect of the invention relates to a liquid composition C for use in the cleavage of a di(p-methoxyphenyl)phenylmethyl protecting group from a hydroxyl group, the composition comprising an aprotic solvent, at least one alcohol selected from the group consisting of trifluoroethanol, hexafluoroisopropanol, pentafluoro-propanol, 1,1,1,3,3,3-hexafluoro-2-methyl-2-propanol, and nonafluoro tertiary butyl alcohol, and a salt of a base with a strong acid, wherein:
- the aprotic solvent is non-halogenated and selected from the group consisting of a (hetero)aromatic solvent, an alkyl (hetero)aromatic solvent, a (hetero)aromatic ether, or an alkyl (hetero)aryl ether;
- the base is a pyridine, which is substituted with one or more electron withdrawing substituents selected from the group consisting of a halogen atom, a cyano group, an aldehyde group, a keto group, a carboxyester group, and a carboxamide group; and
- the strong acid contained in the composition C is selected from the group consisting of trifluoroacetic acid, hydrochloric acid, and sulfonic acids.

A further aspect of the invention relates to a liquid composition C for use in the cleavage of a di(p-methoxyphenyl)phenylmethyl protecting group from a hydroxyl group, the composition comprising a non-halogenated and/or (hetero)aromatic, aprotic solvent, at least one alcohol selected from the group consisting of trifluoroethanol, hexafluoroisopropanol, pentafluoro-propanol, 1,1,1,3,3,3-hexafluoro-2-methyl-2-propanol, and nonafluoro tertiary butyl alcohol, and a salt of a base with a strong acid, wherein:
- the aprotic solvent is non-halogenated and selected from the group consisting of a (hetero)aromatic solvent, an alkyl (hetero)aromatic solvent, a (hetero)aromatic ether, and an alkyl (hetero)aryl ether;
- the base is a pyridine, in which exactly one hydrogen residue is substituted by an electron-withdrawing substituent selected from the group consisting of a cyano group and a halogen atom, more preferably, wherein the base is selected from the group consisting of 4-cyanopyridine, 3-cyanopyridine, 4-chloropyridine, 3-chloropyridine, and a mixture thereof; and
- the strong acid contained in the composition C is selected from the group consisting of trifluoroacetic acid, hydrochloric acid, methanesulfonic acid, and a mixture thereof.

A further aspect of the invention relates to a method for the synthesis of oligonucleotides, comprising the following steps a) through f):
a) providing a nucleoside or oligonucleotide bound to a supporting moiety, wherein the nucleoside or oligonucleotide comprises a backbone hydroxyl moiety, which is protected by a di(p-methoxyphenyl)phenylmethyl protecting group;
b) cleaving the di(p-methoxyphenyl)phenylmethyl protecting group from the nucleoside or oligonucleotide by incubating said compound with a liquid composition C, thereby generating a free backbone hydroxyl moiety;
c) reacting the free backbone hydroxyl group resulting from step b) with a phosphorus moiety of a nucleoside or oligonucleotide building block, which building block further comprises a hydroxyl group protected by a di(p-methoxyphenyl)phenylmethyl protecting group, thereby producing a covalent linkage between the oxygen atom of said free hydroxyl group and the phosphorus atom of said building block;
d) optionally modifying the phosphorus moiety;
e) optionally reiterating the sequence of steps b) to c) or b) to d); and
f) cleaving the oligonucleotide from the supporting moiety;
wherein the liquid composition C comprises an aprotic solvent, at least one alcohol selected from the group consisting of trifluoroethanol, hexafluoroisopropanol, pentafluoropropanol, 1,1,1,3,3,3-hexafluoro-2-methyl-2-propanol, and nonafluoro tertiary butyl alcohol, and a salt of a base with a strong acid, wherein:
- the base is a pyridine, which is substituted with one or more electron withdrawing substituents selected from the group consisting of a halogen atom, a cyano group, an aldehyde group, a keto group, a carboxyester group, and a carboxamide group; and
- the strong acid contained in the composition C is selected from the group consisting of trifluoroacetic acid, hydrochloric acid, sulfonic acids, and a mixture thereof.

A further aspect of the invention relates to a method for the synthesis of oligonucleotides, comprising the following steps a) through f):
a) providing a nucleoside or oligonucleotide bound to a supporting moiety, wherein the nucleoside or oligonucleotide comprises a backbone hydroxyl moiety, which is protected by a di(p-methoxyphenyl)phenylmethyl protecting group;
b) cleaving the di(p-methoxyphenyl)phenylmethyl protecting group from the nucleoside or oligonucleotide by incubating said compound with a liquid composition C, thereby generating a free backbone hydroxyl moiety;
c) reacting the free backbone hydroxyl group resulting from step b) with a phosphorus moiety of a nucleoside or oligonucleotide building block, which building block further comprises a hydroxyl group protected by a di(p-methoxyphenyl)phenylmethyl protecting group, thereby producing a covalent linkage between the oxygen atom of said free hydroxyl group and the phosphorus atom of said building block;
d) optionally modifying the phosphorus moiety;
e) optionally reiterating the sequence of steps b) to c) or b) to d); and
f) cleaving the oligonucleotide from the supporting moiety;
wherein the liquid composition C comprises an aprotic solvent, at least one alcohol selected from the group consisting of trifluoroethanol, hexafluoroisopropanol, pentafluoropropanol, 1,1,1,3,3,3-hexafluoro-2-methyl-2-propanol, and nonafluoro tertiary butyl alcohol, and a salt of a base with a strong acid, wherein:
- the base is a pyridine, in which exactly one hydrogen residue is substituted by an electron-withdrawing substituent selected from the group consisting of a cyano group and a halogen atom, more preferably, wherein the base is selected from the group consisting of 4-cyanopyridine, 3-cyanopyridine, 4-chloropyridine, 3-chloropyridine, and a mixture thereof; and
- the strong acid contained in the composition C is selected from the group consisting of trifluoroacetic acid, hydrochloric acid, methanesulfonic acid, and a mixture thereof.

The salt comprised in the cleavage composition may act as a proton donor in the deprotection reaction. The skilled person will therefore select the salt such that the pKa of its protonated cation corresponds to the nature of the triarylmethyl group to be cleaved. For example, the salt used for the cleavage of a protecting group comprising an optionally substituted triarylmethyl residue with two methoxy substituents, e.g. for the cleavage of the dimethoxytrityl (DMT, preferably di(p-methoxyphenyl)phenylmethyl) group, may preferably comprise a protonated cation having a pKa of between 1 and 2.5, preferably a pKa of between 1 and 2. In some embodiments, the protonated form of the base (i.e. the salt's cation) contained in the liquid composition C has a pKa in the range of 1-4, 1-3.5, 1-3, 1-2.5, or 1-2. Examples are the conjugated acids of 4-cyanopyridine, 3-cyanopyridine, pyrimidine, 2-methylpyrazine, 2,5-dimethylpyrazine, and 3-bromo-pyridine. Preferably, the cleavage composition of the present invention comprises a salt selected from the group consisting of 4-cyanopyridinium trifluoroacetate, 4-cyanopyridinium chloride, a 4-cyanopyridinium sulfonate such as an 4-cyanopyridinium alkylsulfonate or 4-cyanopyridinium p-toluenesulfonate, 3-cyanopyridinium trifluoroacetate, 3-cyanopyridinium chloride, a 3-cyanopyridinium sulfonate such as an 3-cyanopyridinium alkylsulfonate or 3-cyanopyridinium p-toluenesulfonate, pyrimidinium trifluoroacetate, pyrimidinium chloride, a pyrimidinium sulfonate such as an pyrimidinium alkylsulfonate or pyrimidinium p-toluenesulfonate, 2-methylpyrazinium trifluoroacetate, 2-methylpyrazinium chloride, a 2-methylpyrazinium sulfonate such as an 2-methylpyrazinium alkylsulfonate or 2-methylpyrazinium p-toluenesulfonate, 2,5-dimethylpyrazinium trifluoroacetate, 2,5-dimethylpyrazinium chloride, and a 2,5-dimethylpyrazinium sulfonate such as an 2,5-dimethylpyrazinium alkylsulfonate or 2,5-dimethylpyrazinium p-toluenesulfonate. Particularly preferred is 4-cyanopyridinium trifluoroacetate.

In some embodiments of the liquid composition C, the use thereof, and the method of the invention, the salt of a base with a strong acid is selected from the group consisting of 4-cyanopyridinium trifluoroacetate, 3-cyanopyridinium trifluoroacetate, 3-chloropyridiniumtrifluoroacetate, and 4-chloropyridinium hydrochloride. In some embodiments of the liquid composition C, the use thereof, and the method of the invention, the salt of a base with a strong acid is selected from the group consisting of 4-cyanopyridinium trifluoroacetate, 3-cyanopyridinium trifluoroacetate, and 3-chloropyridiniumtrifluoroacetate. In some embodiments of the liquid composition C, the use thereof, and the method of the invention, the salt of a base with a strong acid is 4-cyanopyridinium trifluoroacetate.

The composition C comprises at least one alcohol selected from the group consisting of trifluoroethanol, hexafluoroisopropanol, pentafluoropropanol, 1,1,1,3,3,3-hexafluoro-2-methyl-2-propanol, and nonafluoro tertiary butyl alcohol, or mixtures thereof. The use of trifluoroethanol, hexafluoroisopropanol, or mixtures thereof is particularly preferred.

In some embodiments of the liquid composition C, the use thereof, and the method of the invention, the at least one alcohol contained in the liquid composition C is selected from the group consisting of trifluoroethanol, hexafluoroisopropanol, and a mixture thereof. In some embodiments of the liquid composition C, the use thereof, and the method of the invention, the at least one alcohol contained in the liquid composition C is trifluoroethanol. In some embodiments of the liquid composition C, the use thereof, and the method of the invention, the at least one alcohol contained in the liquid composition C is hexafluoroisopropanol.

The total molar amount of said alcohol in the cleavage composition may be at least 2, 5, 10, 20, 40, 60, 70, 80, or 100 fold higher than the total amount of nucleobases. In some embodiments, the molar concentration of said alcohol in the composition C is at least 2 times more than the molar concentration of said salt's cation contained in the composition C. It is to be understood that in embodiments, where the composition C comprises more than one of of trifluoroethanol, hexafluoroisopropanol, pentafluoropropanol, 1,1,1,3,3,3-hexafluoro-2-methyl-2-propanol, and nonafluoro tertiary butyl alcohol, the molar concentration of said alcohol in the composition C is calculated as the sum of the molar amounts of each of the alcohols divided by the liquid volume of the composition C. In some embodiments, the molar concentration of said alcohol in the composition C is at least 4, 6, 8, 10, 12, 14, 16, 18, 20, 30, 40, or 50 times more than the molar concentration of said salt's cation contained in the composition C.

In some embodiments of the liquid composition C, the use thereof, and the method of the invention, the molar concentration of said alcohol in the composition C is at least 2 times more than the molar concentration of said base contained in the composition C. In some embodiments of the liquid composition C, the use thereof, and the method of the invention, the molar concentration of said alcohol in the liquid composition C is at least 4, 6, 8, 10, 12, 14, 16, 18, 20, 30, 40, or 50 times more than the molar concentration of said base contained in the composition C. The term "said alcohol" embraces a mixture of alcohols, for example a mixture of TFE and HFIP, if such a mixture is present in the liquid composition C. Likewise, the term "said base" embraces a mixture of bases, for example 4-cyanopyridine and 3-cyaonopyridine, if such a mixture is present in the liquid composition C.

In some embodiments, the composition C further comprises a carbocation scavenger. As used herein, the expression "carbocation scavenger" relates to a nucleophilic compound, which may be used to bind a carbocation formed, thereby preventing unwanted side reactions of the carbocation, or to a compound which may consume carbocations by formal donation of a hydride anion. Examples of carbocation scavengers are alcohols and phenols [e.g. methanol, ethanol, phenol, cresol, water], phenol ethers [e.g. anisole, 1,3-dimethoxybenzene (dimethylanisole), 1,3,5-trimethoxybenzene]; thioethers [e.g. dimethylsulfide, methyl ethyl sulfide, thioanisole-, silanes -e.g. triisopropylsilane (TIS), triethylsilane (TES)], N-heterocycles [e.g. pyrrole, 3-methylpyrrole, 2,4-dimethylpyrrole indole, 2-methylindole, succinimide, phthalimide], thiols and thiophenols [e.g. 1,2-ethanedithiol (EDT), 1,4-dithioerythrol (DTE), 1,4-dithiothreitol (DTT), 3,6-dioxa-1,8-octanedithiol (DODT), 1,4-benzenedimethanthiol (BDMT), 1,4-butanedithiol, 2-mercaptoethanol, cysteine, thiophenol, p-thiocresol], and polyalkylbenzenes [e.g. 1,3,5-trimethylbenzene, pentamethylbenzene].

The aprotic solvent comprised in the composition C is preferably non-halogenated Suitable solvents may be chosen from the group consisting of (hetero)aromatic solvents, alkyl (hetero)aromatic solvents, or (hetero)aromatic ethers, preferably from the group consisting of toluene, o-xylene, m-xylene, p-xylene, mesitylene, and diphenyl ether. The composition C may comprise an aprotic, nonhalogenated and/or (hetero)aromatic solvent and further halogenated solvents in addition to the alcohol selected from the group trifluoroethanol, hexafluoroisopropanol, pentafluoropropanol, 1,1,1,3,3,3-hexafluoro-2-methyl-2-propanol, and nonafluoro tertiary butyl alcohol.

In some embodiments of the use and the method of the invention, the aprotic solvent contained in the composition C is selected from the group consisting of a halogenated hydrocarbon solvent, a (hetero)aromatic solvent, an alkyl (hetero)aromatic solvent, a (hetero)aromatic ether, and an alkyl (hetero)aryl ether.

Non-limiting examples of a halogenated hydrocarbon solvent comprise dichloromethane (DCM), dichloroethane, and chloroform. A non-limiting example of a (hetero)aromatic solvent is benzene. Non-limiting examples of an alkyl (hetero)aromatic solvent are toluene, o-xylene, m-xylene, p-xylene, and mesitylene. A non-limiting example of a (hetero)aromatic ether is diphenyl ether. A non-limiting example of an alkyl (hetero)aryl ether is anisole.

In some embodiments of the use and the method of the invention, the aprotic solvent contained in the composition C is selected from the group consisting of toluene, o-xylene, m-xylene, p-xylene, mesitylene, anisole, dichloromethane, and a mixture thereof. In some embodiments of the use and the method of the invention, the aprotic solvent contained in the composition C is selected from the group consisting of toluene, o-xylene, m-xylene, p-xylene, mesitylene, dichloromethane, and a mixture thereof. In some embodiments of the use and the method of the invention, the aprotic solvent contained in the composition C is selected from the group consisting of toluene, anisole, dichloromethane, and a mixture thereof.

In some embodiments of the use and the method of the invention, the aprotic solvent contained in the composition C is non-halogenated (i.e. does not comprise any halogen atoms in its chemical structure). In some embodiments of the use and the method of the invention, the aprotic solvent contained in the composition C is selected from the group consisting of toluene, o-xylene, m-xylene, p-xylene, mesitylene, anisole, and a mixture thereof.

In some embodiments of the liquid composition C, the use thereof, and the method of the invention, the aprotic solvent contained in the composition C is selected from the group consisting of toluene, o-xylene, m-xylene, p-xylene, mesitylene, anisole, and a mixture thereof. In some embodiments of the liquid composition C, the use thereof, and the method of the invention, the aprotic solvent contained in the composition C is selected from the group consisting of toluene, anisole, and a mixture thereof.

In some embodiments of the liquid composition C of the invention, the aprotic solvent is selected from the group consisting of toluene, anisole, and a mixture thereof. In some embodiments of the liquid composition C of the invention, the aprotic solvent is toluene. In some embodiments of the liquid composition C of the invention, the aprotic solvent is anisole.

In some embodiments of the liquid composition C, the use thereof, and the method of the invention, the total volume of the aprotic solvent contained in the liquid composition C accounts for 60-99%, 70-98%, 80-98%, 81-98%, or 81-95% of the overall volume of the liquid composition C. In some embodiments of the liquid composition C, the use thereof, and the method of the invention, the total volume of the alcohol contained in the liquid composition C accounts for 1-40%, 2-30%, 2-20%, 2-19%, or 5-19% of the overall volume of the liquid composition C.

The term "overall volume of the liquid composition C" refers to the combined volumes of the alcohol, the aprotic solvent, and any other liquid components combined to form the liquid composition C. The change of the overall volume by dissolving solid components such the base, if it should be solid at room temperature, are not considered for said "overall volume of the liquid composition C". Likewise, the volume of the oligonucleotides and the support is not considered part of said "overall volume of the liquid composition C". For example, if the liquid composition C consists of TFE (as the alcohol), toluene (as the aprotic solvent), TFA (as the strong acid), and 4-cyanopyridine (as the base), the "overall volume of the liquid composition C" would be the combined volumes of TFE, toluene, and TFA. The term "total volume" is used to denote that if, for example, the alcohol is indeed a mixture of alcohols, for example a mixture of TFE and HFIP, said "total volume" would refer to the sum of the volumes of the alcohols, e.g. of TFE and HFIP. The same logic applies to the "total volume" of the aprotic solvent.

In some embodiments of the liquid composition C, the use thereof, and the method of the invention:
- the total volume of the aprotic solvent contained in the liquid composition C accounts for 60-99% of the overall volume of the liquid composition C; and
- the total volume of the alcohol contained in the liquid composition C accounts for 1-40% of the overall volume of the liquid composition C.

In some embodiments of the liquid composition C, the use thereof, and the method of the invention:
- the total volume of the aprotic solvent contained in the liquid composition C accounts for 70-98%, of the overall volume of the liquid composition C; and
- the total volume of the alcohol contained in the liquid composition C accounts for 2-30% of the overall volume of the liquid composition C.

In some embodiments of the liquid composition C, the use thereof, and the method of the invention:
- the total volume of the aprotic solvent contained in the liquid composition C accounts for 81-98%, of the overall volume of the liquid composition C; and
- the total volume of the alcohol contained in the liquid composition C accounts for 2-19% of the overall volume of the liquid composition C.

In some embodiments of the liquid composition C, the use thereof, and the method of the invention:
- the total volume of the aprotic solvent contained in the liquid composition C accounts for 81-95%, of the overall volume of the liquid composition C; and
- the total volume of the alcohol contained in the liquid composition C accounts for 5-19% of the overall volume of the liquid composition C.

In some embodiments of the liquid composition C, the use thereof, and the method of the invention, the overall volume of the liquid composition C is in the range of 5-100 mL, 10-90 mL, 10-80 mL, 20-75 mL, 10-70 mL, 10-60 mL, 20-50 mL, 20-40 mL, or 25-37.5 mL per 1 millimole (mmol) of the hydroxyl protecting group comprising an optionally substituted triarylmethyl residue, preferably the DMT group, to be cleaved (i.e. removed).

In some embodiments of the liquid composition C, the use thereof, and the method of the invention, in the liquid composition C, the total molar amount of said strong acid is in the range of 0.80-15.0, 1.0-13.0, 2.0-12.50, or 2.30-12.50 equivalents relative to the total molar amount of protecting groups comprising an optionally substituted triarylmethyl residue, preferably the di(p-methoxyphenyl)phenylmethyl (DMT) groups. The term "total molar amount" when referring to a protecting group such as the DMT group refers to the total molar amount of the compound(s) carrying the respective protecting group multiplied with the amount of said protecting group per molecule of these compound(s). For example, if the composition C comprises 1 mole of a compound having exactly one DMT group per molecule, the total molar amount of DMT groups in the composition C equals the total molar amount of said compound and would in this example also be 1 mole.

In some embodiments of the liquid composition C, the use thereof, and the method of the invention, in the liquid composition C, the total molar amount of said alcohol is in the range of 2.0-150.0, 2.0-120.0, 2.0-100.0, 2.0-95.0, 2.5-95.0, 3.0-95.0, 3.0-90.0, 3.0-85.0, 3.0-80.0, 3.0-75.0, 3.0-70.0, or 3.0-65.0 equivalents relative to the total molar amount of nucleobases. The term "total molar amount of nucleobases" may refer to the overall molar amount of all nucleobases comprised in the liquid composition C. It is understood that the nucleobases will essentially all, or at least most, be part of nucleoside units of the oligonucleotide (i.e. the oligonucleotide molecules), from which the protecting group comprising an optionally substituted triarylmethyl residue, preferably the DMT group, is to be cleaved. For example, if the composition C comprises 1 mole of a compound, whose molecules each comprise 5 nucleobases, the total molar amount of nucleobases in said composition C is 5 mole.

In some embodiments of the liquid composition C, the use thereof, and the method of the invention, in the liquid composition C:
- the total molar amount of said strong acid is in the range of 0.80-15.0 equivalents relative to the total molar amount of di(p-methoxyphenyl)phenylmethyl (DMT) groups; and
- the total molar amount of said alcohol is in the range of 2.0-150.0 equivalents relative to the total molar amount of nucleobases.

In some embodiments of the liquid composition C, the use thereof, and the method of the invention, in the liquid composition C:
- the total molar amount of said strong acid is in the range of 1.0-13.0 equivalents relative to the total molar amount of di(p-methoxyphenyl)phenylmethyl (DMT) groups; and
- the total molar amount of said alcohol is in the range of 2.0-100.0 equivalents relative to the total molar amount of nucleobases.

In some embodiments of the liquid composition C, the use thereof, and the method of the invention, in the liquid composition C:
- the total molar amount of said strong acid is in the range of 2.0-12.5 equivalents relative to the total molar amount of di(p-methoxyphenyl)phenylmethyl (DMT) groups; and
- the total molar amount of said alcohol is in the range of 3.0-95.0 equivalents relative to the total molar amount of nucleobases.

In some preferred embodiments, the present invention provides a method for the synthesis of oligonucleotides, comprising steps a) through f):
a) providing a nucleoside or oligonucleotide bound to a supporting moiety, wherein the nucleoside or oligonucleotide comprises a backbone hydroxyl moiety, which is protected by a di(p-methoxyphenyl)phenylmethyl (DMT) group;
b) cleaving the DMT group from the nucleoside or oligonucleotide by incubating said compound with a liquid composition C, thereby generating a free backbone hydroxyl moiety;
c) reacting the free backbone hydroxyl group resulting from step b) with a phosphorus moiety of a nucleoside or oligonucleotide building block, which building block further comprises a hydroxyl group protected by a DMT group, thereby producing a covalent linkage between the oxygen atom of said free hydroxyl group and the phosphorus atom of said building block;
d) optionally modifying the phosphorus moiety;
e) optionally reiterating the sequence of steps b) to c) or b) to d); and
f) cleaving the oligonucleotide from the supporting moiety;
wherein the liquid composition C comprises an aprotic solvent, at least one alcohol selected from the group consisting of trifluoroethanol, hexafluoroisopropanol, pentafluoropropanol, 1,1,1,3,3,3-hexafluoro-2-methyl-2-propanol, and nonafluoro tertiary butyl alcohol, and a salt of a base with a strong acid, wherein the salt's cation has a pKa in the range of 1 to 4.

In some preferred embodiments, the present invention provides a method for the synthesis of oligonucleotides, comprising steps a) through f):
a) providing a nucleoside or oligonucleotide bound to a supporting moiety, wherein the nucleoside or oligonucleotide comprises a backbone hydroxyl moiety, which is protected by a di(p-methoxyphenyl)phenylmethyl (DMT) group;
b) cleaving the DMT group from the nucleoside or oligonucleotide by incubating said compound with a liquid composition C, thereby generating a free backbone hydroxyl moiety;
c) reacting the free backbone hydroxyl group resulting from step b) with a phosphorus moiety of a nucleoside or oligonucleotide building block, which building block further comprises a hydroxyl group protected by a DMT group, thereby producing a covalent linkage between the oxygen atom of said free hydroxyl group and the phosphorus atom of said building block;
d) optionally modifying the phosphorus moiety
e) optionally reiterating the sequence of steps b) to c) or b) to d); and
f) cleaving the oligonucleotide from the supporting moiety;
wherein the liquid composition C comprises a non-halogenic and/or (hetero)aromatic aprotic solvent, at least one alcohol selected from the group consisting of trifluoroethanol, hexafluoroisopropanol, pentafluoropropanol, 1,1,1,3,3,3-hexafluoro-2-methyl-2-propanol, and nonafluoro tertiary butyl alcohol, and a salt of a base with a strong acid, wherein the salt's cation has a pKa in the range of 1 to 4.

In some preferred embodiments, the present invention provides a method for the synthesis of oligonucleotides, comprising steps a) through f):
a) providing a nucleoside or oligonucleotide bound to a supporting moiety, wherein the nucleoside or oligonucleotide comprises a backbone hydroxyl moiety, which is protected by a di(p-methoxyphenyl)phenylmethyl (DMT) group;
b) cleaving the DMT group from the nucleoside or oligonucleotide by incubating said compound with a liquid composition C, thereby generating a free backbone hydroxyl moiety;
c) reacting the free backbone hydroxyl group resulting from step b) with a phosphorus moiety of a nucleoside or oligonucleotide building block, which building block further comprises a hydroxyl group protected by a DMT group, thereby producing a covalent linkage between the oxygen atom of said free hydroxyl group and the phosphorus atom of said building block;
d) optionally modifying the phosphorus moiety
e) optionally reiterating the sequence of steps b) to c) or b) to d); and
f) cleaving the oligonucleotide from the supporting moiety;
wherein the liquid composition C comprises a non-halogenic and/or (hetero)aromatic aprotic solvent, trifluoroethanol, and a salt of a base with a strong acid, wherein the salt's cation has a pKa in the range of 1 to 4.

In some preferred embodiments, the present invention provides a method for the synthesis of oligonucleotides, comprising steps a) through f):
a) providing a nucleoside or oligonucleotide bound to a supporting moiety, wherein the nucleoside or oligonucleotide comprises a backbone hydroxyl moiety, which is protected by a di(p-methoxyphenyl)phenylmethyl (DMT) group;
b) cleaving the DMT group from the nucleoside or oligonucleotide by incubating said compound with a liquid composition C, thereby generating a free backbone hydroxyl moiety;
c) reacting the free backbone hydroxyl group resulting from step b) with a phosphorus moiety of a nucleoside or oligonucleotide building block, which building block further comprises a hydroxyl group protected by a DMT group, thereby producing a covalent linkage between the oxygen atom of said free hydroxyl group and the phosphorus atom of said building block;
d) optionally modifying the phosphorus moiety
e) optionally reiterating the sequence of steps b) to c) or b) to d); and
f) cleaving the oligonucleotide from the supporting moiety;
wherein the liquid composition C comprises a non-halogenic and/or (hetero)aromatic aprotic solvent, hexafluoroisopropanol, and a salt of a base with a strong acid, wherein the salt's cation has a pKa in the range of 1 to 4.

The salt in the above embodiments may for example be selected from the group consisting of 4-cyanopyridinium trifluoroacetate, 4-cyanopyridinium chloride, a 4-cyanopyridinium sulfonate such as an 4-cyanopyridinium alkylsulfonate or 4-cyanopyridinium p-toluenesulfonate, 3-cyanopyridinium trifluoroacetate, 3-cyanopyridinium chloride, a 3-cyanopyridinium sulfonate such as an 3-cyanopyridinium alkylsulfonate or 3-cyanopyridinium p-toluenesulfonate, pyrimidinium trifluoroacetate, pyrimidinium chloride, a pyrimidinium sulfonate such as an pyrimidinium alkylsulfonate or pyrimidinium p-toluenesulfonate, 2-methyl-pyrazinium trifluoroacetate, 2-methyl-pyrazinium chloride, a 2-methyl-pyrazinium sulfonate such as an 2-methyl-pyrazinium alkylsulfonate or 2-methyl-pyrazinium p-toluenesulfonate, 2,5-dimethyl-pyrazinium trifluoroacetate, 2,5-dimethyl-pyrazinium chloride, and a 2,5-dimethyl-pyrazinium sulfonate such as an 2,5-dimethyl-pyrazinium alkylsulfonate or 2,5-dimethyl-pyrazinium p-toluenesulfonate. Particularly preferred is 4-cyanopyridinium trifluoroacetate.. In some embodiments of the liquid composition C, the use thereof, and the method of the invention, the salt of a base with a strong acid is selected from the group consisting of 4-cyanopyridinium trifluoroacetate, 4-cyanopyridinium methanesulfonate, 4-cyanopyridinium hydrochloride, 3-cyanopyridinium trifluoroacetate, 3-cyanopyridinium methanesulfonate, 3-cyanopyridinium hydrochloride, 4-chloropyridinium trifluoroacetate, 4-chloropyridinium methanesulfonate, 4-chloropyridinium hydrochloride, 3-chloropyridinium trifluoroacetate, 3-chloropyridinium methanesulfonate, and 3-chloropyridinium hydrochloride. In some embodiments of the liquid composition C, the use thereof, and the method of the invention, the salt of a base with a strong acid is selected from the group consisting of 4-cyanopyridinium trifluoroacetate, 4-cyanopyridinium methanesulfonate, 3-cyanopyridinium trifluoroacetate, 4-chloropyridinium hydrochloride, and 3-chloropyridinium trifluoroacetate. In some embodiments of the liquid composition C, the use thereof, and the method of the invention, the salt of a base with a strong acid is 4-cyanopyridinium trifluoroacetate.

The explanations and details, which are explained herein with respect to the methods of the invention, are likewise applicable to the use of the liquid composition C according to the present invention and to the liquid composition itself, unless indicated differently.

In some embodiments of the use and/or the method of the invention:
- the aprotic solvent contained in the composition C is selected from the group consisting of a halogenated hydrocarbon solvent, a (hetero)aromatic solvent, an alkyl (hetero)aromatic solvent, a (hetero)aromatic ether, and an alkyl (hetero)aryl ether;
- the at least one alcohol contained in the composition C is selected from the group consisting of trifluoroethanol, hexafluoroisopropanol, and a mixture thereof;
- the base contained in the composition C is a pyridine, which is substituted with one or more electron withdrawing substituents selected from the group consisting of a halogen atom, a cyano group, an aldehyde group, a keto group, a carboxyester group, and a carboxamide group; and
- the strong acid contained in the composition C is selected from the group consisting of a carboxylic acid, a mineral acid, sulfonic acids, and a mixture thereof.

In some embodiments of the use and/or the method of the invention:
- the aprotic solvent contained in the composition C is selected from the group consisting of toluene, o-xylene, m-xylene, p-xylene, mesitylene, anisole, dichloromethane, and a mixture thereof;
- the at least one alcohol contained in the composition C is selected from the group consisting of trifluoroethanol, hexafluoroisopropanol, and a mixture thereof;
- the base contained in the composition C is a pyridine, in which exactly one hydrogen residue is substituted by an electron-withdrawing substituent selected from the group consisting of a cyano group and a halogen atom; and
- the strong acid contained in the composition C is selected from the group consisting of trifluoroacetic acid, hydrochloric acid, sulfonic acids, and a mixture thereof.

In some embodiments of the use and/or the method of the invention:
- the aprotic solvent contained in the composition C is selected from the group consisting of toluene, o-xylene, m-xylene, p-xylene, mesitylene, anisole, dichloromethane, and a mixture thereof;
- the at least one alcohol contained in the composition C is selected from the group consisting of trifluoroethanol, hexafluoroisopropanol, and a mixture thereof;
- the base contained in the composition C is a pyridine, in which exactly one hydrogen residue is substituted by an electron-withdrawing substituent selected from the group consisting of a cyano group and a halogen atom;
- the strong acid contained in the composition C is selected from the group consisting of trifluoroacetic acid, hydrochloric acid, sulfonic acids, and a mixture thereof;
- the total volume of the aprotic solvent contained in the liquid composition C accounts for 60-99% of the overall volume of the liquid composition C;
- the total volume of the alcohol contained in the liquid composition C accounts for 1-40% of the overall volume of the liquid composition C;
- the total molar amount of said strong acid is in the range of 0.80-15.0 equivalents relative to the total molar amount of the di(p-methoxyphenyl)phenylmethyl groups;
- the total molar amount of said alcohol is in the range of 2.0-150.0 equivalents relative to the total molar amount of nucleobases; and
- the total molar amount of said base is in the range of 1.0-3.0 equivalents relative to the total molar amount of said strong acid.

In some embodiments of the use and/or the method and/or the liquid composition C of the invention:
- the aprotic solvent contained in the composition C is selected from the group consisting of toluene, o-xylene, m-xylene, p-xylene, mesitylene, anisole, and a mixture thereof;
- the at least one alcohol contained in the liquid composition C is selected from the group consisting of trifluoroethanol, hexafluoroisopropanol, and a mixture thereof;
- the base contained in the composition C is a pyridine, in which exactly one hydrogen residue is substituted by an electron-withdrawing substituent selected from the group consisting of a cyano group and a halogen atom;
- the strong acid contained in the composition C is selected from the group consisting of trifluoroacetic acid, hydrochloric acid, sulfonic acids, and a mixture thereof;
- the total volume of the aprotic solvent contained in the liquid composition C accounts for 60-99% of the overall volume of the liquid composition C;
- the total volume of the alcohol contained in the liquid composition C accounts for 1-40% of the overall volume of the liquid composition C;
- the total molar amount of said strong acid is in the range of 0.80-15.0 equivalents relative to the total molar amount of the di(p-methoxyphenyl)phenylmethyl groups;
- the total molar amount of said alcohol is in the range of 2.0-150.0 equivalents relative to the total molar amount of nucleobases; and
- the total molar amount of said base is in the range of 1.0-3.0 equivalents relative to the total molar amount of said strong acid.

In some embodiments of the use and/or the method and/or the liquid composition C of the invention:
- the aprotic solvent contained in the composition C is selected from the group consisting of toluene, o-xylene, m-xylene, p-xylene, mesitylene, anisole, and a mixture thereof;
- the at least one alcohol contained in the liquid composition C is selected from the group consisting of trifluoroethanol, hexafluoroisopropanol, and a mixture thereof;
- the base contained in the composition C is selected from the group consisting of 4-cyanopyridine, 3-cyanopyridine, 4-chloropyridine, 3-chloropyridine, and a mixture thereof; and
- the strong acid contained in the composition C is selected from the group consisting of trifluoroacetic acid, hydrochloric acid, methanesulfonic acid, and a mixture thereof.

In some embodiments of the use and/or the method and/or the liquid composition C of the invention:
- the aprotic solvent contained in the composition C is selected from the group consisting of toluene, o-xylene, m-xylene, p-xylene, mesitylene, anisole, and a mixture thereof;
- the at least one alcohol contained in the liquid composition C is selected from the group consisting of trifluoroethanol, hexafluoroisopropanol, and a mixture thereof;
- the base contained in the composition C is selected from the group consisting of 4-cyanopyridine, 3-cyanopyridine, 4-chloropyridine, 3-chloropyridine, and a mixture thereof;
- the strong acid contained in the composition C is selected from the group consisting of trifluoroacetic acid, hydrochloric acid, methanesulfonic acid, and a mixture thereof;
- the total volume of the aprotic solvent contained in the liquid composition C accounts for 70-98% of the overall volume of the liquid composition C;
- the total volume of the alcohol contained in the liquid composition C accounts for 2-30% of the overall volume of the liquid composition C;
- the total molar amount of said strong acid is in the range of 1.0-13.0 equivalents relative to the total molar amount of the di(p-methoxyphenyl)phenylmethyl groups;
- the total molar amount of said alcohol is in the range of 2.0-100.0 equivalents relative to the total molar amount of nucleobases; and
- the total molar amount of said base is in the range of 1.0-2.0 equivalents relative to the total molar amount of said strong acid.

In some embodiments of the use and/or the method and/or the liquid composition C of the invention:
- the aprotic solvent contained in the composition C is selected from the group consisting of toluene, o-xylene, m-xylene, p-xylene, mesitylene, anisole, and a mixture thereof;
- the at least one alcohol contained in the liquid composition C is selected from the group consisting of trifluoroethanol, hexafluoroisopropanol, and a mixture thereof;
- the base contained in the composition C is selected from the group consisting of 4-cyanopyridine, 3-cyanopyridine, 4-chloropyridine, 3-chloropyridine, and a mixture thereof;
- the strong acid contained in the composition C is selected from the group consisting of trifluoroacetic acid, hydrochloric acid, methanesulfonic acid, and a mixture thereof;
- the total volume of the aprotic solvent contained in the liquid composition C accounts for 70-98% of the overall volume of the liquid composition C;
- the total volume of the alcohol contained in the liquid composition C accounts for 2-30% of the overall volume of the liquid composition C;
- the total molar amount of said strong acid is in the range of 2.0-12.5 equivalents relative to the total molar amount of the di(p-methoxyphenyl)phenylmethyl groups;
- the total molar amount of said alcohol is in the range of 3.0-95.0 equivalents relative to the total molar amount of nucleobases; and
- the total molar amount of said base is in the range of 1.00-1.50 equivalents relative to the total molar amount of said strong acid.

In some embodiments of the use and/or the method of the invention:
- the aprotic solvent contained in the composition C is selected from the group consisting of toluene, anisole, dichloromethane, and a mixture thereof;
- the at least one alcohol contained in the liquid composition C is selected from the group consisting of trifluoroethanol, hexafluoroisopropanol, and a mixture thereof;
- the base contained in the composition C is selected from the group consisting of 4-cyanopyridine, 3-cyanopyridine, 4-chloropyridine, 3-chloropyridine, and a mixture thereof; and
- the strong acid contained in the composition C is selected from the group consisting of trifluoroacetic acid, hydrochloric acid, methanesulfonic acid, and a mixture thereof.

In some embodiments of the use and/or the method of the invention:
- the aprotic solvent contained in the composition C is selected from the group consisting of toluene, anisole, dichloromethane, and a mixture thereof;
- the at least one alcohol contained in the liquid composition C is selected from the group consisting of trifluoroethanol, hexafluoroisopropanol, and a mixture thereof;
- the base contained in the composition C is selected from the group consisting of 4-cyanopyridine, 3-cyanopyridine, 4-chloropyridine, 3-chloropyridine, and a mixture thereof;
- the strong acid contained in the composition C is selected from the group consisting of trifluoroacetic acid, hydrochloric acid, methanesulfonic acid, and a mixture thereof;
- the total volume of the aprotic solvent contained in the liquid composition C accounts for 81-98% of the overall volume of the liquid composition C;
- the total volume of the alcohol contained in the liquid composition C accounts for 2-19% of the overall volume of the liquid composition C;
- the total molar amount of said strong acid is in the range of 2.0-12.5 equivalents relative to the total molar amount of the di(p-methoxyphenyl)phenylmethyl groups;
- the total molar amount of said alcohol is in the range of 3.0-95.0 equivalents relative to the total molar amount of nucleobases; and
- the total molar amount of said base is in the range of 1.000-1.050 equivalents relative to the total molar amount of said strong acid.

In some embodiments of the use and/or the method and/or the liquid composition C of the invention:
- the aprotic solvent contained in the composition C is selected from the group consisting of toluene, anisole, and a mixture thereof;
- the at least one alcohol contained in the liquid composition C is selected from the group consisting of trifluoroethanol, hexafluoroisopropanol, and a mixture thereof;
- the base contained in the composition C is selected from the group consisting of 4-cyanopyridine, 3-cyanopyridine, 4-chloropyridine, 3-chloropyridine, and a mixture thereof; and
- the strong acid contained in the composition C is selected from the group consisting of trifluoroacetic acid, hydrochloric acid, methanesulfonic acid, and a mixture thereof.

In some embodiments of the use and/or the method and/or the liquid composition C of the invention:
- the aprotic solvent contained in the composition C is selected from the group consisting of toluene, anisole, and a mixture thereof;
- the at least one alcohol contained in the liquid composition C is selected from the group consisting of trifluoroethanol, hexafluoroisopropanol, and a mixture thereof;
- the base contained in the composition C is selected from the group consisting of 4-cyanopyridine, 3-cyanopyridine, 4-chloropyridine, 3-chloropyridine, and a mixture thereof;
- the strong acid contained in the composition C is selected from the group consisting of trifluoroacetic acid, hydrochloric acid, methanesulfonic acid, and a mixture thereof;
- the total volume of the aprotic solvent contained in the liquid composition C accounts for 81-98% of the overall volume of the liquid composition C;
- the total volume of the alcohol contained in the liquid composition C accounts for 2-19% of the overall volume of the liquid composition C;
- the total molar amount of said strong acid is in the range of 2.0-12.5 equivalents relative to the total molar amount of the di(p-methoxyphenyl)phenylmethyl groups;
- the total molar amount of said alcohol is in the range of 3.0-95.0 equivalents relative to the total molar amount of nucleobases; and
- the total molar amount of said base is in the range of 1.000-1.050 equivalents relative to the total molar amount of said strong acid.

In some embodiments of the method of the invention,
- step a) comprises providing a compound according to formula I 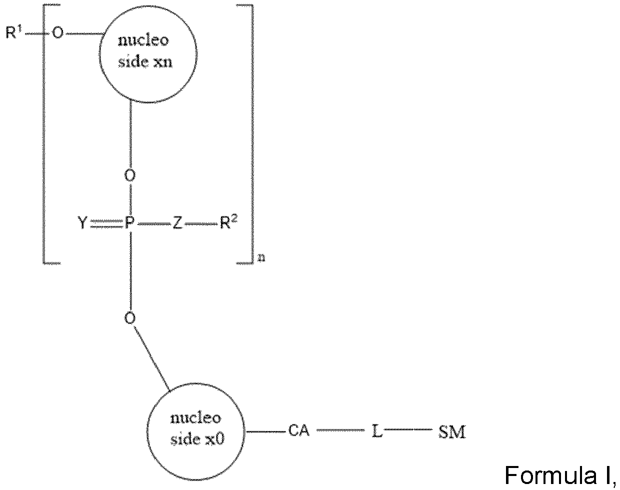 wherein:
   R¹ is a di(p-methoxyphenyl)phenylmethyl protecting group ;
   n is an integer equal to or larger than 0;
   Y is selected independently for each repetitive unit n from the group consisting of S and O;
   Z is selected independently for each repetitive unit n from the group consisting of O and S;
   R² is a protecting group, which may be the same or different for each repetitive unit n;
   each of the nucleosides x0 to xn may be the same or different;
   CA is a capping moiety or single bond;
   L is a linker moiety or single bond; and
   SM is a supporting moiety;
- step c) comprises reacting the free hydroxyl group resulting from step b) with a nucleoside or oligonucleotide phosphoramidite building block, which building block comprises a backbone hydroxyl moiety protected by a di(p-methoxyphenyl)phenylmethyl protecting group, thereby producing a covalent linkage between the oxygen atom of said free hydroxyl group and the phosphorus (III) atom of said building block, wherein;
   the nucleoside or oligonucleotide phosphoramidite building block is a compound according to formula II: 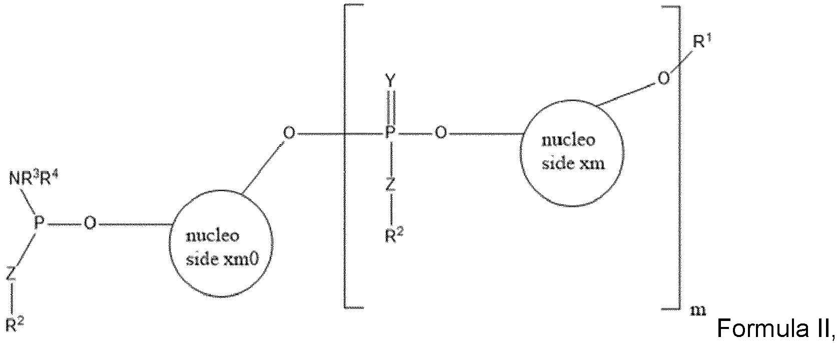 wherein:
   R¹ is a di(p-methoxyphenyl)phenylmethyl protecting group ;
   m is an integer equal to or larger than 0;
   Y is selected independently for each repetitive unit m from the group consisting of S and O;
   Z is selected independently for each position from the group consisting of O and S;
   R² is a protecting group, which may be the same or different for each position;
   R³ and R⁴ are protecting groups, which may be the same or different; and each of the nucleosides xm0 to xm may be the same or different; and
- step e) comprises optionally reiterating the series of steps b) through d).

In some embodiments of the method of the invention,
- step a) comprises providing a compound according to formula I-a 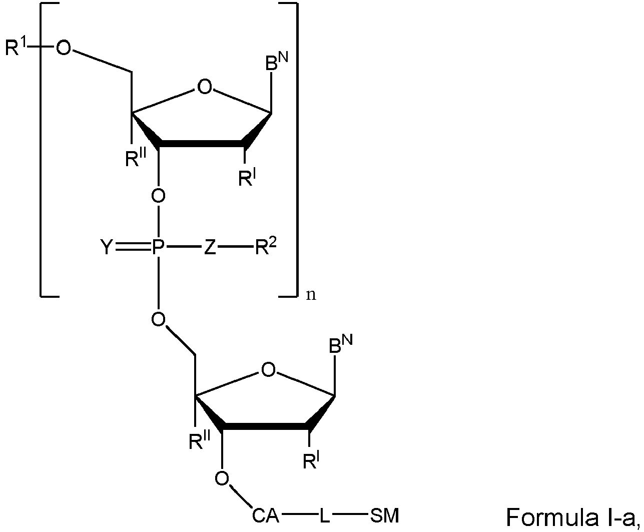 wherein:
   R¹ is a di(p-methoxyphenyl)phenylmethyl protecting group ;
   n is an integer equal to or larger than 0;
   Y is selected independently for each repetitive unit n from the group consisting of S and O;
   Z is selected independently for each repetitive unit n from the group consisting of O and S;
   R² is a protecting group, which may be the same or different for each repetitive unit n;
   CA is a capping moiety or single bond;
   L is a linker moiety or single bond; and
   SM is a supporting moiety;
   B^{N} is a nucleobase which may be the same or different at each occurrence;
   R¹ is selected independently at each occurrence from the group consisting of H, F, -O-(C₁-C₅ alkyl), -O-(C₁-C₅ alkyl)-O-(C₁-C₅ alkyl), -O-Si(C₁-C₅ alkyl)₃, and -O-CH₂-O-Si(C₁-C₅ alkyl)₃; and
   R^{II} is independently at each occurrence H or R^{II} and R^{I} together form a methylene or ethylene bridge of the chemical structure +-CH₂-O-++, +-CH(CH₃)-O-++, or +-CH₂-CH₂-O-++, where + is the point of attachment to the 4'-carbon atom (i.e. the carbon atom to which R^{II} is bonded) and ++ is the point of attachment to the 2'-carbon (i.e. the carbon atom to which R^{I} is bonded); and
- step c) comprises reacting the free hydroxyl group resulting from step b) with a nucleoside or oligonucleotide phosphoramidite building block, which building block comprises a backbone hydroxyl moiety protected by a di(p-methoxyphenyl)phenylmethyl protecting group, thereby producing a covalent linkage between the oxygen atom of said free hydroxyl group and the phosphorus (III) atom of said building block, wherein;
   the nucleoside or oligonucleotide phosphoramidite building block is a compound according to formula II-a: 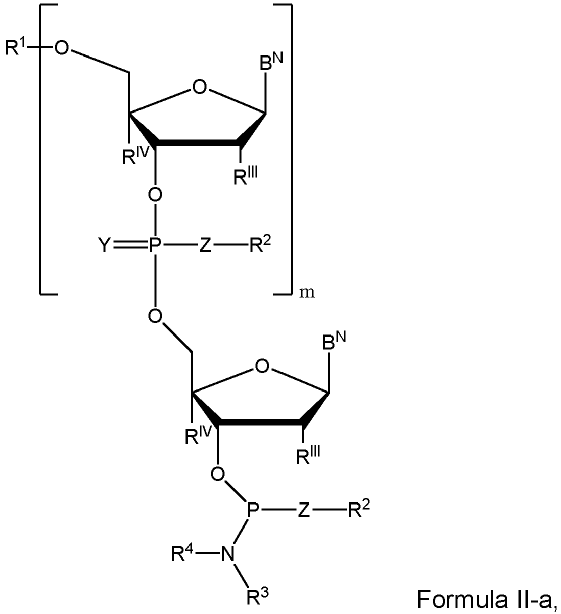 wherein:
   R¹ is a di(p-methoxyphenyl)phenylmethyl protecting group ;
   m is an integer equal to or larger than 0;
   Y is selected independently for each repetitive unit m from the group consisting of S and O;
   Z is selected independently for each position from the group consisting of O and S;
   R² is a protecting group, which may be the same or different for each position;
   R³ and R⁴ are protecting groups, which may be the same or different;
   B^{N} is a nucleobase which may be the same or different at each occurrence;
   R^{III} is selected independently at each occurrence from the group consisting of H, F, -O-(C₁-C₅ alkyl), -O-(C₁-C₅ alkyl)-O-(C₁-C₅ alkyl), -O-Si(C₁-C₅ alkyl)₃, and -O-CH₂-O-Si(C₁-C₅ alkyl)₃; and
   R^{IV} is independently at each occurrence H or R^{III} and R^{IV} together form a methylene or ethylene bridge of the chemical structure +-CH₂-O-++, +-CH(CH₃)-O-++, or +-CH₂-CH₂-O-++, where + is the point of attachment to the 4'-carbon atom (i.e. the carbon atom to which R^{IV} is bonded) and ++ is the point of attachment to the 2'-carbon (i.e. the carbon atom to which R^{III} is bonded); and
- step e) comprises optionally reiterating the series of steps b) through d).

In some embodiments of the method of the invention,
- step a) comprises providing a compound according to formula I-a 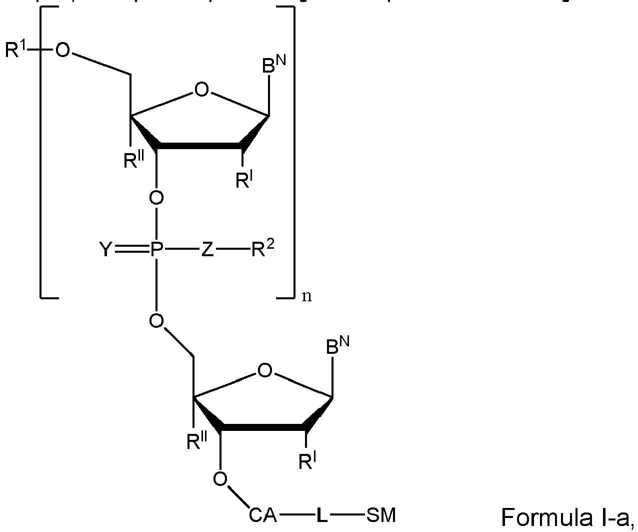 wherein:
   R¹ is a di(p-methoxyphenyl)phenylmethyl protecting group ;
   n is an integer in the range of 0-150;
   Y is selected independently for each repetitive unit n from the group consisting of S and O;
   Z is for each repetitive unit n O;
   R² is at each occurrence a 2-cyanoethyl group;
   CA is a single bond;
   L is a linker moiety or single bond; and
   SM is a supporting moiety;
   B^{N} is selected independently at each occurrence from the group consisting of adenine, guanine, cytosine, thymine, and uracil;
   R^{I} is selected independently at each occurrence from the group consisting of H, F, and -O-CHs (i.e. methoxy); and
   R^{II} is H at each occurrence; and
- step c) comprises reacting the free hydroxyl group resulting from step b) with a nucleoside or oligonucleotide phosphoramidite building block, which building block comprises a backbone hydroxyl moiety protected by a di(p-methoxyphenyl)phenylmethyl protecting group, thereby producing a covalent linkage between the oxygen atom of said free hydroxyl group and the phosphorus (III) atom of said building block, wherein;
   the nucleoside or oligonucleotide phosphoramidite building block is a compound according to formula II-a: 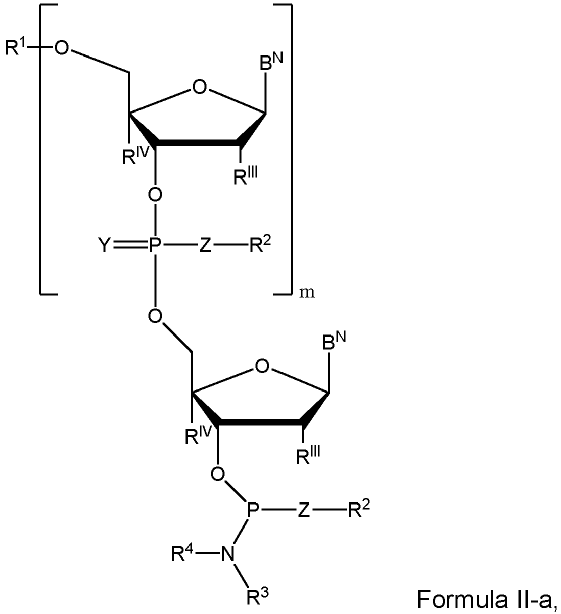 wherein:
   R¹ is a di(p-methoxyphenyl)phenylmethyl protecting group ;
   m is an integer in the range of 0-2;
   Y is selected independently for each repetitive unit m from the group consisting of S and O;
   Z is for each position O;
   R² is at each occurrence a 2-cyanoethyl group;
   R³ and R⁴ are both isopropyl groups;
   B^{N} is selected independently at each occurrence from the group consisting of adenine, guanine, cytosine, thymine, and uracil;
   R^{III} is selected independently at each occurrence from the group consisting of H, F, and -O-CHs (i.e. methoxy); and
   R^{IV} is H at each occurrence; and
- step e) comprises optionally reiterating the series of steps b) through d).

The following Figures and Examples, including the experiments conducted and the results achieved, are provided for illustrative purposes only and are not to be construed as limiting the scope of the claims.

The invention for example pertains to the following phrases:
1. Use of a liquid composition C comprising an aprotic solvent, at least one alcohol selected from the group consisting of trifluoroethanol, hexafluoroisopropanol, pentafluoropropanol, 1,1,1,3,3,3-hexafluoro-2-methyl-2-propanol, and nonafluoro tertiary butyl alcohol, and a salt of a base with a strong acid, wherein the salt's cation has a pKa in the range of 1 to 4 and the strong acid has a pKa of less than 1, for suppressing nucleobase cleavage while effecting cleavage of a di(p-methoxyphenyl)phenylmethyl protecting group from a hydroxyl group during the chemical synthesis of an oligonucleotide.
2. A method for the synthesis of oligonucleotides, comprising the following steps a) through f):
   a) providing a nucleoside or oligonucleotide bound to a supporting moiety, wherein the nucleoside or oligonucleotide comprises a backbone hydroxyl moiety, which is protected by a di(p-methoxyphenyl)phenylmethyl protecting group;
   b) cleaving the di(p-methoxyphenyl)phenylmethyl protecting group from the nucleoside or oligonucleotide by incubating said compound with a liquid composition C, thereby generating a free backbone hydroxyl moiety;
   c) reacting the free backbone hydroxyl group resulting from step b) with a phosphorus moiety of a nucleoside or oligonucleotide building block, which building block further comprises a hydroxyl group protected by a di(p-methoxyphenyl)phenylmethyl protecting group, thereby producing a covalent linkage between the oxygen atom of said free hydroxyl group and the phosphorus atom of said building block;
   d) optionally modifying the phosphorus moiety;
   e) optionally reiterating the sequence of steps b) to c) or b) to d); and
   f) cleaving the oligonucleotide from the supporting moiety;
   wherein the liquid composition C comprises an aprotic solvent, at least one alcohol selected from the group consisting of trifluoroethanol, hexafluoroisopropanol, pentafluoropropanol, 1,1,1,3,3,3-hexafluoro-2-methyl-2-propanol, and nonafluoro tertiary butyl alcohol, and a salt of a base with a strong acid, wherein the salt's cation has a pKa in the range of 1 to 4 and the strong acid has a pKa of less than 1.
3. The method according to phrase 2, wherein:
   - the phosphorus moiety of the nucleoside or oligonucleotide building block used in step c) is a phosphorus (III) moiety;
   - the method comprises a further step d) of oxidizing or sulfurizing the phosphorus (III) atom to a phosphorus (V) atom, thereby creating the desired type of internucleoside linkage; and
   - step e) comprises optionally either reiterating the sequence of steps b) through d) or reiterating the sequence of steps b) and c) before executing step d).
4. The method according to any one of phrases 2 to 3, wherein:
   - step a) comprises providing a compound according to formula I 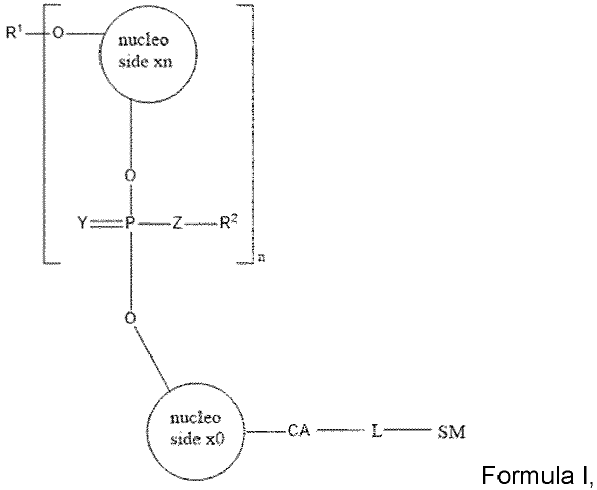 wherein:
      R¹ is a di(p-methoxyphenyl)phenylmethyl protecting group ;
      n is an integer equal to or larger than 0;
      Y is selected independently for each repetitive unit n from the group consisting of S and O;
      Z is selected independently for each repetitive unit n from the group consisting of O and S;
      R² is a protecting group, which may be the same or different for each repetitive unit n;
      each of the nucleosides x0 to xn may be the same or different;
      CA is a capping moiety or single bond;
      L is a linker moiety or single bond; and
      SM is a supporting moiety;
   - step c) comprises reacting the free hydroxyl group resulting from step b) with a nucleoside or oligonucleotide phosphoramidite building block, which building block comprises a backbone hydroxyl moiety protected by a di(p-methoxyphenyl)phenylmethyl protecting group, thereby producing a covalent linkage between the oxygen atom of said free hydroxyl group and the phosphorus (III) atom of said building block; and
   - step e) comprises optionally reiterating the series of steps b) through d).
5. The method according to any one of phrases 2 to 4, wherein the nucleoside or oligonucleotide building block is a compound according to formula II 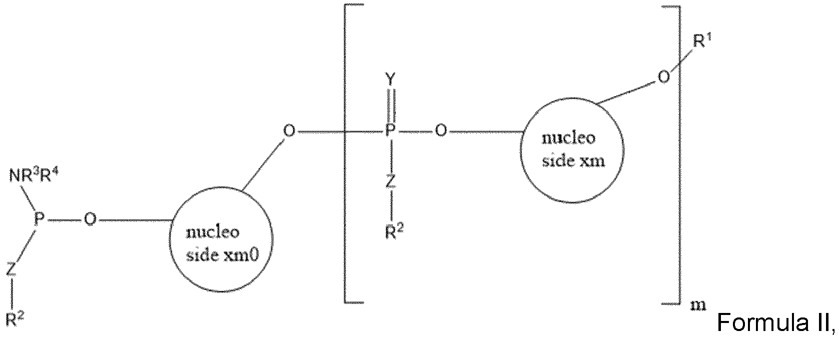 wherein:
   R¹ is a di(p-methoxyphenyl)phenylmethyl protecting group ;
   m is an integer equal to or larger than 0;
   Y is selected independently for each repetitive unit m from the group consisting of S and O;
   Z is selected independently for each position from the group consisting of O and S;
   R² is a protecting group, which may be the same or different for each position;
   R³ and R⁴ are protecting groups, which may be the same or different; and each of the nucleosides xm0 to xm may be the same or different.
6. The method according to any one of phrases 2 to 3, wherein:
   - step a) comprises providing a compound according to formula III 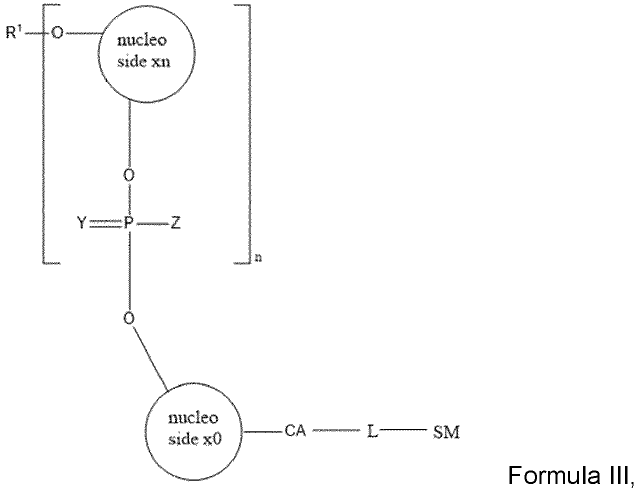 wherein:
      R¹ is a di(p-methoxyphenyl)phenylmethyl protecting group;
      n is an integer equal to or larger than 0;
      Y is O; and
      Z is H;
      each of the nucleosides x0 to xn may be the same or different;
      CA is a capping moiety or a single bond;
      L is a linker moiety or a single bond; and
      SM is a supporting moiety;
   - step c) comprises reacting the free hydroxyl group resulting from step b) with a H-phosphonate building block, which building block comprises a backbone hydroxyl moiety protected by a di(p-methoxyphenyl)phenylmethyl protecting group, thereby producing a covalent linkage between the oxygen atom of said free hydroxyl group and the phorsphorus (III) atom of said building block; and
   - step e) comprises optionally assembling a desired oligonucleotide sequence by reiterating the sequence of steps b) and c) before executing step d).
7. The method according any one of phrases 2 to 6, further comprising a step g) of blocking unreacted free hydroxyl groups after step c) or after step d).
8. The method according to any one of phrases 2 to 7, wherein at least step b) is carried out in a batch reactor.
9. The method according to any one of phrases 2 to 7, wherein the method is carried out in a column reactor and the flow rate of the liquid composition C through the column reactor is below 300 cm/h in at least one iteration of step b).
10. The method according to any one of phrases 2 to 9, wherein the synthesis is carried out at a scale of 100 mmol oligonucleotide product or above.
11. The method according to any one of phrases 2 to 10, wherein the method further comprises the following step h):
   h) isolating the support-cleaved oligonucleotide.
12. The method according to any one of phrases 2 to 11 or the use according to claim 1, wherein the aprotic solvent contained in the composition C is selected from the group consisting of a halogenated hydrocarbon solvent, a (hetero)aromatic solvent, an alkyl (hetero)aromatic solvent, a (hetero)aromatic ether, and an alkyl (hetero)aryl ether.
13. The method according to any one of phrases 2 to 12 or the use according to any one of claims 1 and 12, wherein the aprotic solvent contained in the composition C is non-halogenated.
14. A liquid composition C for use in the cleavage of a di(p-methoxyphenyl)phenylmethyl protecting group from a hydroxyl group, the composition comprising an aprotic solvent, at least one alcohol selected from the group consisting of trifluoroethanol, hexafluoroisopropanol, pentafluoropropanol, 1,1,1,3,3,3-hexafluoro-2-methyl-2-propanol, and nonafluoro tertiary butyl alcohol, and a salt of a base with a strong acid, wherein the salt's cation has a pKa in the range of 1 to 4 and the strong acid has a pKa of less than 1, and the aprotic solvent is non-halogenated and selected from the group consisting of a (hetero)aromatic solvent, an alkyl (hetero)aromatic solvent, a (hetero)aromatic ether, and an alkyl (hetero)aryl ether.
15. The method according to any one of phrases 2 to 13 or the use according to any one of phrases 1, 12, or 13, or the liquid composition according to phrase 14, wherein the base contained in the composition C is a cyclic amine, preferably wherein the cyclic amine is selected from the group consisting of a pyridine, a pyrimidine, a pyrazine, a thiazole, a pyridazine, a pyrazole, and a triazole.
16. The method according to any one of phrases 2 to 13 or 15 or the use according to any one of phrases 1, 12, 13, or 15 or the liquid composition according to any one of phrases 14 to 15, wherein the base contained in the composition C is a pyridine, which is substituted with one or more electron withdrawing substituents selected from the group consisting of a halogen atom, a cyano group, an aldehyde group, a keto group, a carboxyester group, and a carboxamide group.
17. The method according to any one of phrases 2 to 13 or 15 to 16, or the use according to any one of phrases 1, 12, 13 or 15 to 16, or the liquid composition according to any one of phrases 14 to 16, wherein the base is selected from the group consisting of 4-cyanopyridine, 3-cyanopyridine, 4-chloropyridine, 3-chloropyridine, and a mixture thereof.
18. The method according to any one of phrases 2 to 13 or 15 to 17 or the use according to any one of phrases 1, 12, 13 or 15 to 17, or the liquid composition according to any one of phrases 14 to 17, wherein the strong acid contained in the composition C is selected from the group consisting of a carboxylic acid, a mineral acid, sulfonic acids, and a mixture thereof.
19. The method according to any one of phrases 2 to 13 or 15 to 18, or the use according to any one of phrases 1, 12, 13 or 15 to 18, or the liquid composition according to any one of phrases 14 to 18, wherein the strong acid contained in the composition C is selected from the group consisting of trifluoroacetic acid, hydrochloric acid, sulfonic acids, and a mixture thereof, preferably wherein the strong acid is selected from the group consisting of trifluoroacetic acid, hydrochloric acid, methanesulfonic acid, and a mixture thereof.
20. The method according to any one of phrases 2 to 13 or 15 to 19, or the use according to any one of phrases 1, 12, 13 or 15 to 19, or the liquid composition according to any one of phrases 14 to 19, wherein the aprotic solvent contained in the composition C is selected from the group consisting of toluene, o-xylene, m-xylene, p-xylene, mesitylene, anisole, and a mixture thereof.
21. The method according to any one of phrases 2 to 13 or 15 to 20, or the use according to any one of phrases 1, 12, 13 or 15 to 20, or the liquid composition according to any one of phrases 14 to 20, wherein the at least one alcohol contained in the liquid composition C is selected from the group consisting of trifluoroethanol, hexafluoroisopropanol, and a mixture thereof.
22. The method according to any one of phrases 2 to 13 or 15 to 21 or the use according to any one of phrases 1, 12, 13 or 15 to 21, or the liquid composition according to any one of phrases 14 to 21, wherein:
   - the total volume of the aprotic solvent contained in the liquid composition C accounts for 60-99% of the overall volume of the liquid composition C; and
   - the total volume of the alcohol contained in the liquid composition C accounts for 1-40% of the overall volume of the liquid composition C.
23. The method according to any one of phrases 2 to 13 or 15 to 22 or the use according to any one of phrases 1, 12, 13 or 15 to 22, or the liquid composition according to any one of phrases 14 to 22, wherein the molar concentration of said alcohol in the composition C is at least 2 times more than the molar concentration of said base contained in the composition C.
24. The method according to any one of phrases 2 to 13 or 15 to 23 or the use according to any one of phrases 1, 12, 13 or 15 to 23, or the liquid composition according to any one of phrases 14 to 23, wherein in the liquid composition C:
   - the total molar amount of said strong acid is in the range of 0.80-15.0 equivalents relative to the total molar amount of the di(p-methoxyphenyl)phenylmethyl groups; and
   - the total molar amount of said alcohol is in the range of the 2.0-100.0 equivalents relative to the total molar amount of nucleobases.

### Description of the Figures

**Figure 1** Non-limiting examples of building blocks suitable for the synthesis of oligonucleotides with a phosphoribose backbone.
   (a) Building block for synthesis of DNA by the phosphoramidite approach;
   (b) Building block for synthesis of RNA by the phosphoramidite approach (The terms "Dmt" and "DMT" are used interchangeably herein);
   (c) Phosphoramidite building block with 2'-O-methyl ribose;
   (d) Phosphoramidite building block with 2'-O-(2-methoxyethyl) ribose;
   (e) Phosphoramidite building block with 2'-fluoro ribose.
**Figure 2** shows phosphoramidite building blocks for the synthesis of oligonucleotide analogs containing ribose surrogates (a) protected building block for obtaining arabinose nucleic acid (ANA), (b) building block for obtaining 2'-fluoroarabinose nucleic acid (FANA), (c) building block for obtaining threose nucleic acid (TNA). The following types of building blocks can be obtained as stereoisomers, the displayed structures represent one of these compounds: (d) building block for obtaining 1',3'-anhydrothreitol nucleic acid (HNA), (e) building block for obtaining locked nucleic acid (LNA), (f) building block for obtaining ethylene-bridged nucleic acid (ENA), (g) building block for obtaining tricyclic nucleic acid (tc-DNA), (h) building block for obtaining cyclohexene nucleic acid (CeNA), (i) shows a building block in which ribose has been replaced by glycerol (GNA). The protected building block (j) for obtaining unlocked nucleic acid (UNA) is obtained by oxidative ring cleavage of ribose.
**Figure 3** Analytical HPLC traces of crude 5'-TTT TAT TTT T-3' (SEQ ID NO: 1). Dashed line - EOP4149994/02: DMT deprotection using 10% DCA in toluene (2 × 10 minutes); purity (HPLC): 89%.
   Solid line - EOP4149994/01: DMT deprotection using cleavage cocktail (2 × 10 minutes); purity (HPLC): 93%. No capping was performed in each of the coupling cycles.
**Figure 4** Analytical HPLC traces of crude 5'-ATA CCG ATT AAG CGA AGT TT-3' (SEQ ID NO: 2). Dashed line - EOP4149467/22: DMT deprotection using 10% DCA in toluene (2 × 10 minutes); purity (HPLC): 50%.
   Solid line - EOP4149467/24: DMT deprotection using cleavage cocktail (2 × 10 minutes; purity (HPLC): 64%. No capping was performed in each of the coupling cycles.
**Figure 5a****,b** Analytical HPLC traces of crude 5'-ATA CCG ATT AAG CGA AGT TT-3' (SEQ ID NO: 2). Dashed line - EOP4149467/01: DMT deprotection using 10% DCA in toluene (1 × 1 minutes); purity (HPLC): 68%.
   Solid line - EOP4149467/02: DMT deprotection using cleavage cocktail (2 × 10 minutes); purity (HPLC): 79%.
   Figure 5b is a zoomed view of figure 5a, where the traces corresponding to both samples have been superimposed.
**Figure 6a****,b** Analytical HPLC-MS traces of crude 5'-AAA AT-3'. Solid line - EOP4153156/03-5: DMT deprotection using 2.0% DCA, 0.1% ethanol in DCM (v/v) (5 + 20 min); purity (HPLC-MS UV trace): 91%. Dashed line - EOP4153156/04-5: DMT deprotection using cleavage cocktail of the present invention (5 + 20 min); purity (HPLC-MS UV trace): 94%.
   Figure 6b is a zoomed view of figure 6a, wherein peaks have been assigned a peak ID as listed in Tables B.1 and B.2.
**Figure 7a**,b Analytical HPLC traces of crude 5'-[mAs][fCs][mA][fA][fA][fA][mG][fC][mA][fA][mA][mA][mC][fA][mG][fG][mU][fC][mU][ mA][mGs][mAs][mA]-3'. Solid line - EOP4150430/28-1: DMT deprotection using cleavage cocktail of the present invention (5 + 5 + 5 + 20 min) in last cycle; purity (HPLC-MS UV trace): 78%. Dashed line - EOP4150430/36-1: DMT deprotection using 10% DCA in toluene (5 + 5 + 5 + 20 min) in last cycle; purity (HPLC-MS UV trace): 50%.
   Figure 7b is a zoomed view of figure 7a, wherein the target product and the peak range containing the depurination products described in Table C.1 and C.2 are marked.

The invention is illustrated by the examples, Figures and claims.

### Examples

### General methods

### 1. Determination of weight gain and yield

To determine the theoretical weight gain the mass of the dimethoxytrityl group was subtracted from the amount of starting resin according to the scale of the synthesis. The sum of the β-cyanoethyl phosphatetriester of the base protected nucleosides were added according to the coupling cycles applied for the sequence. The experimental weight gain was calculated as the difference between the determined mass of the dried resin minus the mass of the starting resin, in which the dimethoxytrityl group was already subtracted. The yield was given in percent as the ratio between the experimental to the theoretical weight gain.

In more detail, the experimental weight gain was determined by subtracting the mass of the solid support from the mass of the dried on-resin oligonucleotide after completion of all elongation cycles and the final dimethoxytrityl removal step. To determine the mass of the solid support, the mass of the dimethoxytrityl groups was subtracted from the mass of the starting resin (which consists of the solid support with dimethoxytrityl groups) according to the scale of the synthesis and the molecular weight of the dimethoxytrityl group. The theoretical maximum weight gain was determined as the sum of the β-cyanoethyl-protected phosphate triesters of the nucleobase protected nucleosides according to the coupling cycles applied for the sequence, assuming a quantitative yield in all steps. Unless indicated differently, yields are given in percent (%) and were obtained by dividing the experimental weight gain by the theoretical maximum weight gain, followed by multiplication with 100% to arrive at a value in percent.

### 2. Determination of purity

Unless indicated differently, oligonucleotide purity was determined by analytical reversed phase HPLC using a C18 stationary phase, and elution was effected by a gradient (eluent A: HFIP/TEA/H₂O; eluent B: methanol). The detection wavelength was 260 nm. Unless specified otherwise, the reported oligonucleotide purities in percent (%) were obtained by dividing the area underneath the peak of the target oligonucleotide by the sum of areas of all significant peaks, followed by multiplication with 100% to arrive at a value in percent (%). Unless indicated differently, the UV trace was integrated applying a minimal relative area threshold of 0.05%. Unless indicated differently, the determination of purity was performed using the support-cleaved oligonucleotides. Such support-cleaved oligonucleotides were obtained as stated in section "3. Synthesis of oligonucleotides", unless indicated differently.

### Depurination assay

The impact of elongated contact times on the depurination of A and G was assessed by comparing the detritylation cocktail with 10% dichloroacetic acid (DCA) in toluene and cleavage times of 2 × 10 min. As adenosine is the most prone to depurination, the following sequences were chosen.

A capping step was omitted in all synthesis cycles. Therefore, any observed strand breaks are expected to be a consequence of the different detritylation protocols.

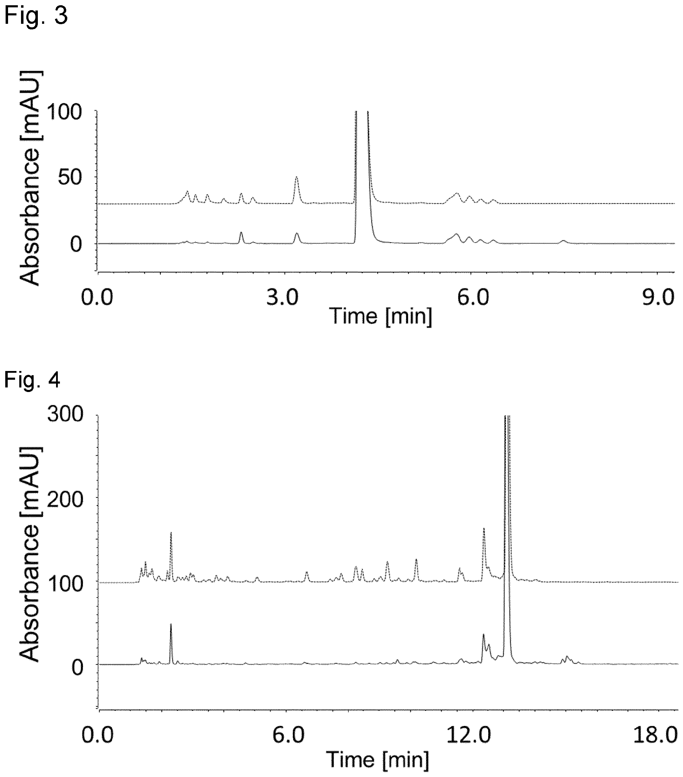

Superiority of deprotection cocktail could be shown by comparison with standard conditions, 10% DCA in toluene and cleavage times of 2 × 10 minutes. For the sequence 5'-TTT TAT TTT T-3' (SEQ ID NO: 1) an increase in purity of 4% could be observed (cf. Fig. 3). In case of the 10% DCA protocol a species of 1537 Da [M-H]⁻ was detected by LC-MS. This species was assigned to a 5'-phosphorylated-TTT TT-3' sequence. It is believed that this species is a consequence of depurination.

For the more challenging 20-mere 5'-ATA CCG ATT AAG CGA AGT TT-3' (SEQ ID NO: 2) an increase in purity of 14% was observed (cf. Fig. 4).

### 3. Synthesis of oligonucleotides

### General Protocol (GP) A: Preparation of the phosphoramidite solution

The 5'-DMT protected nucleoside phosphoramidite derivatives with optional 2'-modifications (2'-deoxy, 2'-fluoro or 2'-O-methyl, i.e. 2'-methoxy) of the benzoyl protected adenosine (A^{Bz}), benzoyl protected cytidine (C^{Bz}) [or acetyl protected cytidine (C^{Ac}) if indicated specifically], isobutyryl protected guanosine (G^{iBu}), thymidine (T), and uridine (U), were typically dissolved at a concentration of 0.2 M in dry acetonitrile or in a combination of dry acetonitrile and N,N-dimethylformamide. Molecular sieves (3 Å) were added and the headspace was flushed with nitrogen. The solution was then dried for a minimum of 12 h.

### General Protocol (GP) B: Detritylation using the amine salt

The detritylation cocktails were typically prepared by dissolving the base and the strong acid or a pre-formed salt composed of the base and the strong acid in a mixture of the indicated aprotic solvent, the fluorinated alcohol, and, where applicable, an additional carbocation scavenger. The ratio of these components in the respective detritylation cocktail is indicated for each synthetic example compiled in the following tables.

The detritylation cocktails were typically mixed as follows: The base (0.75-1.6%, w/v) and the strong acid (0.5-1.0%, v/v) or alternatively a pre-formed salt form (5.0%, w/v) were added to a solution of fluorinated alcohol (2-19%, v/v) and optionally a carbocation scavenger in dichloromethane (DCM), toluene, or anisole (76-96%, v/v). The following bases were used: 4-cyanopyridine (0.75-1.0%, w/v), 3-cyanopyridine (1.0%, w/v), 3-chloropyridine (1.6 %, w/v). The following strong acids were used: trifluoroacetic acid (TFA) (0.5-1.0%, v/v), methanesulfonic acid (0.8%, w/v)). The following pre-formed salt of a base with a strong acid was used: 4-chloropyridinium hydrochloride (5.0%, w/v). As fluorinated alcohol 2,2,2-trifluoroethanol (TFE) (5-19%, v/v) or 1,1,1,3,3,3-hexafluoroisopropanol (HFIP) (2-5%, v/v) was used. The following carbocation scavengers were used: ethanedithiol (EDT) (1.4%, v/v), 1,8-octanedithiol (DODT) (2.7%, v/v), 1,4-dithioerythrol (DTE) (2.3%, w/v), 1,4-benzenedimethanthiol (BDMT) (2.5%, w/v), H₂O (1%, v/v) and triethylsilane (TES) (1.9%, v/v).

As an example, a detritylation cocktail described as "1.0:0.7:19:80, 4-cyanopyridine:TFA.TFE:toluene, m:v:v:v" refers to a solution as obtained from dissolving 4-cyanopyridine (1.0% (m/v)) in a mixture of TFE (19% (v/v)), TFA (0.7% (v/v)), and toluene (80.3% (v/v)). Unless indicated differently, a ratio written as "m:v:v:v" refers to ratios of masses in g to volumes in mL.

Herein, the expressions "m:v" and "w:v" are used interchangeably, wherein m and w refer to the mass (in g) and v refers to the volume (in mL), unless indicated differently. Along the same lines, the expressions "m/v" and "w/v" are herein used interchangeably, wherein again, m and w refer to the mass (in g) and v refers to the volume (in mL), unless indicated differently.

Prior to detritylation, the resin was typically washed with the solvent, e.g. DCM or toluene. The detritylation was performed using the detritylation cocktail, vide supra. The protected oligonucleotide on the resin was deprotected 1-4 times or 2-3 times, typically for 5-20 min each, and for solid supports loaded with UnyLinker^{™} for typically 30-70 min each. The resin was then typically washed with acetonitrile.

General Protocol (GP) C: Detritylation using 10% dichloroacetic acid (DCA) in toluene (v/v).

The detritylation was typically performed in one step using commercially available 10% DCA in toluene (v/v) for 1 min or in two steps for 2 × 10 min (i.e. two iterations with 10 min each). The resin was then typically washed with acetonitrile.

General Protocol (GP) D: Detritylation using 2% DCA and 0.1% ethanol in dichloromethane (DCM) (v/v).

The detritylation was typically performed once with a contact time of 310 s or in two steps (i.e. two iterations) of 10 + 10 min, 5 + 5 min, 5 + 10 min, or 5 + 20 min. The resin was then typically washed with acetonitrile.

General Protocol (GP) E: Detritylation using 10% DCA in toluene (v/v).

Detritylation with 10% DCA in toluene (v/v) was typically performed in 2-4 steps (i.e 2-4 iterations). The detritylation times were typically 5-20 min per iteration. For solid supports loaded with UnyLinker^{™}, the detritylation time was typically 5 + 5 + 20 min per iterations, which indicates 3 steps (i.e. iterations) of which in the first and second steps the detritylation solution was contacted with the resin for 5 min each, and in the third step for 20 min. The resin was typically washed with acetonitrile after completion of the detritylation procedure.

### Standard protocol for synthesis of the oligonucleotides:

Unless indicated differently, the synthesis was carried out manually in a 10 mL syringe reactor (R-1), manually in a 1 L stirred bed reactor (R-2), or automated in 25 mL sparged bed reactors (agitation by bubbling N₂ gas into a filter reactor) (R-3) using 3'-phosphoramidite nucleosides according to the desired sequence from 3' to 5' direction. All reactor types were equipped with filters, frits, or membranes of appropriate pore sizes for retaining the resin in the reactor and draining solvents and reagents.

The synthesis was typically executed at a scale of 40 µmol, 2.5 mmol, or from 0.04 - 5.0 mmol based on the resin weight and substitution (approx. 0.34-0.41 mmol/g or 0.2-0.4 mmol/g). Commercially available DMT-protected polystyrene starting resins (functionalized with either the first nucleoside or UnyLinker^{™}) were typically used.

The nucleoside and UnyLinker^{™} groups of the commercially available starting resins were typically linked to the polystyrene support by a succinic acid ester or amide.

Prior to the first coupling cycle, the resin was transferred to the reactor and washed with acetonitrile (ACN, 40-50 mL/g) for 10 min. For each of the nucleoside phosphoramidites to be added, one coupling cycle according to Table 1 was carried out, unless indicated differently.

**Table 1: Typical synthesis cycle (i.e. coupling cycle or elongation cycle) for the synthesis of oligonucleotides.**

| step | repetition (i.e. number of iterations) | volume per mass resin | Time per repetition |
|---|---|---|---|
| detritylation (GP B, C, D, or E) | 1-3 | 8.5 - 13.2 mL/g | 1 - 10 min |
| wash | 3-6 | 8.8 - 44 mL/g | 0.5 - 2 min |
| activator wash | 1 | 8.6-13.1 mL/g | 2 - 3 min |
| coupling | 1 | 10.3-18.2 mL/g | 3 - 9 min |
| wash | 3-6 | 8.8 - 44 mL/g | 0.5 - 2 min |
| oxidation | 1 | 17.7 - 28.3 mL/g | 0.5 - 2 min |
| wash | 3-6 | 8.8 - 44 mL/g | 0.5 - 2 min |
| thiolation | 1 | 23.8 mL/g | 2 min |
| wash | 3 | 44 mL/g | 0.5 min |
| capping | 1 | 3.4-7.9 mL/g | 2 min |
| wash | 3-6 | 8.8 - 44 mL/g | 0.5 - 2 min |

The detritylation was typically either performed with a detritylation cocktail as described in GP B, using 10% (v/v) DCA in toluene of GP C, with 2% (v/v) DCA and 0.1% (v/v) ethanol in DCM as laid out in GP D, or with 10% DCA in toluene (v/v) of GP E.

Coupling was typically performed using phosphoramidite in solution prepared under GP A and adding the 5-(ethylthio)-1*H*-tetrazole activator (ETT, 0.5 M).

Oxidation was typically performed using iodine solution (50 mM) in a mixture of water and pyridine (1:9, v/v) solution. In examples, where 25 mL sparged bed reactors (R-3) were used, the oxidation volume per resin mass was 17.7 mL/g.

Thiolation (i.e. sulfurization) was typically performed using a xanthane hydride solution 0.2 M in pyridine or using xanthane hydride solution 0.1 M in a mixture of acetonitrile and pyridine (1:1, v/v). In examples, where 25 mL sparged bed reactors (R-3) were used, the thiolation volume per resin mass was 17.7 mL/g.

It should be noted that a typical synthesis cycle (i.e. coupling cycle) comprises either oxidation or thiolation. In both cases, the phosphorus (III) linkage group is converted to a phosphorus (V) linkage group.

Capping was typically performed with a mixture (1:1, v/v) of Cap A (20% acetic anhydride in acetonitrile, v/v) and Cap B (N-methyl imidazole, 2,6-lutidine, acetonitrile, 20:30:50, v/v/v). In examples, where 25 mL sparged bed reactors (R-3) were used, the capping volume per resin mass was 8.7 mL/g.

Wash steps were typically performed using acetonitrile. Only prior to detritylation, DCM (e.g. when using DCM as solvent of the detritylation cocktail) or toluene (e.g. when using toluene or anisole as solvent for the detritylation cocktail) were used for the wash steps. In examples, where 25 mL sparged bed reactors (R-3) were used, the DCM and toluene washing volumes prior to detritlyation were 14 mL/g, and the ACN washing volume per resin mass was 44 mL/g. The activator wash is only optionally performed. Unless indicated differently, an activator wash has been performed. This term refers to a wash step using a solution of acetonitrile and the activator as specified for the coupling step (i.e. 0.5 M ETT in acetonitrile).

Unless indicated differently, each step of a typical coupling cycle comprised contacting the solid support carrying the growing oligonucleotide chains with the respective reagent solution (e.g. a detritylation cocktail for detritylation steps or neat solvent for wash steps) for the indicated time under stirring (stirred bed reactor) or shaking (syringe reactor) or agitating by nitrogen bubbling (sparged bed reactor), followed by draining of the respective solution through a filter, so as to retain the support-bound growing oligonucleotides inside the reactor. A step of a typical coupling cycle, e.g. the detritylation step may be repeated one or more times. In such cases, the aforementioned procedure is simply repeated, i.e. the reagent solution is drained from the reactor, and the on-resin oligonucleotides are treated with another batch of the respective solution, followed by draining of the solution. In Examples 1 to 10, the number of repetitions of each detritylation step as well as the contact time with the detritylation cocktail per repetition is indicated specifically in tabular form. For example, two repetitions with 10 min contact time each would be referred to as 2 × 10 min or 10 + 10 min.

Unless indicated differently, syntheses were performed at 22°C.

The synthesis cycles were repeated according to the sequence using the appropriate nucleoside phosphoramidites.

The final 5'-DMT detritylation was performed using the same procedure that was used in the previous cycles including the wash steps. The resin was then dried under reduced pressure for a minimum of 16 h.

After completion of all coupling cycles and a final detritylation step, the resin was weighed, and a trial cleavage and deprotection of the sample was performed. For this purpose, typically, 10-50 mg dried resin was weighed into a suitable reaction vessel (e.g. a 2 mL PP-tube). Aqueous ammonium hydroxide solution (i.e. aqueous ammonia solution, 25-28% (w/v), 1-2 mL) was added. The tube was closed, sealed, shaken, and heated to 45-55°C for 2-20 h depending on length and sequence of the oligonucleotide. Optionally, treatment with diethylamine (DEA) in acetonitrile may be performed prior to treatment with the aqueous ammonia. The supernatant was typically removed using a syringe and transferred to a second tube (2-5 mL). The ammonia was typically removed using a vacuum centrifuge or with a slight stream of nitrogen gas. The solution (containing the support-cleaved oligonucleotides) was then diluted with H₂O to the desired concentration, followed by analysis via HPLC or HPLC-MS.

### 4. Depurination assays

### Assay method A:

The impact of elongated (i.e. prolonged) contact times on the depurination of A and G was assessed by comparing the respective detritylation cocktail (according to GP B) with a standard detritylation cocktail (10% (v/v) dichloroacetic acid (DCA) in toluene, GP C) using cleavage times of 2 × 10 min. As adenosine is the most prone to depurination, suitable sequences were chosen.

The syntheses and the results of the depurination assay using assay method A are listed as Example 5 below.

### Assay method B:

The detritylation protocol GP B was compared to prior art that describes a detritylation protocol specifically aiming at reducing or eliminating depurination (WO9603417A1). From this prior art, the detritylation mixture consisting of 2% (v/v) DCA and 0.1% (v/v) ethanol in DCM was chosen for comparison (GP D). The 5mer DNA 5'-AAA AT-3' was chosen as the target oligonucleotide.

Depurination side products were identified by HPLC-MS analysis of the support-cleaved oligonucleotides as products with masses corresponding to depurinated abasic sites and side products produced as the result of degradation reactions of depurinated abasic sites during standard cleavage and deprotection with 25-28% (w/v) aqueous ammonia (see for example: S. Pourshahian et al., Mass Spectrometry Reviews 2021, 40, 75-109). Because of the large number of different depurination products observed, the UV trace of the HPLC-MS measurements was integrated without applying a minimal relative area threshold. This avoids the exclusion of smaller peaks pertaining to depurination-related side products according to their respective masses.

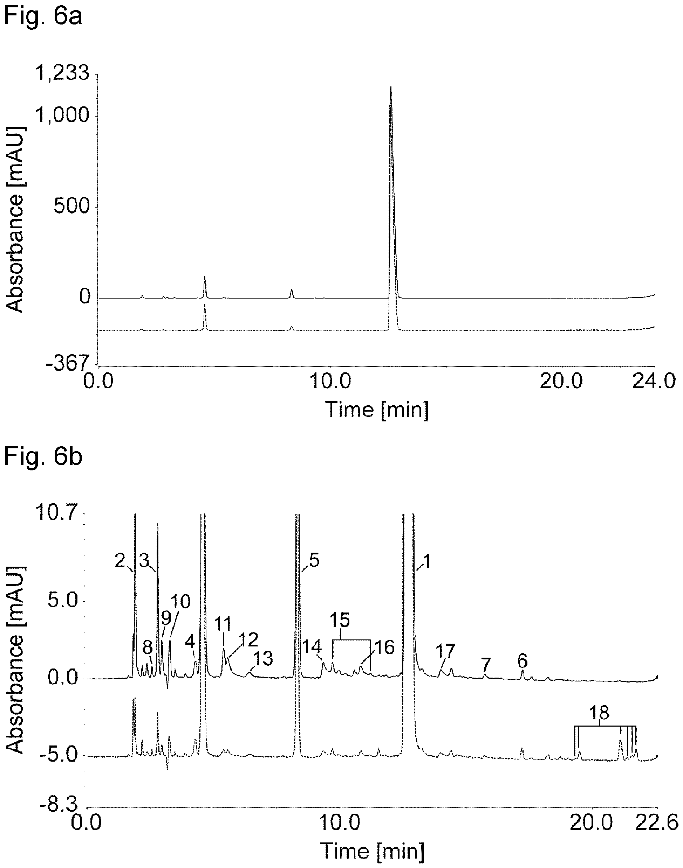

The syntheses and the results of the depurination assay using assay method B are listed as Example 7 below. Chromatograms of these analyses are shown as Figures 6a and 6b. In these chromatograms, peaks have been assigned a peak ID. In the following Table B.1, these peak IDs are assigned to an oligonucleotide or fragment based on mass spectrometry data (calculated and detected monoisotopic masses provided). This is followed by Table B.2 showing the assumed structures of these oligonucleotides or fragments. From the UV trace of the respective HPLC-MS measurements, the degree of depurination was determined by dividing the summed up peak areas of all identified depurination products by the peak area of the product (i.e. the target oligonucleotide, also referred to as full-length product), followed by multiplication with 100% to arrive at a value in percent (%).

**Table B.1: Peaks identified for the crude 5mer DNA 5'-AAA AT-3' obtained in the syntheses of Example 8 (vide infra) used to evaluate depurination assay B.**

| Peak ID | Type | Assigned oligonucleotide or fragment | Calculated mass (Da) | Found mass (Da) |
|---|---|---|---|---|
| 1 | Full-length product | B.1-1 | 1494.32 | 1494.32 |
| 2 | Shortmer | B.1-2 | 242.09 | 242.09 |
| 3 | Shortmer | B.1-3 | 555.15 | 555.15 |
| 4 | Shortmer | B.1-4 | 868.21 | 868.21 |
| 5 | Shortmer | B.1-5 | 1181.26 | 1181.26 |
| 6 | Longmer | B.1-6 | 1807.38 | 1807.38 |
| 7 | Other | B.1-7 | 1270.24 | 1270.24 |
| 8 | Standard depurination product | B.1-8 | 446.13 | 446.13 |
| 9 | Standard depurination product | B.1-9 | 322.06 | 322.06 |
| 10 | Standard depurination product | B.1-10 | 759.19 | 759.19 |
| 11 | Standard depurination product | B.1-11 | 1072.25 | 1072.25 |
| 12 | Standard depurination product | B.1-12 | 635.11 | 635.12 |
| 13 | Standard depurination product | B.1-13 | 644.13 | 644.13 |
| 14 | Standard depurination product | B.1-14 | 948.17 | 948.17 |
| 15 | Standard depurination product | B.1-15 | 1377.28 | 1377.24, 1377.28 |
| 16 | Standard depurination product | B.1-16 | 957.18 | 957.18 |
| 17 | Standard depurination product | B.1-17 | 1261.23 | 1261.23 |
| 18 | TFE-containing depurination product | B.1-18 | 1459.28 | 1459.28 |

**Table B.2: Assumed chemical structures of the assigned oligonucleotides and fragments listed in Table B.1.**

| Peak ID | Assigned oligonucleotide or fragment | Assumed chemical structure |
|---|---|---|
| 1 | B.1-1 | 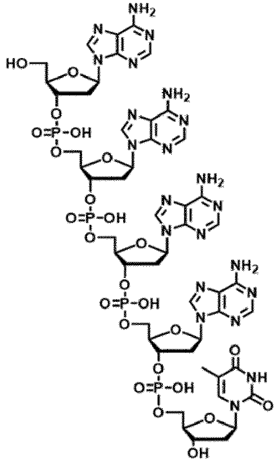 |
| 2 | B.1-2 | 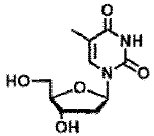 |
| 3 | B.1-3 | 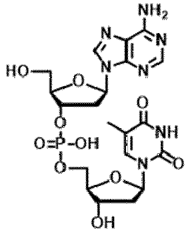 |
| 4 | B.1-4 | 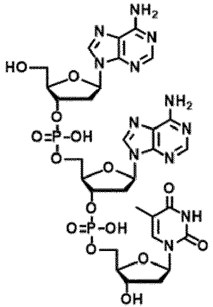 |
| 5 | B.1-5 | 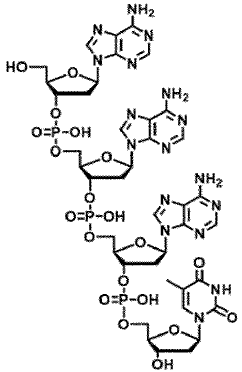 |
| 6 | B.1-6 | 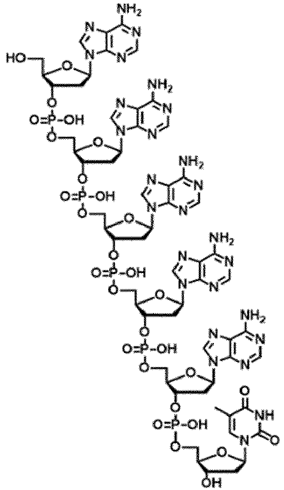 |
| 7 | B.1-7 | 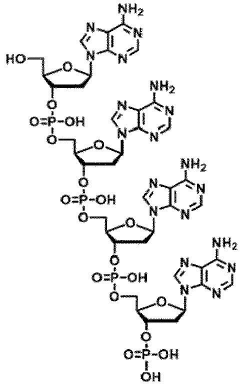 |
| 8 | B.1-8 | 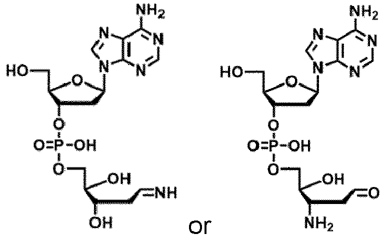 Both structures can form from abasic sites during ammonia treatment, and have the same mass. The peak is assigned to both structures. |
| 9 | B.1-9 | 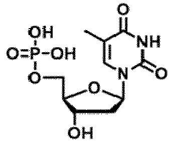 |
| 10 | B.1-10 | 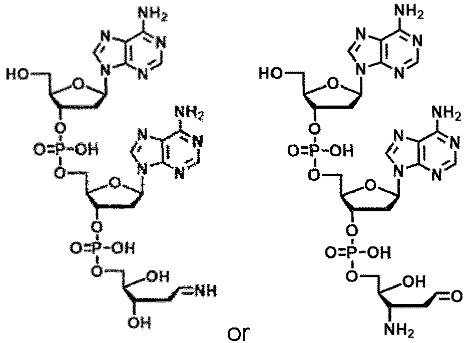 Both structures can form from abasic sites during ammonia treatment, and have the same mass. The peak is assigned to both structures. |
| 11 | B.1-11 | 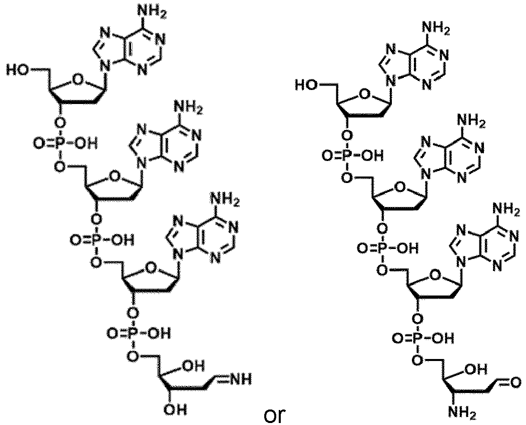 |
| | | Both structures can form from abasic sites during ammonia treatment, and have the same mass. The peak is assigned to both structures. |
| 12 | B.1-12 | 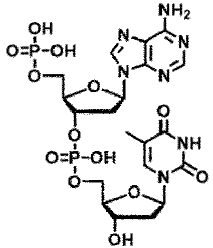 |
| 13 | B.1-13 | 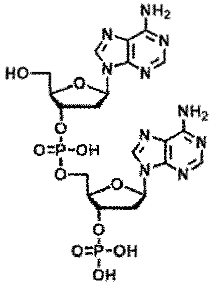 |
| 14 | B.1-14 | 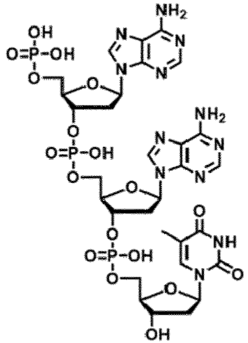 |
| 15 | B.1-15 | 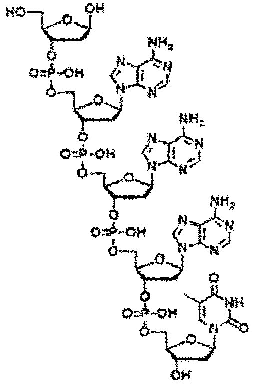 |
| | | Multiple peaks with this mass due to different isomers / positions of the abasic site (1'-OH) |
| 16 | B.1-16 | 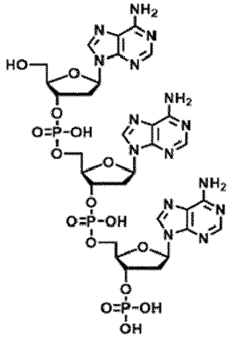 |
| 17 | B.1-17 | 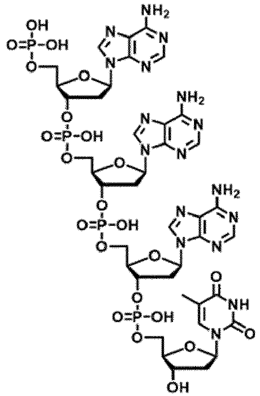 |
| 18 | B.1-18 | 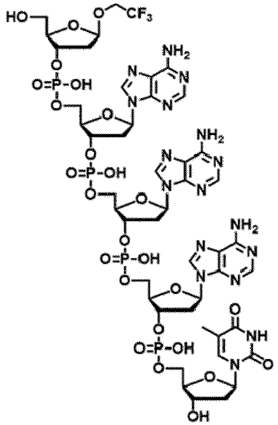 Multiple peaks with this mass due to different isomers / positions of the TFE-substituent |

### Assay method C:

The detritylation protocols GP B as well as GP E were applied to the synthesis of oligonculeotides with electron withdrawing substituents in 2'-position (e.g. 2'-O-methyl ribose, 2'-F deoxyribose).

The 23mer oligonucleotide
5'-[mAs][fCs][mA][fA][fA][fA][mG][fC][mA][fA][mA][mA][mC][fA][mG][fG][mU][fC][mU ][mA][mGs][mAs][mA]-3' with both 2'-F and 2'-OMe nucleosides and a mixed phosphodiester (PO) and phosphorothioate (PS) backbone was chosen as target oligonucleotide. This target oligonucleotide represents building blocks commonly found in oligonucleotide active pharmaceutical ingredients (APIs). The following notation was used: "m" indicates that the ribose moiety of the respective nucleoside-subunit carries a 2'-*O*-Me group; "f" indicates that the ribose moiety of the respective nucleoside-subunit carries a 2'-F (fluoro) substituent; "s" indicates that the internucleosidic linkage group in 3'-position of the respective nucleoside-subunit is a phosphorothioate group instead of a phosphodiester group.

To evaluate the detritylation performance, the overall purity was used. To specifically determine the degree of depurination, the amount of abasic sites missing the adenine nucleobase was quantified by means of HPLC-MS. Due to the stability of abasic sites in 2'-F / 2'-OMe oligonucleotides during ammonia treatment, the abasic site can be used to quantify depurination directly. As the abasic side product co-elutes with other side products, the peak in question was integrated in the UV trace (HPLC-MS), and the relative amount of the different abasic side products were determined by integration of the mass trace after applying charge deconvolution in Bruker Data Analysis software using a maximum entropy algorithm.

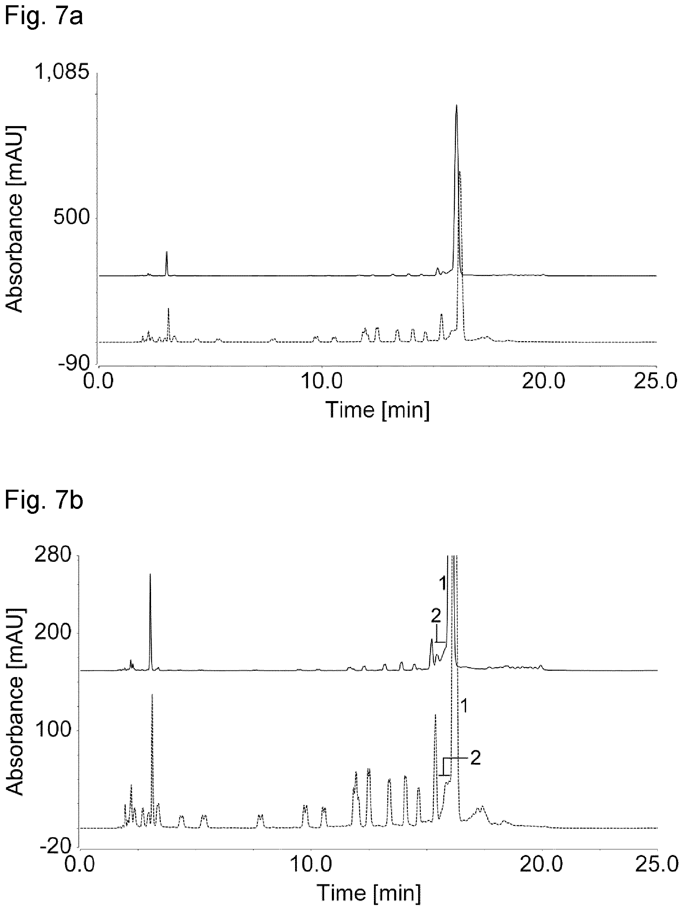

The syntheses and the results of the depurination assay using assay method C are listed as Example 8 below. Chromatograms of these analyses are shown as Figures 7a and 7b. In these chromatograms, peaks have been assigned a peak ID. In the following Table C.1, these peak IDs are assigned to an oligonucleotide or fragment based on mass spectrometry data (calculated and detected monoisotopic masses provided). This is followed by Table C.2 showing the assumed structures of these oligonucleotides or fragments.

To determine the degree of depurination, the relative content of C.1-2 in the mass trace was determined for peaks containing C.1-2, and multiplied with the relative area of the respective peaks in the UV trace. The degree of depurination was obtained by dividing the so-obtained corrected area of C.1-2 in the UV-trace by the area of the product (i.e. the target oligonucleotide or full-length product) in the UV-trace, followed by multiplication with 100% to arrive at a value in percent (%).

**Table C.1: Peaks identified for the 23mer mixed 2'-F / 2'-OMe oligonucleotide to evaluate depurination assay C.**

| Peak ID | Type | Assigned oligonucleotide or fragment | Calc. mass (Da) | Found mass (Da) |
|---|---|---|---|---|
| 1 | Full-length product (SEQ ID NO 3) | C.1-1 | 7706.22 | 7706.23 |
| 2 | Abasic site (-A) | C.1-2 | 7589.18 | 7589.19 |

**Table C.2: Assumed chemical structures of the assigned oligonucleotides and fragments listed in Table C.1.**

| Peak ID | Assigned oligonucleotide | Assumed chemical structure |
|---|---|---|
| 1 | C.1-1 | 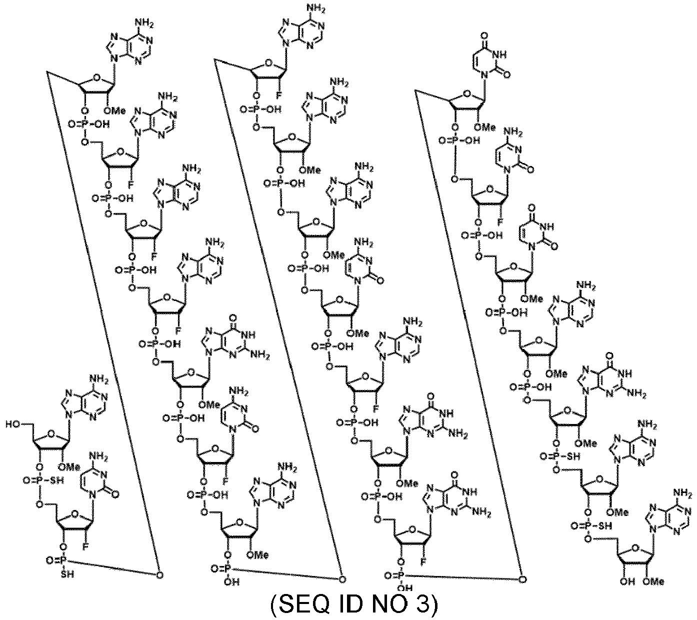 |
| 2 | C.1-2 | SEQ ID NO 3, in which a single adenine nucleobase is replaced by an -OH substituent (i.e. a single abasic site). |
| | | Peak C.1-2 is thus assigned to multiple structures, all of which have one such abasic site instead of adenine. |

### Example 1: comparative example - detritylation with 10% DCA in toluene (v/v).

**Table E-1: comparative example - Detritylation with 10% DCA in toluene (v/v).**

| EOP | sequence^{a}) | detrit. cocktail^{b)} | detrit. time [min] | reactor type | purity | yield |
|---|---|---|---|---|---|---|
| 4148503/34^{c}) | 5'-CTA TA-3' | 10% DCA in toluene | 1 × 1 | syringe (R-1) | 92% | 82% |
| 4148503/70^{c}) | 5'-CTA TA-3' | 10% DCA in toluene | 2 × 10 | syringe (R-1) | 87% | 95% |
| 4149467/01^{c}) | 5'-ATA CCG ATT AAG CGA AGT TT-3' (SEQ ID NO: 2) | 10% DCA in toluene | 1 × 1 | syringe (R-1) | 68% | 82% |
| 4149467/21^{c)} | 5'-ATA CCG ATT AAG CGA AGT TT-3' (SEQ ID NO: 2) | 10% DCA in toluene | 2 × 10 | syringe (R-1) | 56% | 85% |
| 4148503/37^{d)} | 5'-CTA TA-3' | 10% DCA in toluene | 1 × 1 | 1 L reactor (R-2) | 90% | 99% |
| 4148503/73^{d)} | 5'-CTA TA-3' | 10% DCA in toluene | 2 × 10 | 1 L reactor (R-2) | 81% | 86% |

| | | | | | | |
|---|---|---|---|---|---|---|
| a) Syntheses were carried out on nucleoside-loaded polystyrene support; b) Detritylation volume was 2.5 mL 100 µmol starting material (i.e. 100 µmol of DMT-protected OH-groups on the starting resin) per detritylation repetition; c) Synthesis was conducted in 0.04 mmol scale; d) Synthesis was conducted in 2.5 mmol scale. | | | | | | |

Syntheses EOP4148503/34, 37, 70, 73 and 4149467/01, 21 were conducted according to GP C.

Two different model sequences were synthesized using the standard detritylation cocktail of the prior art. Two or four syntheses were carried out for each model sequence. GP C was used for detritylation, with a deprotection time of 1 × 1 min or 2 × 10 min. The protocols of both syntheses differed only with respect to the deprotection time, all other synthesis parameters were kept constant. The comparison demonstrates that the standard detritylation cocktail used in the prior art (10% DCA in toluene, v/v) is disadvantageous, if longer contact times between detritylation cocktail and oligonucleotide occur.

### Example 2: Detritylation with composition according to the present invention

**Table E.2: Detritylation with composition according to the present invention**

| EOP | sequence^{a}) | detrit. cocktail^{b)} | detrit. time [min] | reactor type | purity | yield |
|---|---|---|---|---|---|---|
| 4148503/35^{c)} | 5'-CTA TA-3' | 1:0.7:1.4:19:79, 4-cyanopyridine:TFA:EDT: TFE:DCM, m:v:v:v:v | 2 × 10 | syringe (R-1) | 94% | 88% |
| 4148503/48^{c)} | 5'-CTA TA-3' | 1:0.7:1.4:19:79, 4-cyanopyridine:TFA:EDT: TFE:toluene, m:v:v:v:v | 2 × 10 | syringe (R-1) | 93% | 89% |
| 4149467/02^{c)} | 5'-ATA CCG ATT AAG CGA AGT TT-3' (SEQ ID NO: 2) | 1:0.7:1.4:19:79, 4-cyanopyridine:TFA:EDT: TFE:DCM, m:v:v:v:v | 2 × 10 | syringe (R-1) | 79% | 85% |
| 4148503/41^{d)} | 5'-CTA TA-3' | 1:0.7:1.4:19:79, 4-cyanopyridine:TFA:EDT: TFE:DCM, m:v:v:v:v | 2 × 10 | 1 L reactor (R-2) | 91% | 95% |

| | | | | | | |
|---|---|---|---|---|---|---|
| a) Syntheses were carried out on nucleoside-loaded polystyrene support; b) Detritylation volume was 2.5 mL 100 µmol starting material (i.e. 100 µmol of DMT-protected OH-groups on the starting resin) per detritylation repetition; c) Synthesis was conducted in 0.04 mmol scale; d) Synthesis was conducted in 2.5 mmol scale. | | | | | | |

Syntheses EOP 4148503/35, 41, 48 and 4149467/02 were carried out as described above using GP B.

For EOP4148503/35 and 4149467/02, the detritylation cocktail was prepared by dissolving TFE (19 mL), EDT (1.4 mL) 4-cyanopyridine (1.0 g, 96 mM), TFA (0.7 mL, 92 mM) in DCM (79 mL).

For EOP 4148503/48, the detritylation cocktail was prepared by dissolving TFE (19 mL), EDT (1.4 mL) 4-cyanopyridine (1.0 g, 96 mM), TFA (0.7 mL, 92 mM) in toluene (79 mL).

For EOP4148503/41, the detritylation cocktail was prepared by dissolving TFE (0.23 L), EDT (17 mL) 4-cyanopyridine (12 g, 96 mM), TFA (8.4 mL, 92 mM) in DCM (0.95 L).

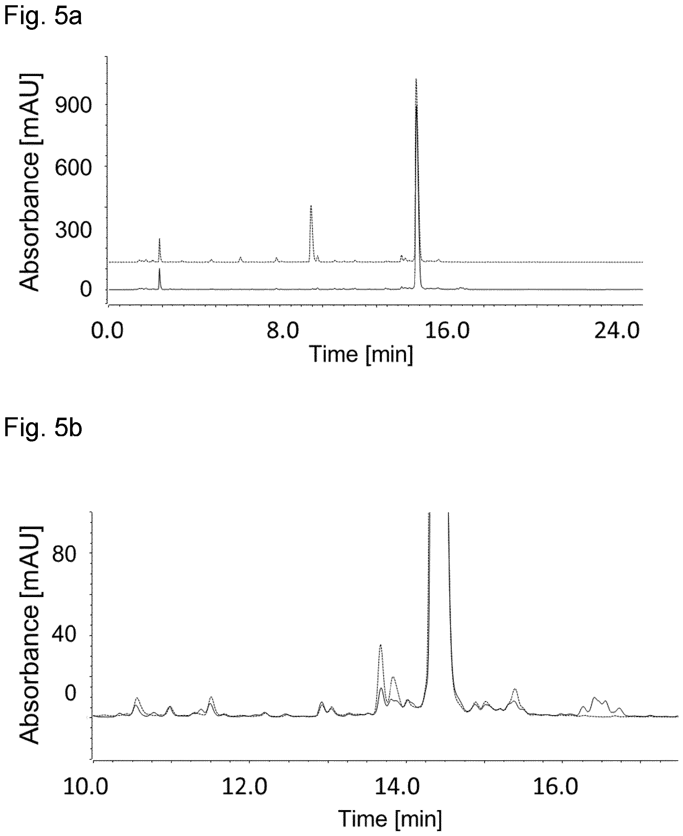

Two different model sequences were synthesized using a detritylation protocol according to the present invention. Apart from the fact that the detritylation protocol GP C was replaced by GP B with 2 × 10 min detritylation time, the synthesis parameters for each sequence were identical with Example 1. Comparison with the results of Example 1 (compare EOP 4148503/48 vs. EOP 4148503/34; EOP 4149467/02 vs. EOP 4149467/01; EOP 4148503/41 vs. EOP 4148503/37) demonstrates that the liquid composition C according to the present invention provides an improved weight gain and purity compared to the standard detritylation method of the prior art, i.e. 10% (v/v) DCA in toluene with a deprotection time of 1 × 1 min. This effect is particularly pronounced for the more demanding 20mer sequence (EOP 4149467/02 vs. EOP 4149467/01): The sample synthesized according to the invention exhibits an overall cleaner impurity profile; the overall purity increased by 11%. The corresponding analytical chromatograms are given in Fig. 5. These observations suggest that the deprotection method and composition according to the present invention enables improved removal of the protecting group while suppressing impurity formation. The disadvantages of the prior art protocol as compared to the protocol of the invention are even more evident if longer contact times cannot be avoided (compare EOP 4148503/48 vs. EOP 4148503/70; EOP 4149467/02 vs. EOP 4149467/21; EOP 4148503/41 vs. EOP 4148503/73).

In EOP 4149467/02, the volume of detritylation solution was 2.5 mL / 100 µmol of the starting material (i.e. 100 µmol of DMT-protected OH-groups on the starting resin) per repetition. For the synthesis scale of 40 µmol, the detritylation volume was 1.0 mL. This contains 92 µmol acid per repetition, which represents 2.3 equivalents relative to the DMT groups, and 0.12 equivalents relative to the nucleobases during the final detritylation step. The detritylation cocktail contains 2.6 mmol TFE per repetition, which equates to 66 equivalents TFE to nucleobases in the first cycle, and 3.3 equivalents TFE to nucleobases when the 5mer oligonucleotide is completed.

### Example 3: Detritylation with composition according to the present invention - use of various carbocation scavengers

**Table E.3: Detritylation with composition according to the present invention - use of various carbocation scavengers**

| EOP | sequence^{a}) | detrit. cocktail^{b)} | detrit. time [min] | reactor type | purity | yield |
|---|---|---|---|---|---|---|
| 4148503/53^{c)} | 5'-CTA TA-3' | 1:0.7:2.7:19:77.6, 4-cyanopyridine:TFA:DODT: TFE:DCM, m:v:v:v:v | 2 × 10 | Syringe (R-1) | 93% | 91% |
| 4148503/50^{c)} | 5'-CTA TA-3' | 1:0.7:2.3:19:78, 4-cyanopyridine:TFA:DTE: TFE:DCM, m:v:m:v:v | 2 × 10 | Syringe (R-1) | 93% | 91% |
| 4148503/52^{c)} | 5'-CTA TA-3' | 1:0.7:2.5:19:78, 4-cyanopyridine:TFA:BDMT: TFE:DCM, m:v:m:v:v | 2 × 10 | Syringe (R-1) | 93% | 89% |
| 4148503/55^{c)} | 5'-CTA TA-3' | 1:0.7:1.9:19:78, 4-cyanopyridine:TFA:TES: TFE:DCM, m:v:v:v:v | 2 × 10 | Syringe (R-1) | 93% | 99% |
| 4148503/54^{c)} | 5'-CTA TA-3' | 1:0.7:1:19:79, 4-cyanopyridine:TFA:H₂O: TFE:DCM, m:v:v:v:v | 2 × 10 | Syringe (R-1) | 93% | 91% |
| 4149467/05^{c)} | 5'-ATA CCG ATT AAG CGA AGT TT-3' (SEQ ID NO: 2) | 1:0.7:1:19:79, 4-cyanopyridine:TFA:H₂O: TFE:toluene, m:v:v:v:v | 2 × 10 | Syringe (R-1) | 76% | 92% |
| 4148503/51^{c)} | 5'-CTA TA-3' | 1:0.7:19:80, 4-cyanopyridine:TFA:TFE: DCM, m:v:v:v | 2 × 10 | Syringe (R-1) | 94% | 91% |
| 4149467/06^{c)} | 5'-ATA CCG ATT AAG CGA AGT TT-3' (SEQ ID NO: 2) | 1:0.7:19:80, 4-cyanopyridine:TFA:TFE: toluene, m:v:v:v | 2 × 10 | Syringe (R-1) | 69% | 89% |

| | | | | | | |
|---|---|---|---|---|---|---|
| a) Syntheses were carried out on nucleoside-loaded polystyrene support; b) Detritylation volume was 2.5 mL 100 µmol starting material (i.e. 100 µmol of DMT-protected OH-groups on the starting resin) per detritylation repetition; c) Synthesis was conducted in 0.04 mmol scale | | | | | | |

The syntheses were carried out as described above using GP B.

For EOP4148503/53, the detritylation cocktail was prepared by dissolving TFE (19 mL), DODT (2.7 mL), 4-cyanopyridine (1.0 g, 96 mM), TFA (0.7 mL, 92 mM) in DCM (77.6 mL).

For EOP4148503/50, the detritylation cocktail was prepared by dissolving TFE (19 mL), DTE (2.3 g), 4-cyanopyridine (1.0 g, 96 mM), TFA (0.70% (v/v), 0.7 mL, 92 mM) in DCM (78 mL).

For EOP4148503/52, the detritylation cocktail was prepared by dissolving TFE (19 mL), BDMT (2.5 g), 4-cyanopyridine (1.0 g, 96 mM), TFA (0.7 mL, 92 mM) in DCM (78 mL).

For EOP4148503/55, the detritylation cocktail was prepared by dissolving TFE (19 mL), TES (1.9 mL) 4-cyanopyridine (1.0 g, 96 mM), TFA (0.7 mL, 92 mM) in DCM (78 mL).

For EOP4148503/54, the detritylation cocktail was prepared by dissolving TFE (19 mL), water (1.0 mL), 4-cyanopyridine (1.0 g, 96 mM), TFA (0.7 mL, 92 mM) in DCM (79 mL).

For EOP4149467/07, the detritylation cocktail was prepared by dissolving TFE (19 mL), water (1.0 mL), 4-cyanopyridine (1.0 g, 96 mM), TFA (0.7 mL, 92 mM) in toluene (79 mL).

For EOP4148503/51, the detritylation cocktail was prepared by dissolving TFE (19 mL), 4-cyanopyridine (1.0 g, 96 mM), TFA (0.7 mL, 92 mM) in DCM (80 mL).

For EOP4149467/06, the detritylation cocktail was prepared by dissolving TFE (19 mL), 4-cyanopyridine (1.0 g, 96 mM), TFA (0.7 mL, 92 mM) in toluene (80 mL).

The examples indicate that various kinds of common carbocation scavengers such as silanes [e.g. triethylsilane (TES)], thiols or thiophenols [e.g. 3,6-dioxa-1,8-octanedithiol (DODT), 1,4-dithioerythrol (DTE), 1,4-benzenedimethanthiol (BDMT)], and water may for example be used.

### Example 4: Detritylation with composition according to the present invention - use of different fluorinated alcohols

**Table E.4: Detritylation with composition according to the present invention - use of different fluorinated alcohols**

| EOP | sequence^{a}) | detrit. cocktail^{b)} | detrit. time [min] | reactor type | purity | yield |
|---|---|---|---|---|---|---|
| 4148503/63^{c)} | 5'-CTA TA-3' | 1:0.7:1.4:5:93, 4-cyanopyridine:TFA:EDT: HFIP:toluene, m:v:v:v:v | 2 × 10 | syringe (R-1) | 93% | 99% |
| 4148503/66^{c)} | 5'-CTA TA-3' | 1:0.7:1.4:5:93, 4-cyanopyridine:TFA: EDT:TFE:toluene, m:v:v:v:v | 2 × 10 | syringe (R-1) | 93% | 87% |
| 4149467/67^{c)} | 5'-CTA TA-3' | 1:0.7:1.4:2:96, 4-cyanopyridine:TFA: EDT:HFIP:toluene, m:v:v:v:v | 2 × 10 | syringe (R-1) | 92% | 96% |

| | | | | | | |
|---|---|---|---|---|---|---|
| a) Syntheses were carried out on nucleoside-loaded polystyrene support; b) Detritylation volume was 2.5 mL / 100 µmol starting material (i.e. 100 µmol of DMT-protected OH-groups on the starting resin) per detritylation repetition; c) Synthesis was conducted in 0.04 mmol scale | | | | | | |

The syntheses were carried out as described above using GP B for detritylation. For EOP4148503/63, the detritylation cocktail was prepared by dissolving HFIP (5 mL), EDT (1.4 mL) 4-cyanopyridine (1.0 g, 96 mM), TFA (0.7 mL, 92 mM) in toluene (93 mL).

For EOP4148503/66, the detritylation cocktail was prepared by dissolving TFE (5 mL), EDT (1.4 mL) 4-cyanopyridine (1.0 g, 96 mM), TFA (0.7 mL, 92 mM) in toluene (93 mL).

For EOP4148503/67, the detritylation cocktail was prepared by dissolving HFIP (2 mL), EDT (1.4 mL) 4-cyanopyridine (1.0 g, 96 mM), TFA (0.7 mL, 92 mM) in toluene (96 mL).

The examples indicate that the concentration of the fluorinated alcohol in the detritylation cocktail may be varied and that fluorinated alcohols other than TFE may be used.

### Example 5: Effect on depurination (Depurination assay, assay method A)

**Table E.5: Effect on depurination (Depurination assay, assay method A)**

| EOP | sequence^{a}) | detrit. cocktail^{b)} | detrit. time [min] | reactor type | purity | yield |
|---|---|---|---|---|---|---|
| 4149994/01^{c)} | 5'-TTT TAT TTT T-3' (SEQ ID NO: 1) | 1:0.7:2.7:19:77.6, 4-cyanopyridine: TFA: DODT: TFE:toluene, m:v:v:v:v | 2 × 10 | Syringe (R-1) | 93% | 93% |
| 4149994/02^{d)} | 5'-TTT TAT TTT T-3' (SEQ ID NO: 1) | 10% DCA in toluene | 2 × 10 | Syringe (R-1) | 89% | 91% |
| 4149467/22^{d)} | 5'-ATA CCG ATT AAG CGA AGT TT-3' (SEQ ID NO: 2) | 10% DCA in toluene | 2 × 10 | Syringe (R-1) | 50% | 88% |
| 4149467/24^{c)} | 5'-ATA CCG ATT AAG CGA AGT TT-3' (SEQ ID NO: 2) | 1:0.7:2.7:19:77.6, 4-cyanopyridine: TFA:DODT:TFE:toluene, m:v:v:v:v | 3 × 10 | Syringe (R-1) | 64% | 95% |

| | | | | | | |
|---|---|---|---|---|---|---|
| a) Syntheses were carried out on nucleoside-loaded polystyrene support; b) Detritylation volume was 2.5 mL / 100 µmol starting material (i.e. 100 µmol of DMT-protected OH-groups on the starting resin) per detritylation repetition; c) according to the invention, conducted in 0.04 mmol scale; d) comparative example, conducted in 0.04 mmol scale | | | | | | |

GP B vs. GP C used for detritylation: 2 × 10 min for EOP4149994/01, EOP4149994/02, EOP4149467/22 and 3 × 10 min for EOP4149467/24, experiments were performed without capping as explained in the general protocol "Depurination assay, assay method A".

For EOP 4149994/01 and 4149467/24, the detritylation cocktail was prepared by dissolving TFE (19 mL), DODT (1.4 mL) 4-cyanopyridine (1.0 g, 96 mM), TFA (0.7 mL, 92 mM) in toluene (77.6 mL).

The benefit of the detritylation cocktail of the invention could be shown by comparison to standard conditions, i.e. 10% DCA in toluene (v/v), and detritylation times of 2 × 10 minutes. For the sequence 5'-TTT TAT TTT T-3' (SEQ ID NO: 1) an increase in purity of 4% could be observed (cf. Fig. 3). In case of the 10% DCA protocol, a species of 1537 Da [M-H]⁻ was detected by LC-MS. For the more challenging 20-mer DNA 5'-ATA CCG ATT AAG CGA AGT TT-3' (SEQ ID NO: 2), an increase in purity of 14% was observed (cf. Fig. 4)

### Example 6: Synthesis of oligonculeotides with electron withdrawing substituents in 2'-position (e.g. RNA synthesis, GP B used for detritylation)

**Table E.6: Synthesis of oligonculeotides with electron withdrawing substituents in 2'-position (e.g. RNA synthesis, GP B used for detritylation: 2 × 10 min deprotection)**

| EOP | Sequence^{a)} | detrit. cocktail^{b)} | detrit. time [min] | reactor type | purity | yield |
|---|---|---|---|---|---|---|
| 4149469/01^{c)} | 5'-[mA][fC][mC] | 1:0.7:1.4:19:79, 4-cyanopyridine:TFA:EDT: TFE:DCM, m:v:v:v:v | 2 × 10 | syringe (R-1) | 83% | 93% |
| | [fU][mG][mU] | | | | | |
| | [fC][fA][mU][mG]-3' | | | | | |
| 4149468/01^{c)} | 5'-[mAs][fCs][mC] | 1:0.7:1.4:19:79, 4-cyanopyridine:TFA:EDT: TFE:DCM, m:v:v:v:v | 2 × 10 | syringe (R-1) | 80% | 76% |
| | [fU][mG][mU] | | | | | |
| | [fC][fA][mU][mG]-3' | | | | | |

| | | | | | | |
|---|---|---|---|---|---|---|
| a) 2'-F and 2'-OMe cytidine phosphoramidites were acetyl-protected; Syntheses were carried out on UnyLinker^{™}-loaded polystyrene support; b) Detritylation volume was 2.5 mL / 100 µmol starting material (i.e. 100 µmol of DMT-protected OH-groups on the starting resin) per detritylation repetition; c) according to the invention, conducted in 0.04 mmol scale | | | | | | |

Syntheses EOP 4149469/01 and EOP 4149468/01 were carried out as described above using GP B.

The detritylation cocktail was prepared by dissolving TFE (19 mL), EDT (1.4 mL), 4-cyanopyridine (1.0 g, 96 mM), TFA (0.7 mL, 92 mM) in DCM (79 mL).

Both syntheses show applicability of GP B to oligonucleotides with 2'-F and 2'-OMe substitution. EOP 4149469/01 furthermore demonstrates applicability to oligonucleotides with a mixed phosphodiester (PO) and phosphorothioate (PS) backbone.

### Example 7: Depurination assay (assay method B)

**Table E.7.1: Synthesis of 5'-AAA AT-3' using the detritylation compositions / methods of WO 96/03417 vs the present invention (comparative studies)**

| EOP^{a)} | detrit. cocktail^{b)} | detrit. time | reactor type | purity | yield |
|---|---|---|---|---|---|
| 4153156/03-1^{c)} | 2.0:0.1:97.9, DCA:ethanol:DCM, v:v:v | 310 s | sparged bed (R-3) | 35% | 69% |
| 4153156/03-2^{c)} | 2.0:0.1:97.9, DCA:ethanol:DCM, v:v:v | 10 + 10 min | sparged bed (R-3) | 92% | 83% |
| 4153156/03-3^{c)} | 2.0:0.1:97.9, DCA:ethanol:DCM, v:v:v | 5 + 5 min | sparged bed (R-3) | 90% | 84% |
| 4153156/03-4^{c)} | 2.0:0.1:97.9, DCA:ethanol:DCM, v:v:v | 5 + 10 min | sparged bed (R-3) | 91% | 82% |
| 4153156/03-5^{c)} | 2.0:0.1:97.9, DCA:ethanol:DCM, v:v:v | 5 + 20 min | sparged bed (R-3) | 91% | 83% |
| 4153156/04-1^{d)} | 1:0.7:19:80, 4-cyanopyridine:TFA: TFE:DCM, m:v:v:v | 310 s | sparged bed (R-3) | 51% | 75% |
| 4153156/04-2^{d)} | 1:0.7:19:80, 4-cyanopyridine:TFA: TFE:DCM, m:v:v:v | 10 + 10 min | sparged bed (R-3) | 94% | 88% |
| 4153156/04-3^{d)} | 1:0.7:19:80, 4-cyanopyridine:TFA: TFE:DCM, m:v:v:v | 5 + 5 min | sparged bed (R-3) | 93% | 86% |
| 4153156/04-4^{d)} | 1:0.7:19:80, 4-cyanopyridine:TFA: TFE:DCM, m:v:v:v | 5 + 10 min | sparged bed (R-3) | 93% | 89% |
| 4153156/04-5^{d)} | 1:0.7:19:80, 4-cyanopyridine:TFA: TFE:DCM, m:v:v:v | 5 + 20 min | sparged bed (R-3) | 94% | 92% |
| 4153156/20-1^{e)} | 1:0.7:19:80. 4-cyanopyridine:TFA:ethanol:toluene , m:v:v:v | 310 s | sparged bed (R-3) | 2% | n.d.^{f)} |
| 4153156/20-2^{e)} | 1:0.7:19:80. 4-cyanopyridine:TFA:ethanol:toluene , m:v:v:v | 10 + 10 min | sparged bed (R-3) | 1% | n.d.^{f)} |
| 4153156/20-3^{e)} | 1:0.7:19:80. 4-cyanopyridine:TFA:ethanol:toluene , m:v:v:v | 5 + 5 min | sparged bed (R-3) | 0% | n.d.^{f)} |
| 4153156/20-4^{e)} | 1:0.7:19:80. 4-cyanopyridine:TFA:ethanol:toluene , m:v:v:v | 5 + 10 min | sparged bed (R-3) | 0% | n.d.^{f)} |
| 4153156/20-5^{e)} | 1:0.7:19:80. 4-cyanopyridine:TFA:ethanol:toluene , m:v:v:v | 5 + 20 min | sparged bed (R-3) | 2% | n.d.^{f)} |

| | | | | | |
|---|---|---|---|---|---|
| a) Activator wash before coupling was omitted; Syntheses were carried out on nucleoside-loaded polystyrene support; b) The volume of detritylation solution was 3.75 mL / 100 µmol of the starting material (i.e. 100 µmol of DMT-protected OH-groups on the starting resin) per detritylation repetition; c) according to WO96/03417; d) according to the invention; e) comparative examples (ethanol instead of TFE); f) no weight gain was determinable, indicating that the yield was close to 0%. | | | | | |

The syntheses in Example 7 (as compiled in Table E.7.1) were performed on a 0.10 mmol scale as laid out in section "3. Synthesis of oligonucleotides", in particular Table 1, using either GP B, GP D, or a protocol similar to GP B in which the fluorinated alcohol was replaced by ethanol.

Syntheses EOP 4153156/03-1 to -5 were conducted according to GP D with different detritylation times as a reference to prior art that describes detritylation with reduced depurination (WO96/03417). The detritylation mixture was prepared by dissolving DCA (9.0 mL, 0.24 M), ethanol (0.45 mL, 17 mM) in DCM (441 mL). The volume of the detritylation solution was 3.75 mL / 100 µmol of starting material (i.e. 100 µmol of DMT-protected OH-groups on the starting resin) per repetition. This volume contains 0.90 mmol acid per repetition, which represents 9.0 equivalents relative to the DMT groups, and 1.8 equivalents relative to the nucleobases during the final detritylation step.

Syntheses EOP 4153156/04-1 to -5 were conducted according to GP B. The detritylation cocktail was prepared by dissolving TFE (85.5 mL), 4--cyanopyridine (4.50 g, 96 mM), TFA (3.2 mL, 92 mM) in DCM (361 mL). The volume of detritylation solution was 3.75 mL / 100 µmol of the starting material (i.e. 100 µmol of DMT-protected OH-groups on the starting resin) per repetition. This volume contains 0.35 mmol acid per repetition, which represents 3.50 equivalents relative to the DMT groups, and 0.70 equivalents relative to the nucleobases during the final detritylation step. DCM was chosen as aprotic solvent to ensure good comparability with the prior art and exclude solvent effects. General synthesis conditions and detritylation times were equivalent to the syntheses conducted with the detritylation cocktail described in EOP 4153156/03-1 to -5.

The shortest detritylation time of 310 s was chosen in light of the prior art (WO96/03417), where 310 s is the preferred detritylation time in a flow-through protocol. For the batch reactor setup, this detritylation time leads to incomplete detritylation and hence low purity (EOP 4153156/03-1, 35% purity and EOP 4153156/04-1, 51% purity, respectively). The detritylation cocktail of the present invention results in higher purity, presumably as the result of faster detritylation. Prolonged detritylation procedures with two detritylation steps (i.e. two repetitions) (10 + 10 min, 5 + 5 min, 5 + 10 min, 5 + 20 min) also all result in higher purities with the detritylation cocktail of the present invention.

To demonstrate that the improved purities achieved with the detritylation cocktail of the invention are not merely the result of a higher alcohol concentration, additional syntheses with 19% (v/v) ethanol were conducted (syntheses EOP 4153156/20-1 to -5). The detritylation cocktail was prepared by dissolving ethanol (86 mL), 4-cyanopyridine (4.5 g, 96 mM), TFA (3.2 mL, 92 mM) in toluene (0.36 L). The volume of detritylation solution was 3.75 mL / 100 µmol of the starting material (i.e. 100 µmol of DMT-protected OH-groups on the starting resin) per detritylation repetition. In all cases, almost no full-length product was observed as detritylation was significantly hindered by the presence of 19% (v/v) ethanol. Since DCM and toluene have been shown to be both suitable for detritylation reactions using GP B (see e.g. Examples 2, 4, and 5), the very poor results obtained when replacing TFE for ethanol in EOP 4153156/20-1 to -5 is mainly attributed to this change of the alcohol.

Next, the degree of depurination was determined by means of HPLC-MS (Figure 6.a, 6.b) for all syntheses listed in Table E.7.1 and the results are compiled in Table E.7.2. For this purpose, the peak areas of all identified depurination products (see Tables B.1 and B.2) were summed up and divided by the peak area of the product (i.e. the target oligonucleotide, also referred to as full-length product), followed by multiplication with 100% to arrive at a value in percent (%).

**Table E.7.2: Degree of depurination for the syntheses of Table E.7.1 by HPLC-MS.**

| EOP^{a)} | detrit. cocktail^{b)} | detrit. time | Degree of depurination |
|---|---|---|---|
| 4153156/ 03-2^{c)} | 2.0:0.1:97.9, DCA:ethanol:DCM, v:v:v | 10 + 10 min | 0.59% |
| 4153156/ 03-3^{c)} | 2.0:0.1 :97.9, DCA:ethanol:DCM, v:v:v | 5 + 5 min | 0.57% |
| 4153156/ 03-4^{c)} | 2.0:0.1 :97.9, DCA:ethanol:DCM, v:v:v | 5 + 10 min | 0.65% |
| 4153156/ 03-5^{c)} | 2.0:0.1 :97.9, DCA:ethanol:DCM, v:v:v | 5 + 20 min | 0.69% |
| 4153156/ 04-2^{d)} | 1:0.7:19:80, 4-cyanopyridine: TFA:TFE:DCM, m:v:v:v | 10 + 10 min | 0.47% |
| 4153156/ 04-3^{d)} | 1:0.7:19:80, 4-cyanopyridine: TFA:TFE:DCM, m:v:v:v | 5 + 5 min | 0.42% |
| 4153156/ 04-4^{d)} | 1:0.7:19:80, 4-cyanopyridine: TFA:TFE:DCM, m:v:v:v | 5 + 10 min | 0.42% |
| 4153156/ 04-5^{d)} | 1:0.7:19:80, 4-cyanopyridine: TFA:TFE:DCM, m:v:v:v | 5 + 20 min | 0.43% |

| | | | |
|---|---|---|---|
| a) Activator wash before coupling was omitted; Syntheses were carried out on nucleoside-loaded support; b) The volume of detritylation solution was 3.75 mL / 100 µmol of the starting material (i.e. 100 µmol of DMT-protected OH-groups on the starting resin) per repetition; c) according to WO96/03417; d) according to the invention | | | |

For all detritylation times, the composition / method of the invention afforded a lower degree of depurination than the composition / method of WO96/03417. Chromatograms of EOP4153156/03-5 and EOP4153156/04-5 are provided in Figure 6.

### Example 8: Synthesis of oligonucleotides with electron withdrawing substituents in 2'-position (Depurination assay, assay method C)

**Table E.8: Syntheses of a 23mer mixed 2'-F / 2'-OMe oligonucleotide (SEQ ID NO 3) using GP B or GP E for detritylation.**

| EOP^{a)} | detrit. Cocktail^{b)} | detrit. time [min] | reactor type | purity | yield | degree of depurination |
|---|---|---|---|---|---|---|
| 4150430/28-1^{c)} | 1:0.7:0.5:19:80, 4-cyanopyridine: TFA:TFE:toluene, m:v:v:v | Cycle 1 (unylinker): | sparged bed (R-3) | 78% | 82% | 0.62% |
| | | 30 + 30 + 60 min | | | | |
| | | Cycles 2-10: | | | | |
| | | 5 + 20 min | | | | |
| | | Cycles 12-20: | | | | |
| | | 5 + 5 + 20 min | | | | |
| | | Cycles 21-23: | | | | |
| | | 5 + 5 + 5 + 20 min | | | | |
| 4150430/36-1^{d)} | 10:90, DCA:toluene, v:v | Cycle 1 (unylinker): | sparged bed (R-3) | 50% | 69% | 0.87% |
| | | 5 + 5 + 20 min | | | | |
| | | Cycles 2-10: | | | | |
| | | 5 + 20 min | | | | |
| | | Cycles 12-20: | | | | |
| | | 5 + 5 + 20 min | | | | |
| | | Cycles 21-23: | | | | |
| | | 5 + 5 + 5 + 20 min | | | | |

| | | | | | | |
|---|---|---|---|---|---|---|
| a) 2'-F and 2'-OMe cytidine phosphoramidites were acetyl-protected; Syntheses were carried out on UnyLinker^{™}-loaded polystyrene support; b) The volume of detritylation solution was 3.75 mL / 100 µmol of the starting material (i.e. 100 µmol of DMT-protected OH-groups on the starting resin) per detritylation repetition; c) according to the invention; d) comparative example | | | | | | |

Synthesis EOP4150430/28-1 was conducted in 0.1 mmol scale using GP B for detritylation. The detritylation cocktail was prepared by dissolving TFE (0.46 L), 4--cyanopyridine (24 g, 96 mM), TFA (17 mL, 92 mM) in toluene (1.9 L).

Synthesis EOP4150430-36-1 was conducted in 0.1 mmol scale using GP E for detritylation. The detritylation cocktail was prepared by dissolving DCA (0.30 L) in toluene (2.7 L).

As can be concluded from Table E.8, the purity obtained in the synthesis using GP B for detritylation (EOP4150430/28-1) is higher and the degree of depurination lower than with the common detritylation solution 10% DCA in toluene (v/v) of GP E (EOP4150430/36-1). With 10% DCA in toluene (v/v), detritylation is incomplete in some steps, leading to a large amount of side products that elute shortly before the target product (Figure 7a, 7b).

### Example 9: Detritylation with composition according to the present invention - use of different non-halogenated aprotic solvents

**Table E.9: Detritylation with composition according to the present invention - use of different non-halogenated aprotic solvents (sequence: 5'-AAA AT-3')**

| EOP^{a)} | detrit. cocktail^{b)} | detrit. time | reactor type | purity | yield |
|---|---|---|---|---|---|
| 4153156/05-1 | 1:0.7:19:80, 4-cyanopyridine:TFA: | 310 s | sparged bed (R-3) | 85% | 84% |
| | TFE :toluene, m:v:v:v | | | | |
| 4153156/12-2 | 1:0.7:19:80, 4-cyanopyridine:TFA: | 10 + 10 min | sparged bed (R-3) | 92% | 79% |
| | TFE :toluene, m:v:v:v | | | | |
| 4153156/05-3 | 1:0.7:19:80, 4-cyanopyridine:TFA: | 5 + 5 min | sparged bed (R-3) | 93% | 76% |
| | TFE :toluene, m:v:v:v | | | | |
| 4153156/05-4 | 1:0.7:19:80, 4-cyanopyridine:TFA: | 5 + 10 min | sparged bed (R-3) | 91% | 83% |
| | TFE :toluene, m:v:v:v | | | | |
| 4153156/12-5 | 1:0.7:19:80, 4-cyanopyridine:TFA: | 5 + 20 min | sparged bed (R-3) | 92% | 83% |
| | TFE :toluene, m:v:v:v | | | | |
| 4153156/15-1 | 1 :0.7:19:80, 4-cyanopyridine:TFA: | 310 s | sparged bed (R-3) | 85% | 75% |
| | TFE :anisole, m:v:v:v | | | | |
| 4153156/15-2 | 1:0.7:19:80, 4-cyanopyridine:TFA: | 10 + 10 min | sparged bed (R-3) | 93% | 79% |
| | TFE :anisole, m:v:v:v | | | | |
| 4153156/15-3 | 1:0.7:19:80, 4-cyanopyridine:TFA: | 5 + 5 min | sparged bed (R-3) | 94% | 79% |
| | TFE :anisole, m:v:v:v | | | | |
| 4153156/15-4 | 1:0.7:19:80, 4-cyanopyridine:TFA: | 5 + 10 min | sparged bed (R-3) | 94% | 77% |
| | TFE :anisole, m:v:v:v | | | | |
| 4153156/15-5 | 1:0.7:19:80, 4-cyanopyridine:TFA: | 5 + 20 min | sparged bed (R-3) | 93% | 76% |
| | TFE :anisole, m:v:v:v | | | | |

| | | | | | |
|---|---|---|---|---|---|
| a) Activator wash before coupling was omitted; Syntheses were carried out on nucleoside-loaded polystyrene support; b) The volume of detritylation solution was 3.75 mL / 100 µmol of the starting material (i.e. 100 µmol of DMT-protected OH-groups on the starting resin) per detritylation repetition | | | | | |

Syntheses EOP 4153156/05-1,3,4 and 4153156/12-2,5 as well as EOP 4153156/15-1 to -5 were conducted under the same conditions as EOP 4153156/04-1 to -5 of Example 7 (see Table E.7.1), except for replacing DCM in the detritylation cocktail with either toluene or anisole. The volume of detritylation solution was 3.75 mL / 100 µmol of the starting material (i.e. 100 µmol of DMT-protected OH-groups on the starting resin) per detritylation repetition. This volume contains 0.35 mmol acid per repetition, which represents 3.50 equivalents relative to the DMT groups, and 0.70 equivalents relative to the nucleobases during the final detritylation step. The detritylation cocktail contains 9.9 mmol TFE per repetition, which equates to 99 equivalents TFE to nucleobases in the first cycle, and 20 equivalents TFE to nucleobases when the 5mer oligonucleotide is completed.

The detritylation cocktail of syntheses EOP 4153156/05-1,3,4 and 4153156/12-2,5 was prepared by dissolving TFE (86 mL), 4-cyanopyridine (4.5 g, 96 mM), TFA (3.2 mL, 92 mM) in toluene (0.36 L). Likewise, the detritylation cocktail of EOP 4153156/15-1 to -5 was prepared by dissolving TFE (86 mL), 4-cyanopyridine (4.5 g, 96 mM), TFA (3.2 mL, 92 mM) in anisole (0.36 L).

The examples listed in Table E.9 show that the detritylation cocktails comprising toluene and the detritylation cocktails comprising anisole both result in successful synthesis of the 5mer DNA 5'-AAA AT3' following the detritylation procedure GP B. The use of non-halogenated solvents presents a large environmental benefit for industrial scale synthesis.

### Example 10: Detritylation with composition according to the present invention - use of different bases and strong acids

**Table E.10: Detritylation with composition according to the present invention - use of different bases and strong acids (sequence 5'-AAA AT-3')**

| EOP^{a)} | detrit. cocktail^{b)} | detrit. time | reactor type | purity | yield |
|---|---|---|---|---|---|
| 4153156/06-1 | 1.6:1.0:19:79, 3-chloropyridine:TFA: | 310 s | sparged bed (R-3) | 77% | 83% |
| | TFE :toluene, m:v:v:v | | | | |
| 4153156/06-2 | 1.6:1.0:19:79, 3-chloropyridine:TFA: | 10 + 10 min | sparged bed (R-3) | 92% | 87% |
| | TFE :toluene, m:v:v:v | | | | |
| 4153156/06-3 | 1.6:1.0:19:79, 3-chloropyridine:TFA: | 5 + 5 min | sparged bed (R-3) | 93% | 86% |
| | TFE :toluene, m:v:v:v | | | | |
| 4153156/06-4 | 1.6:1.0:19:79, 3-chloropyridine:TFA: | 5 + 10 min | sparged bed (R-3) | 93% | 76% |
| | TFE :toluene, m:v:v:v | | | | |
| 4153156/06-5 | 1.6:1.0:19:79, 3-chloropyridine:TFA: | 5 + 20 min | sparged bed (R-3) | 92% | 83% |
| | TFE :toluene, m:v:v:v | | | | |
| 4153156/11-1 | 1:0.7:19:80, 3-cyanopyridine:TFA: | 310 s | sparged bed (R-3) | 88% | 83% |
| | TFE :toluene, m:v:v:v | | | | |
| 4153156/11-2 | 1:0.7:19:80, 3-cyanopyridine:TFA: | 10 + 10 min | sparged bed (R-3) | 93% | 79% 84% |
| | TFE :toluene, m:v:v:v | | | | |
| 4153156/11-3 | 1:0.7:19:80, 3-cyanopyridine:TFA: | 5 + 5 min | sparged bed (R-3) | 93% | |
| | TFE :toluene, m:v:v:v | | | | |
| 4153156/11-4 | 1:0.7:19:80, 3-cyanopyridine:TFA: | 5 + 10 min | sparged bed (R-3) | 93% | 85% |
| | TFE :toluene, m:v:v:v | | | | |
| 4153156/11-5 | 1:0.7:19:80, 3-cyanopyridine:TFA: | 5 + 20 min | sparged bed (R-3) | 93% | 85% |
| | TFE :toluene, m:v:v:v | | | | |
| 4153156/13-1 | 1 :0.6:19:80, 4-cyanopyridine:MSA: | 310 s | sparged bed (R-3) | 89% | 80% |
| | TFE :toluene, m:v:v:v | | | | |
| 4153156/13-2 | 1:0.6:19:80, 4-cyanopyridine:MSA: | 10 + 10 min | sparged bed (R-3) | 85% | 79% |
| | TFE :toluene, m:v:v:v | | | | |
| 4153156/13-3 | 1:0.6:19:80, 4-cyanopyridine:MSA: | 5 + 5 min | sparged bed (R-3) | 88% | 79% |
| | TFE :toluene, m:v:v:v | | | | |
| 4153156/13-4 | 1:0.6:19:80, 4-cyanopyridine:MSA: | 5 + 10 min | sparged bed (R-3) | 85% | 79% |
| | TFE :toluene, m:v:v:v | | | | |
| 4153156/13-5 | 1:0.6:19:80, 4-cyanopyridine:MSA: | 5 + 20 min | sparged bed (R-3) | 85% | 78% |
| | TFE :toluene, m:v:v:v | | | | |
| 4153156/14-1 | 5.0:19:81, 4-chloropyridine-HCl: | 5 + 20 min | sparged bed (R-3) | 80% | 80% |
| | TFE :toluene, m:v:v | | | | |
| 4153156/14-2 | 5.0:19:81, 4-chloropyridine-HCl: | 5 + 30 min | sparged bed (R-3) | 79% | 76% |
| | TFE :toluene, m:v:v | | | | |
| 4153156/14-3 | 5.0:19:81, 4-chloropyridine-HCl: | 5 + 40 min | sparged bed (R-3) | 77% | 79% |
| | TFE :toluene, m:v:v | | | | |

| | | | | | |
|---|---|---|---|---|---|
| a) Activator wash before coupling was omitted; Syntheses were carried out on nucleoside-loaded polystyrene support; b) The volume of detritylation solution was 3.75 mL / 100 µmol of the starting material (i.e. 100 µmol of DMT-protected OH-groups on the starting resin) per detritylation repetition | | | | | |

Syntheses EOP 4153156/06-1 to -5 were conducted in 0.1 mmol scale following GP B. The detritylation cocktail was prepared by dissolving TFE (86 mL), 3-chloropyridine (7.0 g, 0.14 M), TFA (4.5 mL, 0.13 M) in toluene (0.36 L).

Syntheses EOP 4153156/11-1 to -5 were conducted in 0.1 mmol scale following GP B. The detritylation cocktail was prepared by dissolving TFE (86 mL), 3-cyanopyridine (4.5 g, 96 mM), TFA (3.2 mL, 92 mM) in toluene (0.36 L).

Syntheses EOP 4153156/13-1 to -5 were conducted in 0.1 mmol scale following GP B. The detritylation cocktail was prepared by dissolving TFE (86 mL), 4-cyanopyridine (4.5 g, 96 mM), methanesulfonic acid (MSA) (2.7 mL, 92 mM) in toluene (0.36 L).

Syntheses EOP 4153156/14-1 to -3 were conducted in 0.1 mmol scale following GP B. The detritylation cocktail was prepared by dissolving TFE (49 mL), 4-chloropyridine-HCl (13 g, 0.33 M), in toluene (0.20 L).

The 5mer DNA 5'-AAA AT-3' was successfully synthesized with all of the detritylation cocktails described in this example, indicating that the method of the invention allows for good variability of the base and the strong acid. The pKa values of the protonated form of the employed pyridine bases (i.e. the pyridinium ions / the salt's cation) can be retrieved from the literature (Fischer, A., Galloway, W. J., Vaughan, J., Journal of the Chemical Society (Resumed) 1964, 3591-3596): 3-cyanopyridine (protonated form: pKa = 1.35), 4-cyanopyridine (protonated form: pKa = 1.86), 3-chloropyridine (protonated form: pKa = 2.81), 4-chloropyridine (protonated form: pKa = 3.83). Thus, the pKa values of the pyridinium ions cover a range of pKa values from 1.35 to 3.83. Likewise, the employed strong acids cover a broad range of classes of acids: TFA is a carboxylic acid, MSA is a sulfonic acid, and HCl (used in the form of the pre-formed HCl-salt) is a mineral acid.

## Claims

1. A liquid composition C for use in the cleavage of a di(p-methoxyphenyl)phenylmethyl protecting group from a hydroxyl group, the composition comprising an aprotic solvent, at least one alcohol selected from the group consisting of trifluoroethanol, hexafluoroisopropanol, pentafluoropropanol, 1,1,1,3,3,3-hexafluoro-2-methyl-2-propanol, and nonafluoro tertiary butyl alcohol, and a salt of a base with a strong acid, wherein the salt's cation has a pKa in the range of 1 to 4 and the strong acid has a pKa of less than 1, and the aprotic solvent is non-halogenated and selected from the group consisting of a (hetero)aromatic solvent, an alkyl (hetero)aromatic solvent, a (hetero)aromatic ether, and an alkyl (hetero)aryl ether, and the base is a pyridine, which is substituted with one or more electron withdrawing substituents selected from the group consisting of a halogen atom, a cyano group, an aldehyde group, a keto group, a carboxyester group, and a carboxamide group.

2. A method for the synthesis of oligonucleotides, comprising the following steps a) through f):
a) providing a nucleoside or oligonucleotide bound to a supporting moiety, wherein the nucleoside or oligonucleotide comprises a backbone hydroxyl moiety, which is protected by a di(p-methoxyphenyl)phenylmethyl protecting group;
b) cleaving the di(p-methoxyphenyl)phenylmethyl protecting group from the nucleoside or oligonucleotide by incubating said compound with a liquid composition C, thereby generating a free backbone hydroxyl moiety;
c) reacting the free backbone hydroxyl group resulting from step b) with a phosphorus moiety of a nucleoside or oligonucleotide building block, which building block further comprises a hydroxyl group protected by a di(p-methoxyphenyl)phenylmethyl protecting group, thereby producing a covalent linkage between the oxygen atom of said free hydroxyl group and the phosphorus atom of said building block;
d) optionally modifying the phosphorus moiety;
e) optionally reiterating the sequence of steps b) to c) or b) to d); and
f) cleaving the oligonucleotide from the supporting moiety;
wherein the liquid composition C comprises an aprotic solvent, at least one alcohol selected from the group consisting of trifluoroethanol, hexafluoroisopropanol, pentafluoropropanol, 1,1,1,3,3,3-hexafluoro-2-methyl-2-propanol, and nonafluoro tertiary butyl alcohol, and a salt of a base with a strong acid, wherein the salt's cation has a pKa in the range of 1 to 4 and the strong acid has a pKa of less than 1, and the base is a pyridine, which is substituted with one or more electron withdrawing substituents selected from the group consisting of a halogen atom, a cyano group, an aldehyde group, a keto group, a carboxyester group, and a carboxamide group.

3. The method according to claim 2, wherein:
- the phosphorus moiety of the nucleoside or oligonucleotide building block used in step c) is a phosphorus (III) moiety;
- the method comprises a further step d) of oxidizing or sulfurizing the phosphorus (III) atom to a phosphorus (V) atom, thereby creating the desired type of internucleoside linkage; and
- step e) comprises optionally either reiterating the sequence of steps b) through d) or reiterating the sequence of steps b) and c) before executing step d).

4. The method according to any one of claims 2 to 3, wherein:
- step a) comprises providing a compound according to formula I 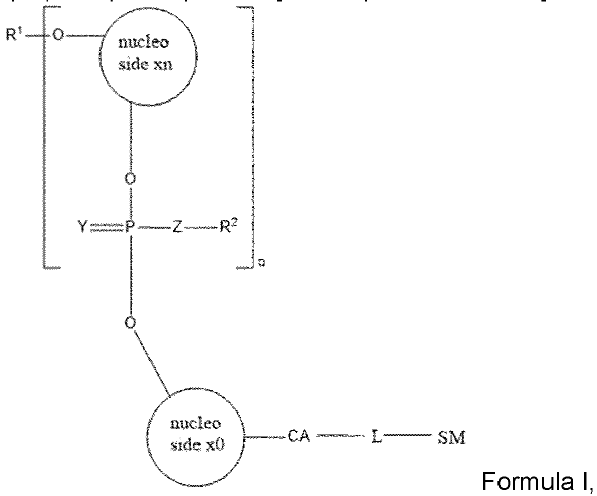 wherein:
R¹ is a di(p-methoxyphenyl)phenylmethyl protecting group ;
n is an integer equal to or larger than 0;
Y is selected independently for each repetitive unit n from the group consisting of S and O;
Z is selected independently for each repetitive unit n from the group consisting of O and S;
R² is a protecting group, which may be the same or different for each repetitive unit n;
each of the nucleosides x0 to xn may be the same or different;
CA is a capping moiety or single bond;
L is a linker moiety or single bond; and
SM is a supporting moiety;
- step c) comprises reacting the free hydroxyl group resulting from step b) with a nucleoside or oligonucleotide phosphoramidite building block, which building block comprises a backbone hydroxyl moiety protected by a di(p-methoxyphenyl)phenylmethyl protecting group, thereby producing a covalent linkage between the oxygen atom of said free hydroxyl group and the phosphorus (III) atom of said building block; and
- step e) comprises optionally reiterating the series of steps b) through d).

5. The method according to any one of claims 2 to 4, wherein the nucleoside or oligonucleotide building block is a compound according to formula II 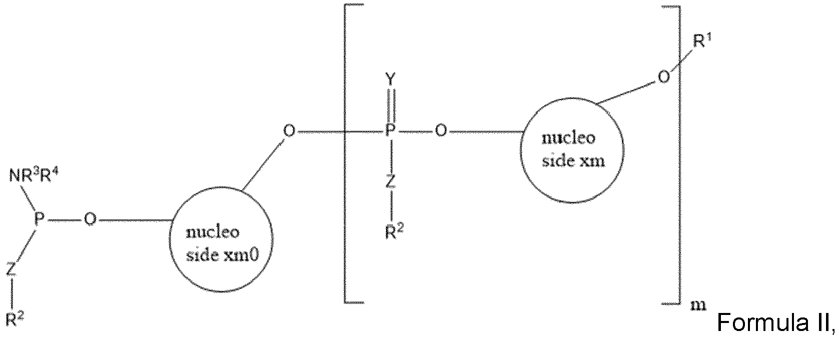 wherein:
R¹ is a di(p-methoxyphenyl)phenylmethyl protecting group ;
m is an integer equal to or larger than 0;
Y is selected independently for each repetitive unit m from the group consisting of S and O;
Z is selected independently for each position from the group consisting of O and S;
R² is a protecting group, which may be the same or different for each position;
R³ and R⁴ are protecting groups, which may be the same or different; and
each of the nucleosides xm0 to xm may be the same or different.

6. The method according to any one of claims 2 to 5, wherein at least step b) is carried out in a batch reactor or wherein the method is carried out in a column reactor and the flow rate of the liquid composition C through the column reactor is below 300 cm/h in at least one iteration of step b).

7. The method according to any one of claims 2 to 6, wherein the synthesis is carried out at a scale of 100 mmol oligonucleotide product or above.

8. The method according to any one of claims 2 to 7, wherein the method further comprises the following step h):
h) isolating the support-cleaved oligonucleotide.

9. The method according to any one of claims 2 to 8, wherein the aprotic solvent contained in the composition C is selected from the group consisting of a halogenated hydrocarbon solvent, a (hetero)aromatic solvent, an alkyl (hetero)aromatic solvent, a (hetero)aromatic ether, and an alkyl (hetero)aryl ether.

10. The method according to any one of claims 2 to 9, wherein the aprotic solvent contained in the composition C is non-halogenated.

11. The method according to any one of claims 2 to 10 or the liquid composition according to claim 1, wherein the strong acid contained in the composition C is selected from the group consisting of a carboxylic acid, a mineral acid, sulfonic acids, and a mixture thereof, preferably from the group consisting of trifluoroacetic acid, hydrochloric acid, sulfonic acids, and a mixture thereof, in particular from the group consisting of trifluoroacetic acid, hydrochloric acid, methanesulfonic acid, and a mixture thereof.

12. The method according to any one of claims 2 to 11 or the liquid composition according to any one of claims 1 and 11, wherein the base is a pyridine, in which exactly one hydrogen residue is substituted by an electron-withdrawing substituent selected from the group consisting of a cyano group and a halogen atom, preferably wherein the base is selected from the group consisting of 4-cyanopyridine, 3-cyanopyridine, 4-chloropyridine, 3-chloropyridine, and a mixture thereof.

13. The method according to any one of claims 2 to 12 or the liquid composition according to any one of claims 1 and 11 to 12, wherein the aprotic solvent contained in the composition C is selected from the group consisting of toluene, o-xylene, m-xylene, p-xylene, mesitylene, anisole, and a mixture thereof.

14. The method according to any one of claims 2 to 13 or the liquid composition according to any one of claims 1 and 11 to 13, wherein the at least one alcohol contained in the liquid composition C is selected from the group consisting of trifluoroethanol, hexafluoroisopropanol, and a mixture thereof.

15. The method according to any one of claims 2 to 14 or the liquid composition according to any one of claims 1 and 11 to 15, wherein:
- the total volume of the aprotic solvent contained in the liquid composition C accounts for 60-99% of the overall volume of the liquid composition C; and
- the total volume of the alcohol contained in the liquid composition C accounts for 1-40% of the overall volume of the liquid composition C;
and/or wherein the molar concentration of said alcohol in the composition C is at least 2 times more than the molar concentration of said base contained in the composition C;
and/or wherein in the liquid composition C:
- the total molar amount of said strong acid is in the range of 0.80-15.0 equivalents relative to the total molar amount of the di(p-methoxyphenyl)phenylmethyl groups; and
- the total molar amount of said alcohol is in the range of the 2.0-100.0 equivalents relative to the total molar amount of nucleobases.
